# EUROPÄISCHE PATENTSCHRIFT

(11) **EP 0 739 332 B1**
(45) Veröffentlichungstag und Bekanntmachung des Hinweises auf die Patenterteilung: **14.10.1998**
(21) Anmeldenummer: 95906933.7
(22) Anmeldetag: 12.01.1995
(51) Int. Cl.: C07C 45/51, C07C 47/02, C07C 29/17, C07C 31/12, C07C 41/06, C07C 43/15, C07C 41/32

(54) **VERFAHREN ZUR HERSTELLUNG VON n-BUTYRALDEHYD UND/ODER n-BUTANOL**
PROCESS FOR PRODUCING n-BUTYRALDEHYDE AND/OR n-BUTANOL
PROCEDE DE PRODUCTION DE n-BUTYRALDEHYDE ET/OU DE n-BUTANOL

(30) Priorität: 14.01.1994 DE 4400837
(43) Veröffentlichungstag der Anmeldung: 30.10.1996
(73) Patentinhaber: BASF AKTIENGESELLSCHAFT, 67056 Ludwigshafen (DE)
(72) Erfinder: KANAND, Jürgen, D-67098 Bad Dürkheim (DE); RÖPER, Michael, D-67157 Wachenheim (DE); PINKOS, Rolf, D-67098 Bad Dürkheim (DE); PACIELLO, Rocco, D-67098 Bad Dürkheim (DE); THOME, Alfred, D-67346 Speyer (DE)
(86) Internationale Anmeldenummer: EP9500114
(87) Internationale Veröffentlichungsnummer: WO9519334

(56) Entgegenhaltungen:
- WO-A-91/03449
- DE-C- 821 202
- FR-A- 2 331 540
- FR-E- 82 549

## Beschreibung

Die vorliegende Erfindung betrifft ein Verfahren zur Herstellung n-Butyraldehyd und/oder n-Butanol.

n-Butyraldehyd und n-Butanol sind Großprodukte der chemischen Industrie und finden vielseitige Verwendung. n-Butyraldehyd z.B. wird weltweit in Mengen von über 4 Millionen t/Jahr produziert und dient u.a. als Ausgangsmaterial zur Herstellung von Weichmacheralkoholen. n-Butanol wird in großem Umfang als Lösungsmittel, beispielsweise für Lacke, eingesetzt.

n-Butyraldehyd wird heute großtechnisch praktisch ausschließlich durch die Hydroformylierung von Propen hergestellt, wozu verschiedene Verfahren, die im wesentlichen Kobalt- oder Rhodium-Hydroformylierungskatalysatoren verwenden, benutzt werden (Kirk-Othmer: Encyclopedia of Chemical Technology, 4. Aufl., Band 4, S. 741 - 746, John Wiley & Sons, New York 1992).

n-Butanol ist eines der mengenmäßig wichtigsten Folgeprodukte des n-Butyraldehyds und wird aus diesem durch Hydrierung gewonnen. Andere Verfahren zur Herstellung von n-Butanol, wie die Hydrierung von Crotonaldehyd, der wiederum durch Aldolkondensation von Acetaldehyd erzeugt wird, sind heute nur noch von historischen Interesse oder haben, wie die mikrobiologische Erzeugung von n-Butanol durch die Vergärung von Melasse, nur regionale Bedeutung (Kirk-Othmer: Encyclopedia of Chemical Technology, 4. Aufl., Band 4, S. 694 - 696, John Wiley & Sons, New York 1992). Diese Verfahren, insbesondere die Hydroformylierung von Propen erfordern hohe Investitionen, beispielsweise für die Errichtung von Hochdruckanlagen für die Kobalt-katalysierte Hydroformylierung oder für den Kauf des teuren Rhodium-Katalysators, die Anlagen für dessen Handhabung bei der Hydroformylierung und zur Aufarbeitung verbrauchter, Rhodium-haltiger Katalysatorlösungen. Des weiteren müssen zur Herstellung von n-Butyraldehyd nach dem Hydroformylierungsverfahren Synthesegasanlagen zur Verfügung stehen, die das zur Hydroformylierung benötigte Synthesegas liefern. Ein weiterer Nachteil des Hydroformylierungsverfahrens ist der hohe Zwangsanfall des Nebenprodukts Isobutyraldehyd, der wirtschaftlich, aufgrund seiner mengenmäßig beschränkten Weiterverwendungsmöglichkeiten, niedrig bewertet wird.

1,3-Butadien ist eine Grundchemikalie, die in großen Mengen in Steamcrackern erzeugt und aus dem C₄-Schnitt des Steamcrackers extraktiv, beispielsweise mittels N-Methylpyrrolidon isoliert wird. Obwohl 1,3-Butadien in großen Mengen zur Verfügung steht und ein sehr preiswerter Rohstoff ist, wurde bislang noch kein großtechnisch anwendbares Verfahren zur Herstellung von n-Butyraldehyd oder n-Butanol auf der Basis von 1,3-Butadien entwickelt. Ein Grund hierfür ist sowohl in der Neigung des 1,3-Butadiens zu Dimerisierungs- und Polymerisationsreaktionen als auch die Bildung von Gemischen aus 1,2- und 1,4-Addukten bei Additionsreaktionen zu sehen. Ursächlich für dieses chemische Verhalten ist das Vorliegen zweier konjugierter Doppelbindungen im 1,3-Butadienmolekül (Kirk-Othmer: Encyclopedia of Chemical Technology, 4. Aufl., Band 4, S. 676 - 683, John Wiley & Sons, New York 1992).

Es ist aus US-A 29 22 822 und DE-A 25 50 902 bekannt, daß Alkohole in flüssiger Phase mit 1,3-Butadien in Gegenwart von sauren Ionenaustauschern zu den entsprechenden ungesättigten Ethern reagieren. In US-A 29 22 822 wird diese Umsetzung in Gegenwart eines großen Überschusses an Methanol durchgeführt, was zu einer erhöhten Bildung des unerwünschten Dimethylethers führt. Gemäß dem Verfahren von DE-A 25 50 902 entsteht bei dieser Umsetzung Vinylcyclohexen als Hauptprodukt. Gemäß EP-A 25 240 wird die Addition von Alkoholen an 1,3-Butadien vorteilhaft in Gegenwart eines polaren, aprotischen Lösungsmittels vorgenommen, das dann wieder abdestilliert werden muß. Gemäß GB-A 943 160 wird die Addition von Alkoholen mit Brönsted-Säuren in Gegenwart von Kupfersalzen durchgeführt.

Auch Übergangsmetallkomplexe mit Phosphinliganden wurden als Katalysatoren für die Addition von Alkoholen an 1,3-Butadien benutzt. Chauvin et al. (Bull. Chim. Soc. France 652 (1974)) untersuchten die Addition von Alkoholen an 1,3-Butadien mit Trialkyl- und Triarylphosphin-Komplexen des Nickels und Palladiums. Teilweise wurden bei dieser Umsetzung Alkoholate, insbesondere Phenolate, als Cokatalysatoren eingesetzt. Gemäß DD-A 206 989 werden für die Umsetzung von Isopren mit Alkoholen Alkylpalladium(II)Komplexe mit Trialkyl- oder Triaryl-Phosphin- oder -Phosphit-Liganden in Gegenwart von Alkalimetallalkoholaten verwendet. Kawazura et al. (J. Chem. Soc. Chem. Com. 2213, (1972)) verwenden Rhodium(III)chlorid als Katalysator, desgleichen Dewhirst (J. Org. Chem. 32, 1297 (1967)). Taylor (Symposium on new Routes to new Olefins; Division of Petroleum 5 Chemistry, Inc.; American Chemical Society, Boston Meeting, 1972) untersuchte die Addition von Alkoholen an 1,3-Butadien mittels Kupfer(I)chlorid und Rhodium(I)-Alkadien-Komplexen. Jolly et al. (Synthesis 771 (1990)) erwähnen die Umsetzung von 1,3-Butadien mit Trialkylphosphin-Palladium-Komplexen. Bei all den genannten Umsetzungen bilden sich Gemische aus 3-Alkoxybut-1-enen und 1-Alkoxybut-2-enen. In vielen dieser Umsetzungen des Standes der Technik sind die Umsätze und Ausbeuten unbefriedigend und es bilden sich eine Vielzahl von oligomeren Butadien-Derivaten, für die es praktisch keine Verwendung gibt oder die nur in so geringen Mengen Anwendung finden, daß der größte Teil dieser als Zwangsanfall in einem großtechnischen Verfahren entstehenden Nebenprodukte entsorgt werden müßte.

Zur Isomerisierung von Allylethern zu Enolethern wurden bereits eine Reihe von Reagenzien untersucht. Nach Baudry et al. (J. Chem. Soc. Chem. Comm. 694 (1978)) entsteht bei der Umsetzung von 1-Methoxybut-2-en in Gegenwart eines kationischen Iridium-Komplexes 1-Methoxybut-1-en. Tatsumi et al. (J. Organomet. Chem. 252, 105 (1983)) verwenden für diese Umsetzung einen Molybdän-Komplex. Von Menicagli et al. (J. Org. Chem. 52, 5700 (1987)) wird ein Ruthenium-Triphenylphosphin-Hydrido-Komplex zur Isomerisierung acetalischer Allylether zu acetalischen Vinylethern benutzt. Suzuki et al. (Tetrahedron Lett. 21, 4927 (1980)) verwenden für die Isomerisierung von cyclischen Allyletheracetalen zu den entsprechenden Vinyletheracetalen ähnliche Ruthenium-Komplexe.

Außer den vorgenannten Homogenkatalysatoren wurden auch heterogene Katalysatoren zur Isomerisierung von Allylethern zu Enolethern in flüssiger Phase eingesetzt. Die Umlagerung von Allylphenyl- und -alkylethern in Gegenwart von Palladium auf Aktivkohle-Katalysatoren führt nach Boss et al. (Angew. Chem. 88, 578 (1976)) zum entsprechenden Enolether, aus dessen Methylvinyl-Gruppierung Propionaldehyd anschließend in Gegenwart von Wasser und Säure freigesetzt wird. WO 91/03 449 betrifft ein Verfahren zur Isomerisierung von Allylethern zu Enolethern mittels Ruthenium- oder Rhodium-Trägerkatalysatoren unter wasserfreien Bedingungen.

Die direkte einstufige Umwandlung von Allylethern zu den entsprechenden gesättigten Alkoholen ist nicht bekannt.

Der vorliegenden Erfindung lag somit die Aufgabe zugrunde, ein großtechnisch anwendbares, wirtschaftliches Verfahren zur Herstellung von n-Butyraldehyd und/oder n-Butanol zu finden, das es ermöglicht, diese Produkte mit hoher Ausbeute und Selektivität herzustellen, insbesondere sollte die Menge der im Verfahren gebildeten Nebenprodukte gering sein oder diese Nebenprodukte selbst gesuchte Handelsprodukte sein. Weiterhin sollte das Verfahren flexibel sein, um zu ermöglichen, daß wahlweise n-Butyraldehyd und/oder n-Butanol, entsprechend dem Bedarf für diese Verbindungen, hergestellt werden können. Die Ausübung des Verfahrens sollte nicht auf das Vorhandensein einer Synthesegas-Anlage angewiesen sein und ohne Hochdruckanlagen auskommen.

Dementsprechend wurde ein Verfahren zur Herstellung -Butyraldehyd und/oder n-Butanol gefunden, das dadurch gekennzeichnet ist, daß man
a) 1,3-Butadien oder 1,3-Butadien-haltige Kohlenwasserstoffatome mit einem Alkohol der Formel I

   ROH I,

   in der der Rest R eine unsubstituierte oder mit bis 2 C₁- bis C₁₀-Alkoxy- oder Hydroxygruppen substituierte C₂- bis C₂₀-Alkyl- oder Alkenyl-, eine C₆- bis C₁₀-Aryl- oder eine C₇- bis C₁₁-Aralkylgruppe oder die Methylgruppe ist, bei erhöhter Temperatur und bei erhöhtem Druck in Gegenwart einer Brönsted-Säure oder in Gegenwart eines Komplexes eines Elementes aus der Gruppe Ib, VIIb oder VIIIb des Periodensystems der Elemente mit Phosphor- oder Stickstoff-haltigen Liganden zu einem Gemisch der Addukte der Formeln II 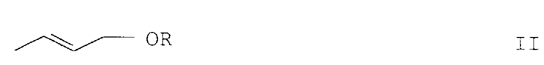 und III 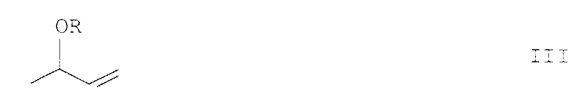 umsetzt,
b) das Addukt III zum Addukt II isomerisiert,
c) das Addukt II in Gegenwart eines homogenen oder heterogenen Übergangsmetallelement-Katalysators in flüssiger Phase oder in Gegenwart eines heterogenen, Übergangsmetallelement-haltigen Katalysators in der Gasphase zum Enolether der Formel IV 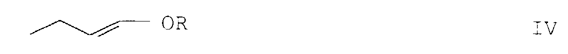 isomerisiert, und
d) aus diesem Enolether IV durch dessen Umsetzung mit Wasserstoff und Wasser oder Wasser in Gegenwart eines homogenen oder heterogenen Übergangsmetallelement-Katalysators in flüssiger Phase oder in Gegenwart eines heterogenen Übergangsmetallelement-haltigen Katalysators in der Gasphase n-Butyraldehyd und/oder n-Butanol erzeugt und den Alkohol ROH I wieder freisetzt, und den freigesetzten Alkohol ROH I wieder in die Umsetzung gemäß Teilreaktion a) zurückführt.

Das erfindungsgemäße Verfahren setzt sich somit aus 4 Teilreaktionen a) bis d) zusammen. Die Teilreaktionen c) und d) können wahlweise einzeln, nacheinander, in mindestens 2 Verfahrensstufen oder praktisch simultan in einer einzigen Verfahrensstufe durchgeführt werden. Ähnliches gilt für die Teilreaktionen a) und b), wobei die Isomerisierung des Addukts III zum Addukt II gemäß Teilreaktion b) nach Rückführung des Addukts III in die Verfahrensstufe der Addition des Alkohols ROH an 1,3-Butadien simultan mit der Addition gemäß Teilreaktion a) verläuft. Dadurch wird es auf einfache Weise möglich, die Verfahrensbedingungen für das erfindungsgemäße Verfahren an die örtlichen Gegebenheiten des Standortes, an dem eine Anlage zur Ausübung des Verfahrens installiert wird, anzupassen, beispielsweise indem man vor Ort bereits bestehende Anlagenteile in die Anlage für das erfindungsgemäße Verfahren integriert. Weiterhin kann das erfindungsgemäße Verfahren wahlweise so ausgestaltet werden, daß keine teuren Edelmetall-Katalysatoren eingesetzt werden müssen.

Der Ausdruck "Verfahrensstufe" wird in dieser Anmeldung für eine Anlageneinheit gebraucht, in der jeweils eine einzelne der Teilreaktionen a) bis d) an dem oder den in dieser Anlageneinheit eingesetzten Katalysator oder Katalysatoren abläuft oder in der mehrere, insbesondere 2 dieser Teilreaktionen, nebeneinander an dem oder den in dieser Anlageneinheit verwendeten Katalysator oder Katalysatoren ablaufen. Die Hydrolyse bzw. die kombinierte Hydrolyse/Hydrierung des Enolethers IV gemäß Teilreaktion d) wird dabei, sofern in dieser Anmeldung nichts anderes erwähnt wird, als eine einzelne Teilreaktion betrachtet.

Ist der in einer Anlageneinheit verwendete Katalysator oder jeder der in einer Anlageneinheit verwendeten Katalysatoren in der Lage, unter den dort angewandten Reaktionsbedingungen beispielsweise die Isomerisierung des Addukts II zum Enolether IV gemäß Teilreaktion c) und die Hydrolyse oder Hydrierung des Enolethers IV zu n-Butyraldehyd und/oder n-Butanol gemäß Teilreaktion d) zu katalysieren, so daß keine strenge räumliche Trennung des Ablaufs dieser Teilreaktionen in der Anlageneinheit festgestellt werden kann, so wird in dieser Anmeldung von der Durchführung der Teilreaktionen c) und d) in einer einzigen Verfahrensstufe gesprochen. Eine Anlageneinheit kann dabei sowohl einen einzigen Reaktor als auch mehrere, in Reihe geschaltete Reaktoren umfassen, die mit dem gleichen oder gegebenenfalls mit verschiedenen Katalysatoren befüllt sind und in der gleichen Betriebsweise und bei gleichen oder verschiedenen Temperatur- und Druckbedingungen betrieben werden. Als Betriebsweise wird dabei jeweils das Arbeiten in flüssiger Phase unter Anwendung eines Homogenkatalysators oder das Arbeiten in flüssiger Phase unter Anwendung eines Heterogenkatalysators oder das Arbeiten in der Gasphase verstanden. Daraus folgt, daß in dieser Anmeldung beispielsweise dann nicht von einer "Umsetzung in einer einzigen Verfahrensstufe" gesprochen wird, wenn in den einzelnen, aufeinanderfolgenden Reaktoren Katalysatoren angewandt werden, die allein zur Katalyse einer bestimmten Teilreaktion befähigt sind oder wenn in diesen Reaktoren mit unterschiedlichen Betriebsweisen gearbeitet wird.

Im folgenden wird das erfindungsgemäße Verfahren näher erläutert:

Im Stufe a) wird 1,3-Butadien in Gegenwart eines Katalysators mit dem Alkohol ROH I gemäß Gleichung (1) 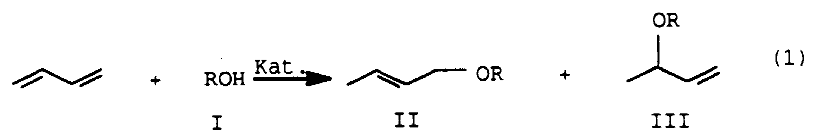 zum 1,4-Addukt der Formel II und dem 1,2-Addukt der Formel III umgesetzt. Im entstandenen 1,4-Addukt II kann die Doppelbindung sowohl in der cis- als auch trans-Form vorliegen, dies ist für den weiteren Ablauf des Verfahrens aber nicht erheblich. Die Addukte II und III entstehen dabei je nach Reaktionsbedingungen und angewandtem Katalysator im allgemeinen in einem Molverhältnis von 1:1 bis 1:3.

Die Art des in die Umsetzung eingesetzten Alkohols ROH I ist in der Regel für das Verfahren nicht kritisch. Es können sowohl primäre als auch sekundäre Alkohole verwendet werden, bevorzugt werden aber primäre Alkohole eingesetzt. Es können sowohl aliphatische, cycloaliphatische, aromatische als auch araliphatische Alkohole benutzt werden, vorzugsweise finden aliphatische und araliphatische Alkohole Anwendung. Im allgemeinen werden Alkohole ROH I im erfindungsgemäßen Verfahren verwendet, in denen der Rest R eine C₁- bis C₂₀-Alkyl-, C₂- bis C₁₀-Alkenyl-, z.B. die But-2-enyl-, vorzugsweise eine C₁- bis C₄-Alkyl-, insbesondere die n-Butylgruppe, eine C₆- bis C₁₀-Aryl-, vorzugsweise die Phenylgruppe oder eine C₇- bis C₁₁-Aralkyl-, vorzugsweise die Benzylgruppe ist. Die Reste R können gegebenenfalls mit Substituenten wie C₁- bis C₁₀-Alkoxy- und/oder Hydroxyl-Gruppen substituiert sein. Als Alkohole ROH I können somit auch Diole oder Triole oder Alkoxyalkohole verwendet werden. Da diese Substituenten in der Regel keinen kritischen Einfluß auf die Umsetzung haben, werden vorzugsweise Alkohole ROH I mit unsubstituierten Resten R verwendet. Selbstverständlich können auch Alkohole mit einer höheren Anzahl an Kohlenstoffatomen eingesetzt werden, da solche höheren Alkohole in der Regel teurer sind als niedere Alkohole, werden aus wirtschaftlichen Gründen niedere Alkohole bevorzugt verwendet.

Als Katalysatoren können in Stufe a) eine Vielzahl von Katalysatoren eingesetzt werden, beispielsweise Brönsted-Säuren oder auch Phosphin-Komplexe von Übergangsmetallen aus den Gruppen Ib, VIIb oder VIIIb des Periodensystems der Elemente, insbesondere Phosphin-Komplexe des Palladiums und Nickels.

Als Brönsted-Säuren können beispielsweise herkömmliche, nicht oxidierende Brönsted-Säuren, wie Halogenwasserstoffsäuren, z.B. Salzsäure, Schwefelsäure, Phosphorsäure, Perchlorsäure, Flußsäure, Tetrafluoroborsäure, Methansulfonsäure oder Toluolsulfonsäure Verwendung finden, vorzugsweise werden aber feste Brönsted-Säuren, insbesondere organische oder anorganische Kationentauscher, eingesetzt.

Unter organischen Kationenaustauschern werden pulverförmige, gelartige oder makroporöse, polymere Polyelektrolyte verstanden, die Brönsted-acide funktionelle Gruppen, wie Sulfonsäure-, Phosphonsäure- oder Carboxylgruppen auf einer polymeren Matrix tragen, beispielsweise sulfonierte Phenol-Formaldehyd-Harze, sulfonierte Styrol-Divinylbenzol-Copolymere, sulfoniertes Polystyrol, Poly (perfluoralkylen)sulfonsäuren oder sulfonierte Kohlen. Im erfindungsgemäßen Verfahren können diese Kationenaustauscher in Form handelsüblicher Produkte eingesetzt werden, wie sie beispielsweise unter den Handelsnamen Amberlite®, Dowex®, Amberlyst®, Lewatit®, Wofatit®, Permutit® und Nafion® im Handel erhältlich sind. Zweckmäßigerweise kommen die Kationenaustauscher im erfindungsgemäßen Verfahren in ihrer protonierten Form, der sogenannten H⁺-Form zur Anwendung. Als geeignete organische Kationenaustauscher seien beispielhaft die Handelsprodukte Amberlite® 200, Amberlite® IR 120, Amberlite® IR 132 E, Lewatit® SC 102, Lewatit® SC 104, Lewatit® SC 108, Lewatit® SPC 108, Lewatit® SPC 112, Lewatit® SPC 118 und Amberlyst® 15 genannt.

Vorteilhafte Ergebnisse konnen im erfindungsgemäßen Verfahren weiterhin mit modifizierten organischen Kationenaustauschern erzielt werden, beispielsweise solchen, die zusätzlich Lewis-Säuren, wie Kupfer(II)Halogenide, insbesondere Kupfer(II)chlorid, Kupfer(II)bromid, Kupfer(II)jodid, oder Kupfer(II)salze, wie Kupfer(II)sulfat, Kupfer(II)nitrat oder Kupfer(II)acetat enthalten. Solche Lewis-Säure-haltigen kationische Ionenaustauscher können z.B. nach dem Verfahren von GB-A 943 160 hergestellt werden. Vorzugsweise werden die Lewis-Säure-haltigen Ionenaustauscher in einer Form eingesetzt, in der nur ein Teil der Wasserstoffionen der Brönsted-aciden Gruppen des Ionenaustauschers gegen das Lewis-Säure-Kation ausgetauscht ist, während die restlichen Brönsted-aciden Gruppen weiterhin als Brönsted-Säuren fungieren. Im allgemeinen werden die organischen Ionenaustauscher mit einer solchen Menge an Lewis-Säure dotiert, daß 5 bis 15 mol-%, vorzugsweise 10 bis 40 mol-%, insbesondere 15 bis 30 mol-% der Wasserstoffionen der auf dem Ionenaustauscher vorliegenden Brönsted-aciden Gruppen gegen die betreffende Lewis-Säure ausgetauscht sind.

Anstelle organischer, saurer Kationenaustauscher können im erfindungsgemäßen Verfahren auch feste, Brönsted-acide wirkende anorganische Feststoffe eingesetzt werden, beispielsweise Zeolithe, wie β-Zeolithe oder Y-Zeolithe in der H⁺-Form, Bleicherden, wie Bentonite, Montmorillonite oder Attapulgite, nichtzeolitische Molekularsiebe auf Phosphatbasis, wie sie beispielsweise Gegenstand von US-A 4 440 871, US-A 4 310 440, US-A 4 567 029, US-A 4 554 143, US-A 4 500 651, EP-A 158 976, EP-A 158 349, EP-A 159 624 sind, sowie saure oder säureimprägnierte Metalloxide, deren Herstellung z.B. in US-A 4 873 017 beschrieben ist. Bevorzugte Brönsted-acide anorganische Feststoffe sind β-Zeolithe oder Y-Zeolithe in der H⁺-Form, insbesondere β-Zeolithe in der H⁺-Form. β-Zeolithe sind z.B. nach dem Verfahren von US A 4 891 458 erhältlich.

Vorzugsweise werden im erfindungsgemäßen Verfahren zur Addition von Alkoholen ROH I an 1,3-Butadien in Teilreaktion a) organische Ionenaustauscher verwendet.

Werden in Teilreaktion a) des erfindungsgemäßen Verfahrens flüssige oder gelöste Brönsted-Säure-Katalysatoren, insbesondere Schwefelsäure, Phosphorsäure, Toluolsulfonsäure, Methansulfonsäure oder Tetrafluoroborsäure eingesetzt, wird im allgemeinen so vorgegangen, daß man den Alkohol ROH und 1,3-Butadien in flüssiger oder vorzugsweise in gasförmiger Form in die vorgelegte Säure einleitet und die gebildeten Addukte der Formeln II und III, destillativ oder durch Strippen aus der Reaktionszone entfernt. Hierzu können an sich übliche Reaktoren wie Blasensäulen, Schlaufenreaktoren u.ä. verwendet werden. Vorteilhaft kann das Alkohol/1,3-Butadien-Gemisch z.B. mittels Strahldüsen in die Säure eingebracht werden. Die Addukte II und III können von der wäßrigen Lösung der Brönsted-Säure auch mittels Phasenabscheidern abgetrennt werden. Anstelle von Blasensäulen oder Schlaufenreaktoren können auch Rührkesselkaskaden verwendet werden, wobei vorteilhaft bei einem Druck gearbeitet wird, bei dem das 1,3-Butadien unter den gewählten Reaktionsbedingungen flüssig ist.

Vorzugsweise werden im erfindungsgemäßen Verfahren jedoch feste Brönsted-Säuren in Form der obengenannten organischen oder anorganischen Katalysatoren verwendet, insbesondere organische Ionenaustauscher. Diese werden vorzugsweise in einem Festbett angeordnet und von der flüssigen Reaktionsmischung in der Sumpf- oder Rieselfahrweise durchströmt. Das Katalysatorfestbett kann z.B. in Rohrreaktoren oder vorzugsweise in Reaktorkaskaden eingebaut werden. Es ist auch möglich, die Reaktanten gasförmig durch die Katalysatorschüttung zu leiten, bevorzugt wird in der flüssigen Phase gearbeitet. Es versteht sich von selbst, daß die Addition des Alkohols ROH an 1,3-Butadien gemäß Teilreaktion a) sowohl kontinuierlich als auch diskontinuierlich durchgeführt werden kann.

Das Molverhältnis Alkohol/1,3-Butadien kann im erfindungsgemäßen Verfahren aus einem weiten Bereich gewählt werden. Im allgemeinen wird ein Molverhältnis Alkohol ROH/1,3-Butadien von 0,5:1 bis 5:1 vorzugsweise von 1:1 bis 2,5:1 und besonders bevorzugt von 1,5:1 bis 2,5:1 angewandt. Die Umsetzung des Alkohols ROH mit 1,3-Butadien wird bei Durchführung des Verfahrens in flüssiger Phase im allgemeinen bei Temperaturen von 20 bis 150°C, vorzugsweise von 50 bis 120°C, insbesondere von 70 bis 110°C und bei einem Druck von im allgemeinen 10 bis 100 bar, vorzugsweise von 10 bis 50 bar, insbesondere von 20 bis 30 bar, vorgenommen. Zweckmäßigerweise wird der Druck so gewählt, daß das 1,3-Butadien bei der angewandten Reaktionstemperatur flüssig ist. Die Anwendung eines höheren Drucks ist möglich. Die angewandte Reaktionstemperatur wird zweckmäßigerweise hinsichtlich des jeweils verwendeten Brönsted-Säure-Katalysators in einem Vorversuch optimiert. Werden Mineralsäuren oder stark saure Ionenaustauscher, wie Nafion®, als Brönsted-Säure-Katalysatoren eingesetzt, findet die Umsetzung schon bei Raumtemperatur statt.

Im allgemeinen wird das Alkohol ROH/1,3-Butadien-Gemisch mit einer Raumgeschwindigkeit von 0,01 bis 0,5 g/cm³·h, vorzugsweise von 0,02 bis 0,4 g/cm³·h und besonders bevorzugt von 0,02 bis 0,05 g/cm³·h durch das Katalysatorfestbett geleitet. Der Zusatz eines Lösungsmittels zum Reaktionsgemisch ist möglich, im allgemeinen aber nicht erforderlich, da der eingesetzte Alkohol als auch die Addukte II und III auch als Lösungsmittel fungieren können. Die Verweilzeit des Alkohol ROH/1,3-Butadiengemisches im Reaktor beträgt im allgemeinen 1 bis 6 Stunden und ist in der Regel von der angewandten Reaktionstemperatur abhängig.

Wird die Addition des Alkohols ROH an 1,3-Butadien in der Gasphase durchgeführt, werden im allgemeinen Temperaturen von weniger als 120°C und ein Druck von im allgemeinen von weniger als 20 bar angewandt. Gewünschtenfalls kann das Reaktionsgas mit einem unter den Reaktionsbedingungen inerten Gas, z.B. Stickstoff, vermischt werden, im allgemeinen wird das Reaktionsgas unverdünnt eingesetzt.

In einer weiteren Ausgestaltung des erfindungsgemäßen Verfahrens kann die Addition des Alkohols ROH I mittels eines homogen im Reaktionsmedium gelösten oder heterogenisierten Übergangsmetallelementkatalysators, der ein Element aus der Gruppe Ib, VIIb oder VIIIb des Periodensystems der Elemente, wie Kupfer, Nickel, Rhodium, Palladium, Platin, Rhenium oder Iridium, vorzugsweise Palladium oder Nickel, enthält, vorgenommen werden.

Zweckmäßigerweise werden diese Übergangsmetallelementkatalysatoren, insbesondere die Palladium- und Nickel-Katalysatoren, in Form ihrer homogen im Reaktionsmedium löslichen Komplexe mit z.B. Phosphin-, 2,2'-Bipyridin- oder 1,10-Phenanthrolin-Liganden eingesetzt. Im erfindungsgemäßen Verfahren können zu diesem Zweck zur Komplexierung der Gruppe Ib, VIIb- oder VIIIb-Metalle, insbesondere des Palladiums und Nickels, eine Vielzahl unterschiedlicher Phosphin-, 2,2'-Bipyridin- oder 1,10-Phenanthrolin-Liganden verwendet werden. Als Liganden können sowohl einzähnige oder mehrzähnige, insbesondere zweizähnige, Phosphin-Liganden verwendet werden. Geeignete Phosphin-Liganden sind z.B. Trialkylphosphine, Triarylphosphine, Alkyldiarylphosphine, Aryldialkylphosphine, Aryldiphosphine, Alkyldiphosphine und Arylalkyldiphosphine. Die Alkylgruppen-tragenden Phosphin-Liganden können gleiche oder verschiedene C₁- bis C₁₀-, vorzugsweise C₁- bis C₆-Alkyl- oder -Cycloalkylgruppen enthalten. Die Arylgruppentragenden Phosphin-Liganden können gleiche oder verschiedene C₆- bis C₁₂-Arylgruppen, insbesondere die Phenyl- oder Naphthylgruppe aber auch Diphenylgruppen enthalten. Weiterhin können Phosphin-Liganden zur Komplexierung der Gruppe Ib, VIIb- oder VIIIb-Elemente eingesetzt werden, die heterocycloaliphatische Gruppen wie Pyrrolidin-, Imidazolidin-, Piperidin-, Morpholin-, Oxazolidin-, Piperazin- oder Triazolidin-Gruppen oder heteroaromatische Gruppen, wie Pyrrol-, Imidazol-, Oxazol-, Indol-, Pyridin-, Chinolin-, Pyrimidin-, Pyrazol-, Pyrazin-, Pyridazin- oder Chinoxalin-Gruppen gemeinsam mit anderen Alkyl- oder Aryl-Gruppen tragen. Die Alkyl- oder Arylgruppen der Liganden können unsubstituiert sein oder unter den Reaktionsbedingungen inerte Substituenten, wie C₁- bis C₄-Alkoxy- oder Di-C₁- bis C₄-alkylamino-, C₁- bis C₆-Alkyl-, Nitro-, Cyano- oder Sulfonat-Gruppen tragen.

Prinzipiell besteht keine Beschränkung für die Anwendbarkeit derartiger Liganden zur Komplexierung der Gruppe Ib-, VIIb- oder VIIIb-Elemente, insbesondere des Palladiums und Nickels im erfindungsgemäßen Verfahren. Vorzugsweise werden aber aus Kostengründen solche Liganden eingesetzt, die auf einfache Weise hergestellt werden können.

Eine lediglich beispielhafte Aufzählung derartiger Liganden wird im folgenden gegeben: Trimethylphosphin, Triethylphosphin, Tripropylphosphin, Triisopropylphosphin, Tributylphosphin, Trioctylphosphin, Tridecylphosphin, Tricyclopentylphosphin, Tricyclohexylphosphin, Triphenylphosphin, Tritolylphosphin, Cyclohexyldiphenylphosphin, Tetraphenyldiphosphinomethan, 1,2-Bis(diphenylphosphino)ethan, Tetramethyldiphosphinomethan, Tetraethyldiphosphinomethan, 1,3-Bis (diphenylphosphino)propan, 1,4-Bis(diphenylphosphino)butan, Tetra-t-butyl-diphosphinomethan, 1,2-Bis-(dimethylphosphino)ethan, 1,2-Bis(diethylphosphino)ethan, 1,2-Bis(dipropylphosphino)ethan, 1,2-Bis(diisopropylphosphino)ethan, 1,2-Bis(dibutylphosphino)ethan, 1,2-Bis(di-t-butylphosphino)ethan, 1,2-Bis(dicyclohexylphosphino)ethan, sowie die in EP-A 279 018, EP-A 311 619, WO 90/06 810 und EP-A 71 281 beschriebenen Bisphosphin-Liganden. Außer nach den in den zuvor genannten Patentanmeldungen beschriebenen Verfahren können die Alkyl- bzw. Arylphosphin-Liganden nach an sich herkömmlichen Methoden, beispielsweise gemäß den in Houben-Weyl, Methoden der Organischen Chemie, Band XII/1, 4. Auflage, S. 17-65 und S. 182-186, Thieme, Stuttgart, 1963 und Band E 1, 4. Auflage, S. 106-199, Thieme, Stuttgart, 1982 angegebenen Verfahren hergestellt werden.

Außer Phosphin-Liganden können im erfindungsgemäßen Verfahren vorteilhaft auch 2,2'-Bipyridin- oder 1,10-Phenanthrolin-Liganden der Alkyl- oder Aryl-substituierte oder anellierte 2,2'-Bipyridin- oder 1,10-Phenanthrolin-Derivate, die die für die Komplexbildungseigenschaft der 2,2'-Bipyridin- oder 1,10-Phenanthrolin-Liganden ursächliche (-N=C-C=N-)-Gruppierung enthalten, beispielsweise 2,2'-Bichinolin, 4,7-Diphenyl-1,10-phenanthrolin, 2,9-Dimethyl-4,7-diphenyl-1,10-phenanthrolin, 4,5-Diazafluoren, Dipyrido[3,2-a:2',3'-c]phenazin, 2,2',6',2"-Terpyridin u.ä., eingesetzt werden. Diese Liganden sind zum Teil im Handel erhältlich, z.B. 2,2'-Bipyridin oder 1,10-Phenanthrolin oder können nach den in Synthesis 1, (1976) oder Aust. J. Chem. 23, 1023 (1970) angegebenen Methoden hergestellt werden.

Die im erfindungsgemäßen Verfahren für die Teilreaktion a) anwendbaren Komplexe der Elemente der Gruppe Ib, VIIb oder VIIIb, insbesondere des Palladiums und Nickels, können sowohl in situ im Reaktionsgemisch erzeugt oder präformiert der Reaktionsmischung zugesetzt werden. Zur in-situ-Erzeugung dieser Komplexe wird im allgemeinen so vorgegangen, daß man Verbindungen der Gruppe Ib-, VIIb- oder VIIIb-Elemente, z.B. deren Halogenide, vorzugsweise deren Chloride, Bromide oder Iodide, die Nitrate, Cyanide oder Sulfate oder Komplexverbindungen dieser Metalle, wie Acetylacetonate, Carboxylate, Carbonylkomplexe oder Olefinkomplexe, wie Ethen oder Butadien-Komplexe, zusammen mit dem betreffenden Liganden der Reaktionsmischung zuführt, worauf sich die erfindungsgemäß in Teilreaktion a) anwendbaren Komplexe in der Reaktionsmischung bilden. Im allgemeinen wird hierbei der Komplexbildner bezüglich des Gruppe Ib-, VIIb- oder VIIIb-Elementes in einem Molverhältnis von 2 bis 200, vorzugsweise von 2 bis 10, insbesondere von 2 bis 4 zugegeben.

Im allgemeinen wird bei der Addition des Alkohols ROH an 1,3-Butadien in Stufe a) des erfindungsgemäßen Verfahrens bei Anwendung der genannten Gruppe Ib, VIIb oder VIIIb-Element-Komplex-Katalysatoren, insbesondere der Palladium-KomplexKatalysatoren ein Molverhältnis 1,3-Butadien/Gruppe Ib, VIIb- oder VIIIb-Element von 100:1 bis 100000:1, vorzugsweise von 200:1 bis 2000:1 und besonders bevorzugt von 400:1 bis 1000:1 eingestellt, wobei dieses Molverhältnis im Falle der kontinuierlichen Durchführung des Verfahrens auf die stationäre 1,3-Butadien-Konzentration in der flussigen Reaktionsmischung bezogen ist.

Das Molverhältnis Alkohol ROH/1,3-Butadien kann bei dieser Verfahrensausgestaltung in weiten Grenzen gewählt werden und ist in der Regel nicht kritisch. Beispielsweise kann der an 1,3-Butadien zu addierende Alkohol außer als Reagenz auch als Lösungsmittel für den Komplexkatalysator fungieren. Im allgemeinen wird deshalb im erfindungsgemäßen Verfahren in der Teilreaktion a) ein Alkohol/1,3-Butadien-Molverhältnis von 0:1 bis 10:1, vorzugsweise von 1:1 bis 5:1 und besonders bevorzugt von 1:1 bis 2:1 angewandt, wobei sich diese Angaben im Falle der kontinuierlichen Ausgestaltung des Verfahrens auf die stationäre 1,3-Butadien-Konzentration in der flüssigen Reaktionsmischung beziehen.

Die Addition des Alkohols ROH an 1,3-Butadien gemäß Teilreaktion a) des erfindungsgemäßen Verfahrens mit Hilfe der genannten Komplexkatalysatoren wird vorzugsweise in flüssiger Phase durchgeführt. Im allgemeinen wird der Katalysator gelöst im flüssigen Reaktionsmedium vorgelegt und 1,3-Butadien in flüssiger oder gasförmiger Form, zusammen mit dem Alkohol in die Reaktionsmischung eingeleitet. Als Reaktionsmedium kann der an 1,3-Butadien zu addierende Alkohol oder ein unter den Reaktionsbedingungen inertes Lösungsmittel, vorzugsweise ein hochsiedendes Lösungsmittel, dienen. Als Lösungsmittel eignen sich beispielsweise Kondensationsprodukte, die im Zuge der Umsetzung entstehen, wie Alkoxyoctadiene, Alkoxydodecatriene, ferner Ether, wie Dibutylether, Diethylenglykoldibutylether, niedermolekulare Polyethylenglykolether sowie Sulfone, wie Sulfolan.

Bei der diskontinuierlichen Ausgestaltung des Verfahrens wird die Umsetzung im allgemeinen in einem Rührautoklaven durchgeführt. Die dabei gebildeten Addukte der Formeln II und III werden anschließend zweckmäßigerweise aus der Reaktionsmischung destillativ abgetrennt, wobei der das Gruppe Ib-, VIIb- oder VIIIb-Element, insbesondere Palladium oder Nickel, enthaltende Homogenkatalysator im Sumpf der Destillation, gelöst im hochsiedenden Lösungsmittel, zurückbleibt. Die so im Destillationssumpf verbliebene Katalysatorlösung kann für weitere Umsetzungen wiederverwendet werden.

Bei der kontinuierlichen Ausgestaltung des Verfahrens wird das 1,3-Butadien vorzugsweise in flüssiger Form unter Druck in die den Alkohol ROH und den homogen gelösten Übergangsmetallelementkomplex-Katalysator sowie gegebenenfalls ein hochsiedendes Lösungsmittel enthaltende Reaktionsmischung eingespeist. Die Umsetzung wird vorteilhaft in einem Rohrreaktor oder vorzugsweise in einer Reaktorkaskade durchgeführt. Nicht umgesetztes 1,3-Butadien wird dabei vorteilhaft im Kreis geführt. Der Alkohol ROH wird vorteilhaft entsprechend seinem Verbrauch bei der Umsetzung der Reaktionsmischung kontinuierlich zudosiert.

Nach einer weiteren kontinuierlichen Ausgestaltung des erfindungsgemäßen Verfahrens kann das 1,3-Butadien gasförmig durch das den Katalysator enthaltende flüssige Reaktionsmedium geleitet werden, wobei nicht umgesetztes 1,3-Butadien dazu verwendet wird, die bei der Umsetzung mit dem Alkohol gebildeten, relativ leichtflüchtigen Addukte der Formeln II und III aus der Reaktionsmischung zu strippen. Der Alkohol ROH kann dabei, entsprechend seinem Verbrauch bei der Umsetzung, der Reaktionsmischung kontinuierlich zudosiert werden.

Die Addition des Alkohols ROH an 1,3-Butadien in Gegenwart der genannten Komplexe der Gruppe Ib-, VIIb- oder VIIIb-Elemente, insbesondere des Palladiums oder Nickels, wird im allgemeinen bei einer Temperatur von 20 bis 180°C, vorzugsweise von 50 bis 150°C und besonders bevorzugt von 80 bis 120°C und bei einem Druck von vorzugsweise 6 bis 10 bar und besonders bevorzugt unter Eigendruck durchgeführt.

Vorteilhaft können im erfindungsgemäßen Verfahren zur Addition des Alkohols ROH an 1,3-Butadien in Teilreaktion a) heterogenisierte Komplexkatalysatoren verwendet werden, vorzugsweise solche, in denen das Gruppe Ib-, VIIb- oder VIIIb-Element, insbesondere das Palladium oder Nickel, an polymere Matrizes fixiert ist. Solche polymeren Matrizes können Harze, wie Styrol-Divinylbenzol-Harze oder Phenol-Formaldehyd-Harze sein, an die die betreffenden Chelatliganden, also Phosphine, 1,10-Phenanthroline oder 2,2'-Bipyridine, gebunden sind, welche wiederum mit den Gruppe Ib-, VIIb- oder VIIIb-Elementen, insbesondere dem Palladium oder Nickel, Komplexe bilden und diese derart quasi immobilisieren. Als heterogene Matrizes zur Immobilisierung der Gruppe-Ib-, VIIb- oder Gruppe VIIIb-Element-Komplexe, insbesondere der Palladium- und Nickel-Komplexe, können auch anorganische Trägermaterialien, nach vorausgegangener Hydrophobisierung und chemischer Modifizierung ihrer Oberflächen mittels organischer Reagenzien dienen. Solche heterogenisierten, polymer gebundenen Gruppe Ib-, VIIb- oder Gruppe VIIIb-Element-Komplexe, insbesondere Palladium- und Nickel-Komplexe, sind beispielsweise nach dem Verfahren von Zhuangyu et al. (Reactive Polymers 9, 249 (1988)) erhältlich. Immobilisierte Phosphin-Komplexe der Gruppe Ib-, VIIb- und VIIIb-Elemente sind z.B. nach den Verfahren von Hartley, Adv. Organomet. Chem. 15, 189 (1977), F.R. Hartley "Supported Metal Complexes", Riedel, Dordrecht 1985, K. Smith, "Solid Supports and Catalysis in Organic Synthesis", Ellis Horwood, Prentice Hall, N.Y. 1992; C.H. Pittman "Polymer supported Reactions in Organic Synthesis, S. 249, Wiley, Chichester 1980 und C.H. Pittmann J. Am. Chem. Soc. 98, 5407 (1976) sowie Ann. N.Y. Acad. Sci. 245, 15 (1977) erhältlich. Der Vorteil der Anwendung solcher heterogenisierter Katalysatoren liegt insbesondere in der leichteren und schonenderen Abtrennbarkeit des Katalysators von den Reaktionsprodukten. Dieser kann in einem Festbett angeordnet und von der Reaktionsmischung durchströmt werden oder aber auch in der Reaktionsmischung suspendiert und nach beendeter Umsetzung, mechanisch abgetrennt werden.

Anstelle von reinem 1,3-Butadien können im erfindungsgemäßen Verfahren auch 1,3-Butadien-haltige Kohlenwasserstoffströme als Rohstoff eingesetzt werden. Derartige Kohlenwasserstoffströme fallen beispielsweise als sogenannter C₄-Schnitt in Steamcrackern an. Zweckmäßigerweise werden diese Kohlenwasserstoffströme vor ihrem Einsatz im erfindungsgemäßen Verfahren von gegebenenfalls darin enthaltenen acetylenischen oder allenischen Kohlenwasserstoffen durch deren partielle Hydrierung (Weissermel, Arpe: Industrielle Organische Chemie; 3. Auflage, VCH Verlagsgesellschaft, Weinheim 1988) befreit. Die 1,3-Butadien-haltigen Kohlenwasserstoffströme können sodann in analoger Weise wie reines 1,3-Butadien in Teilreaktion a) des erfindungsgemäßen Verfahrens eingetragen werden. Zweckmäßigerweise werden die in diesen Kohlenwasserstoffströmen enthaltenen gesättigten oder monoolefinischen Kohlenwasserstoffe, die bei der Umsetzung in Teilreaktion a) nicht reagiert haben, aus dem Reaktionsaustrag von Teilreaktion a) entfernt, beispielsweise mittels eines Gas-Flüssigkeit-Abscheiders. Die bei der Umsetzung dieser Kohlenwasserstoffströme in Teilreaktion a) des erfindungsgemäßen Verfahrens erhaltenen Addukte der Formeln II und III können, wie im folgenden beschrieben, auf die gleiche Weise zu n-Butyraldehyd und/oder n-Butanol weiterverarbeitet werden, wie die mit reinem 1,3-Butadien in Teilreaktion a) erzeugten Addukte II und III.

Der Reaktionsaustrag aus Teilreaktion a) des erfindungsgemäßen Verfahrens enthält im allgemeinen neben nicht umgesetztem 1,3-Butadien die Addukte der Formeln II und III sowie gegebenenfalls, insbesondere bei Anwendung von Brönsted-Säuren als Katalysatoren in Teilreaktion a), mehrere Isomere des betreffenden Alkoxyoctadiens, von denen im folgenden unter der Sammelbezeichnung Alkoxyoctadien gesprochen wird. Das Alkoxyoctadien entsteht bei der Addition des Alkohols ROH an 1,3-Butadien in einer Nebenreaktion, in der zunächst 1,3-Butadien zu Octatrien dimerisiert, an das sich nachfolgend der Alkohol ROH unter Bildung des Alkoxyoctadiens addiert. Neben diesen Bestandteilen kann der Reaktionsaustrag aus Teilreaktion a) noch geringe Mengen an anderen Nebenprodukten enthalten, beispielsweise Octatrien, Vinylcyclohexen, Alkoxydodecatriene, entstanden durch Trimerisierung des 1,3-Butadiens zu Dodecatetraen und nachfolgende Addition des Alkohols ROH, ferner Dodecatetraen, Dialkoxyocten und Dialkoxybutan. Die Bildung dieser Nebenprodukte kann durch die Art und Weise der Reaktionsführung in Teilreaktion a), beispielsweise durch die Wahl des 1,3-Butadien/Alkohol ROH-Verhältnisses in der Reaktionsmischung, die Wahl der Reaktionstemperatur und des Drucks beeinflußt und gewünschtenfalls minimiert werden.

Das zur Herstellung von n-Butyraldehyd und/oder n-Butanol im erfindungsgemäßen Verfahren benötigte Addukt ist das 1-Alkoxybuten-2 der Formel II, das für die Herstellung der Zielverbindungen im erfindungsgemäßen Verfahren von seinem im Reaktionsaustrag größenordnungsmäßig in der gleichen Menge enthaltenen Isomeren 3-Alkoxy-buten-1 der Formel III abgetrennt werden kann. Da die Addukte II und III bei der Addition des Alkohols ROH an 1,3-Butadien in etwa den gleichen Mengen gebildet werden, wäre das erfindungsgemäße Verfahren großtechnisch nicht wirtschaftlich, wenn es nicht gelänge, das 3-Alkoxy-buten-1 III in wirtschaftlicher Weise in das gewünschte 1-Alkoxy-buten-2 II umzuwandeln. Es wurde nun überraschenderweise gefunden, daß die Umwandlung des Addukts III in das gewünschte Addukt II auf einfache und wirtschaftliche Weise vollzogen werden kann.

Zu diesem Zweck wird das Addukt III zunächst aus dem im Reaktionsaustrag von Teilreaktion a) enthaltenen, isomeren Addukt II abgetrennt. Dies kann vorteilhaft so geschehen, daß der Reaktionsaustrag aus Teilreaktion a), nach vorheriger Abtrennung nicht umgesetzten 1,3-Butadiens, z.B in einem Gas-Flüssigkeit-Abscheider in eine Destillationsapparatur geleitet und darin durch fraktionierte Destillation abgetrennt wird.

Bei dieser fraktionierten Destillation können auch die im Reaktionsaustrag von Teilreaktion a) enthaltenen Nebenprodukte, 1,3-Butadien-Dimere und -Trimere sowie deren Addukte mit dem Alkohol ROH und gegebenenfalls mehrfach alkoxylierte Nebenprodukte vom Addukt II abgetrennt werden. Da diese Nebenprodukte sich im weiteren Gang des erfindungsgemäßen Verfahrens im allgemeinen nicht störend auswirken, kann deren Abtrennung auch unterbleiben. Es kann bei der Destillation auch so verfahren werden, daß man außer dem Addukt III nur einen Teil der Nebenprodukte, insbesondere die olefinischen 1,3-Butadien-Dimere und -Trimere sowie mehrfach alkoxylierte Nebenprodukte, abtrennt, hingegen andere Nebenprodukte, insbesondere das Alkoxyoctadien und gewünschtenfalls das Alkoxydodecatrien, gemeinsam mit dem Addukt II in den nachfolgenden Teilreaktionen weiterverarbeitet, wobei aus diesen Nebenprodukten der Teilreaktion a) als Endprodukte Octanole bzw. Dodecanole entstehen, die als Weichmacheralkohole begehrt sind.

Die destillative Abtrennung des leichter flüchtigen Addukts III vom Addukt II gelingt auf einfache Weise z.B. in herkömmlichen Destillationskolonnen. Das vom gewünschten Addukt abgetrennte Addukt III kann dann, ebenso wie das nicht umgesetzte 1,3-Butadien in die Verfahrensstufe der Teilreaktion a) des erfindungsgemäßen Verfahrens zurückgeführt werden. Die Rückführung des Addukts III in die Verfahrensstufe der Teilreaktion a) des erfindungsgemäßen Verfahrens bewirkt die Isomerisierung des Addukts III zum Addukt II in dieser Verfahrensstufe und führt letztendlich zur Unterdrückung der Neubildung des unerwünschten Addukts III, so daß bei Anwendung dieser Kreislauffahrweise in der Gesamtbilanz dieses Kreisprozesses praktisch nur das erwünschte Addukt II, nicht jedoch dessen unerwünschtes Isomer III gebildet wird.

Die Isomerisierung des Addukts III kann statt durch dessen Rückführung in die Verfahrensstufe der Teilreaktion a) des erfindungsgemäßen Verfahrens auch in einer separaten Isomerisierungsstufe bewerkstelligt werden, indem man das vom Addukt II abgetrennte Addukt III z.B. durch einen mit einem der in Teilreaktion a) anwendbaren Katalysatoren beschickten Reaktor leitet, den Austrag dieses Reaktors, der aus dem darin gebildeten Isomerisierungsgemisch aus Addukt III und Addukt II besteht, beispielsweise destillativ in Addukt II und Addukt III auftrennt, das neu gebildete Addukt II im weiteren Gang des erfindungsgemäßen Verfahrens zu n-Butyraldehyd und/oder n-Butanol weiterverarbeitet und das Addukt III wieder in den Isomerisierungsreaktor zurückführt.

Die Isomerisierung des Addukts III zu Addukt II im Isomerisierungsreaktor kann in An- oder Abwesenheit eines Lösungsmittels erfolgen. Vorzugsweise wird bei dieser Umsetzung ohne Lösungsmittel gearbeitet. Wird die Isomerisierung in Gegenwart eines Lösungsmittels durchgeführt, werden im allgemeinen hochsiedende Lösungsmittel wie Ether, beispielsweise Di- oder Triethylenglykoldimethylether, Di- oder Triethylenglykoldibutylether, Sulfoxide, z.B. Dimethylsulfoxid oder Sulfone, wie Sulfolan, hochsiedende aromatische oder aliphatische Kohlenwasserstoffe oder halogenierte aliphatische oder aromatische Lösungsmittel, z.B. Dichlorbenzol, eingesetzt. Die Verwendung niedrig siedender Lösungsmittel ist ebenfalls möglich, erfordert in der Regel aber einen erhöhten Aufwand bei der destillativen Auftrennung des Auftrags aus dem Isomerisierungsreaktor in die Addukte II und III.

Im weiteren Verlauf des erfindungsgemäßen Verfahrens zur Herstellung von n-Butyraldehyd und/oder n-Butanol wird das Addukt II in der Teilreaktion c) katalytisch zum Enolether der Formel IV isomerisiert, der anschließend in Teilreaktion d) katalytisch in Gegenwart von Wasser zu n-Butyraldehyd hydrolysiert und/oder katalytisch in Gegenwart von Wasser und Wasserstoff in n-Butanol umgewandelt wird. Die Teilreaktionen c) und d) können im erfindungsgemäßen Verfahren wahlweise sukzessive in zwei Verfahrensstufen oder sukzessive in einem einzelnen Reaktor oder besonders vorteilhaft einstufig, in einer einzigen Verfahrensstufe durchgeführt werden. Beide Teilreaktionen c) und d) können sowohl in der Gasphase als auch in flüssiger Phase stattfinden.

Wie erwähnt werden die Teilreaktionen c), die Isomerisierung des Addukts II zum Enolether IV und d), dessen Umsetzung mit Wasser oder Wasserstoff und Wasser zu n-Butyraldehyd und/oder n-Butanol besonders bevorzugt in einer einzigen Verfahrensstufe durchgeführt. Diese Verfahrensstufe umfaßt folglich die folgenden chemischen Umsetzungen gemäß Reaktionsgleichung (2)

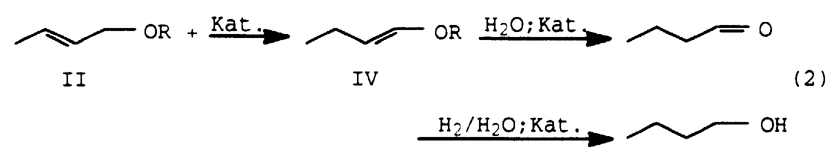

Der jeweils letzte Reaktionsschritt, d.h. die Hydrolyse des Enolethers IV zu n-Butyraldehyd einerseits oder die kombinierte Hydrolyse/Hydrierung des Enolethers IV zu n-Butanol andererseits, kann durch die Wahl der Reaktionsbedingungen, insbesondere durch die Wahl des Katalysators und die Wahl der bei der Umsetzung zur Verfügung gestellten Menge der Reaktanten Wasser und Wasserstoff, so gesteuert werden, daß wahlweise das Endprodukt n-Butyraldehyd oder n-Butanol selektiv gebildet wird oder daß Gemische dieser beiden Wertprodukte als Endprodukt des erfindungsgemäßen Verfahrens entstehen.

Es wurde überraschenderweise gefunden, daß die Katalysatoren, welche die Isomerisierung des Addukts II zum Enolether IV katalysieren, im allgemeinen auch gut als Katalysatoren für die Hydrolyse des Enolethers IV zu n-Butyraldehyd oder für die kombinierte Hydrolyse/Hydrierung des Enolethers IV zu n-Butanol geeignet sind. Dementsprechend können bei der besonders bevorzugten Ausführungsform des erfindungsgemäßen Verfahrens, also der Durchführung der Teilreaktionen c) und d) in einer einzigen Verfahrensstufe, sowohl für die Herstellung des Endprodukts n-Butyraldehyd als auch für die Herstellung des Endprodukts n-Butanol die gleichen Katalysatoren Anwendung finden.

Sowohl die Isomerisierung des Addukts II zum Enolether IV als auch die Hydrolyse des Enolethers IV zu n-Butyraldehyd bzw. die kombinierte Hydrolyse/Hydrierung des Enolethers IV zu n-Butanol können in der Gasphase oder in flüssiger Phase ausgeführt werden. Bei der Durchführung dieser Reaktionsschritte in einer einzigen Verfahrensstufe in flüssiger Phase können sowohl homogene als auch heterogene Katalysatoren angewandt werden. Wird in der Gasphase gearbeitet, werden im allgemeinen heterogene Katalysatoren bevorzugt.

Als Homogenkatalysatoren zur Isomerisierung des Addukts II zum Enolether IV und dessen Hydrolyse oder kombinierte Hydrolyse/ Hydrierung zu n-Butyraldehyd und/oder n-Butanol in einer einzigen Verfahrensstufe können eine Vielzahl von Übergangsmetallelementverbindungen eingesetzt werden, insbesondere solche, die Elemente aus der I., V., VI, VII. und VIII. Nebengruppe des Periodensystems der Elemente, vorzugsweise Kupfer, Vanadium, Chrom, Molybdän, Wolfram, Rhenium, Eisen, Kobalt, Nickel, Ruthenium, Rhodium, Palladium, Platin, Osmium und/oder Iridium enthalten.

Als Katalysatoren eignen sich beispielsweise die Salze dieser Übergangsmetalle, insbesondere deren im Reaktionsmedium löslichen Halogenide, Nitrate, Sulfate, Phosphate, Carboxylate, beispielsweise deren C₁- bis C₂₀-Carboxylate, wie Formiate, Acetate, Propionate, 2-Ethylhexanoate, ferner die Citrate, Tartrate, Malate, Malonate, Maleinate oder Fumarate, Sulfonate, beispielsweise Methansulfonate, Benzolsulfonate, Naphthalinsulfonate, Toluolsulfonate oder Trifluormethansulfonate, Cyanide, Tetrafluoroborate, Perchlorate oder Hexafluorophosphate, weiterhin lösliche Salze der Oxosäuren dieser Metalle, insbesondere die Alkalimetall-, Erdalkalimetall- oder Oniumsalze, wie Ammonium-, Phosphonium-, Arsonium- oder Stiboniumsalze, der Vanadiumsauerstoffsäuren, Rheniumsauerstoffsäuren oder der Perrheniumsäure oder die Anhydride dieser Säuren, insbesondere das Dirheniumheptoxid, lösliche anorganische Komplexverbindungen dieser Elemente, insbesondere deren Aquo-, Ammin-, Halogeno-, Phosphin-, Phosphit-, Cyano- oder Amino-Komplexe sowie die Komplexe dieser Übergangsmetalle mit Chelatbildnern, wie Acetylaceton, Dioximen, beispielsweise Diacetyldioxim, Furildioxim oder Benzildioxim, Ethylendiamintetraessigsäure, Nitrilotriessigsäure, Nitrilotriethanol, Harnstoffen oder Thioharnstoffen, Bisphosphinen, Bisphosphiten, Bipyridinen, Terpyridinen, Phenanthrolinen, 8-Hydroxychinolin, Kronenethern oder Polyalkylenglykolen, sowie Organometallverbindungen dieser Übergangsmetallelemente, beispielsweise Carbonylkomplexe wie HRuCl(CO) (PPh₃)₃, HRuCl(CO) (Hexyldiphenylphosphin)₃, RuH₂(CO) (PPh₃)₃, RuH₂(PPh)₃ oder IrCl(CO) (PPh₃)₃- die Abkürzung PPh₃ bedeutet Triphenylphosphin -, Fe₂(CO)₉ oder Fe₃(CO)₁₂, Organotrioxorhenium(VII)-Verbindungen wie C₁- bis C₄-Alkyltrioxorhenium(VII), insbesondere Methyltrioxorhenium(VII), Cyclopentadienyltrioxorhenium(VII) oder Phenyltrioxorhenium(VII).

Bevorzugte salzartige Homogenkatalysatoren sind die Halogenide, insbesondere die Chloride, Nitrate, Sulfate, Sulfonate, Carboxylate und Cyanide des Rhodiums, Rutheniums, Palladiums, Platins, Iridiums, Rheniums und Vanadiums sowie die Alkalimetall-, Erdalkalimetall-, Ammonium-, Alkylammonium-, Arylammonium-, Arylphosphonium- und Alkylphosphoniumsalze der Vanadiumsäuren, insbesondere deren Monovanadate, der Rheniumsäuren, insbesondere deren Rhenate (IV), Rhenate (VI) und Perrhenate.

Ein geeigneter Homogenkatalysator ist weiterhin das Dirheniumheptoxid (Re₂O₇).

Anorganische Komplexverbindungen, die bevorzugt im erfindungsgemäßen Verfahren zur Durchführung der Teilreaktionen c) und d) eingesetzt werden, sind z.B. Rutheniumtrichlorid, Rhodiumtrichlorid oder Iridiumhexaquoditosylat.

Übergangsorganometallelement-Verbindungen, die erfindungsgemäß bevorzugt als Homogenkatalysatoren zur Durchführung der Teilreaktionen c) und d) verwendet werden, sind z.B. Carbonylkomplexe, wie HRh(PPh₃)₃(CO), HRuCl(CO) (PPh₃)₃ oder RuCl₂(CO)₂(PPh₃)₃, sowie Organotrioxorhenium-Verbindungen der Formel V 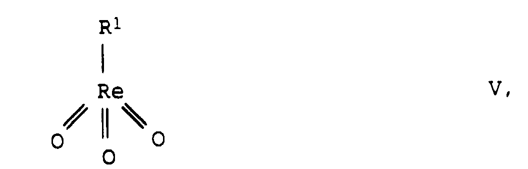 in der R¹ eine C₁- bis C₁₀-Alkylgruppe, eine unsubstituierte oder eine mit 1 bis 5 C₁- bis C₄-Alkylgruppen substituierte Cyclopentadienylgruppe, eine C₆- bis C₁₀-Aryl- oder eine C₇- bis C₁₁-Aralkylgruppe ist. Zur Herstellung dieser Organotrioxorhenium-Verbindungen wird auf die in Angew. Chem. 100, 420 (1988), Angew. Chem. 103, 183 (1991), J. Organomet. Chem. 297, C 5 (1985), Angew. Chem. 100, 1269 (1988) und J. Organomet. Chem. 382, 1 (1990) angegebenen Verfahren verwiesen.

Besonders bevorzugte Homogenkatalysatoren für die Durchführung der Teilreaktionen c) und d) in einer einzigen Verfahrensstufe sind Komplexe der genannten Übergangsmetallelemente, insbesondere des Kobalts, Nickels, Rhodiums, Rutheniums, Palladiums, Platins und Iridiums mit einzähnigen oder mehrzähnigen, insbesondere zweizähnigen, Phosphin- oder Phosphit-Liganden und/oder mit stickstoffhaltigen Liganden, für deren Eigenschaft als Komplexbildner die (-N=C-C=N-)-Struktureinheit ursächlich ist, beispielsweise 2,2'-Bipyridin oder 1,10-Phenanthrolin, sowie die durch Substitution oder Anellierung von diesen Grundkörpern abgeleiteten Liganden.

Geeignete Phosphin-Liganden sind beispielsweise die zur Durchführung der Teilreaktion a) des erfindungsgemäßen Verfahrens geeigneten und bei der Beschreibung dieser Teilreaktion in dieser Anmeldung genannten Phosphin-Liganden, auf die hiermit verwiesen wird. Geeignete 2,2'-Bipyridin- oder 1,10-Phenanthrolin-Liganden sind beispielsweise die zur Durchführung der Teilreaktion a) des erfindungsgemäßen Verfahrens geeigneten und bei der Beschreibung dieser Teilreaktion genannten 2,2'-Bipyridin- oder 1,10-Phenanthrolin-Liganden sowie deren am angegebenen Ort genannten Derivate und Strukturanaloge, auf die hiermit verwiesen wird.

Geeignete Phosphit-Liganden sind z.B. Trialkylphosphite, Alkyldiarylphosphite, Triarylphosphite, Alkyl-bis-phosphite, Aryl-bisphosphite, Alkyl-aryl-bis-phosphite. Die Alkylgruppen-tragenden Phosphit-Liganden können gleiche oder verschiedene C₁- bis C₁₀-, vorzugsweise C₁- bis C₆-Alkyl- oder Cycloalkylgruppen enthalten. Die Arylgruppen-tragenden Phosphit-Liganden können gleiche oder verschiedene C₆- bis C₁₂-Arylgruppen, insbesondere die Phenyl- oder Naphthylgruppe, aber auch die Diphenylgruppe enthalten. Weiterhin können Phosphit-Liganden zur Komplexierung der Übergangsmetalle eingesetzt werden, die heterocycloaliphatische Gruppen, wie Pyrrolidin-, Imidazolidin-, Piperidin-, Morpholin-, Oxazolidin-, Piperazin- oder Triazolidin-Gruppen oder heteroaromatische Gruppen, wie Pyrrol-, Imidazol-, Oxazol-, Indol-, Pyridin-, Chinolin-, Pyrimidin-, Pyrazol-, Pyrazin-, Pyridazin- oder Chinoxazolin-Gruppen gemeinsam mit anderen Alkyl- oder Aryl-Gruppen tragen. Die Alkyl- oder Arylgruppen der Phosphit-Liganden können unsubstituiert sein oder unter den Reaktionsbedingungen inerte Substituenten, wie C₁- bis C₄-Alkoxy-, Di-C₁- bis C₄-alkylamino-, C₁- bis C₆-Alkyl-, Hydroxy-, Nitro-, Cyano- oder Sulfonat-Gruppen tragen. Die Sulfonat-substituierten Phosphit-Liganden und deren Komplexe sind im allgemeinen wasserlöslich. Geeignete Phosphit-Liganden sind z.B. Trimethylphosphit, Triethylphosphit, Tripropylphosphit, Triisopropylphosphit, Tributylphosphit, Tricyclopentylphosphit, Tricyclohexylphosphit, Triphenylphosphit sowie die in EP-A 472 071, EP-A 213 639, EP-A 214 622, DE-A 2 733 796, EP-A 2261, EP-A 2821, EP-A 9115, EP-A 155 508, EP-A 353 770, US-A 4 318 845, US-A 4 204 997 und US-A 4 362 830 beschriebenen Mono- und Bisphosphit-Liganden.

Bei der Durchführung der Teilreaktionen c) und d) mit homogen im Reaktionsmedium löslichen Phosphin- oder Phosphit-Komplexen als Katalysatoren kann es sich als vorteilhaft erweisen, zusätzlich ein Phosphin oder Phosphit, vorzugsweise das im eingesetzten Homogenkatalysator als Ligand dienende Phosphin oder Phosphit, zur Reaktionsmischung zu geben. Ein solcher Zusatz kann eine Verlängerung der Standzeit des Homogenkatalysators bewirken und darüber hinaus die Selektivität der Isomerisierung des Addukts II zum Enolether IV als auch die Selektivität bei der kombinierten Hydrolyse/Hydrierung des Enolethers IV zu n-Butanol und somit letztendlich die Selektivität des Gesamtverfahrens verbessern. Einen ähnlichen vorteilhaften Effekt kann der Zusatz von Kohlenmonoxid zum Reaktionsgemisch auslösen, insbesondere bei Verwendung carbonylgruppenhaltiger Übergangsmetallelementkomplexe als Homogenkatalysatoren.

Obgleich zur Herstellung des Endproduktes n-Butyraldehyd ein Zusatz von Wasserstoff zur Reaktionsmischung nicht erforderlich ist, kann die Zuführung geringer Mengen Wasserstoff, gegebenenfalls zusammen mit der Zufuhr geringer Mengen von Kohlenmonoxid bei Verwendung carbonylgruppenhaltiger Homogenkatalysatoren, zu einer Verlängerung der Standzeit dieser Homogenkatalysatoren führen. Praktischerweise kann zu diesem Zweck Synthesegas eingesetzt werden.

Zur Erzielung der obengenannten Wirkungen wird im allgemeinen das Phosphin oder Phosphit bezüglich des Phosphin- oder Phosphit-Komplexes des Übergangsmetallelementes in einer 2 bis 100 molaren, vorzugsweise in einer 2 bis 20 molaren und besonders bevorzugt in einer 2 bis 5 molaren Menge zugesetzt. Wird der als Homogenkatalysator dienende Übergangsmetallelementkomplex in situ im Reaktionsgemisch erzeugt, so setzt man zweckmäßigerweise einen entsprechend hohen Überschuß an Phosphin- oder Phosphit-Ligand bezüglich des betreffenden Übergangsmetallelementes ein.

Die homogen im Reaktionsmedium löslichen Übergangsmetallkatalysatoren werden im allgemeinen bezüglich des dem Reaktor zugeführten Addukts II in Mengen von vorzugsweise 0,05 bis 0,2 mol-% eingesetzt. Es versteht sich für den Fachmann von selbst, daß die zuzugebende Menge an Homogenkatalysator von der katalytischen Aktivität des jeweils verwendeten Homogenkatalysators abhängig ist. Je nach Art des eingesetzten Homogenkatalysators kann somit vorteilhaft auch eine höhere oder geringere Katalysatormenge der Reaktionsmischung zugesetzt werden. Zweckmäßigerweise wird die optimale Menge für den jeweils verwendeten Homogenkatalysator in einem Vorversuch ermittelt.

Die Durchführung der Teilreaktionen c) und d) in einer einzigen Verfahrensstufe mit Hilfe der genannten Homogenkatalysatoren kann diskontinuierlich, z.B. in Rührkesseln oder kontinuierlich, z.B. in Rohrreaktoren, bei Temperaturen von im allgemeinen mehr als 80°C und bei einem Druck von im allgemeinen 5 bis 100 bar, vorzugsweise von 10 bis 60 bar vorgenommen werden. Die Isomerisierung des Addukts II zum Enolether IV und dessen Umsetzung zu n-Butyraldehyd und/oder n-Butanol in einer einzigen Verfahrensstufe kann in An- oder Abwesenheit von zugesetzten Lösungsmitteln, wie aliphatischen oder aromatischen Kohlenwasserstoffen, z.B. Toluol, Benzol oder Cyclohexan, Alkoholen, z.B. Butanole, insbesondere n-Butanol, höhere Fettalkohole oder Glykole, Ethern, z.B. Dibutylether, Tetrahydrofuran, Dioxan oder niedermolekulare Polyalkylenglykole, halogenierten aliphatischen oder aromatischen Kohlenwasserstoffen, z.B. Chloroform, Dichlormethan, Chlorbenzol, Dichlorbenzol, Sulfoxiden oder Sulfonen, z.B. Dimethylsulfoxid oder Sulfolan erfolgen.

Statt in diesen herkömmlichen Lösungsmitteln kann die Isomerisierung des Addukts II zum Enolether IV und dessen Umsetzung zu n-Butyraldehyd und/oder n-Butanol auch in einer Phosphin-Schmelze erfolgen. Vorteilhaft kann diese Verfahrensweise bei Phosphin-haltigen Homogenkatalysatoren angewandt werden. Das quasi als Lösungsmittel dienende Phosphin kann dabei im allgemeinen prinzipiell beliebig gewählt werden, vorzugsweise wird jedoch dasjenige Phosphin in der Schmelze verwendet, das im als Homogenkatalysator dienenden Übergangsmetallelementkomplex als Ligand dient.

Werden bei der einstufigen Umsetzung des Addukts II zu den Endprodukten n-Butyraldehyd und/oder n-Butanol keine weiteren Lösungsmittel zugesetzt, so bewirken die Reaktanten selbst, also das Addukt II, der Enolether IV und das erfindungsgemäß zur Hydrolyse des Enolethers IV eingesetzte Wasser, als auch die Endprodukte der Umsetzung, die Lösung der erfindungsgemäß eingesetzten Homogenkatalysatoren.

Zur Herstellung der Endprodukte n-Butyraldehyd und n-Butanol wird der Reaktionsmischung Wasser in einem Molverhältnis, bezogen auf das dem Reaktor zugeführte Addukt II, von im allgemeinen 1:1 bis 100:1, vorzugsweise 2:1 bis 20:1 und besonders bevorzugt von 5:1 bis 10:1 zugesetzt. Bei der diskontinuierlichen Betriebsweise kann das Wasser gemeinsam mit den anderen Reaktanten, dem Addukt II und dem Homogenkatalysator, im Reaktor vorgelegt werden, es kann aber auch vorteilhaft sein, das Wasser erst nach dem Beginn der Umsetzung in den Reaktor zu dosieren. Welche dieser Verfahrensweisen letztlich gewählt wird, hängt vom jeweils verwendeten Katalysator und den angewandten Druck- und Temperaturbedingungen ab. Zweckmäßigerweise wird die optimale Verfahrensweise für den jeweils verwendeten Katalysator in einem Vorversuch ermittelt. In analoger Weise kann bei der kontinuierlichen Ausgestaltung des Verfahrens, z.B. in einem Rohr- oder Kaskadenreaktor, das Wasser gemeinsam mit den übrigen Reaktanten in den Reaktor geleitet oder aber erst nach einer gewissen Verweilzeit der Reaktanten im Reaktor, über einen separaten Einlaß in den Reaktor dosiert werden.

Ist das gewünschte Endprodukt n-Butanol, wird der Reaktionsmischung, zusätzlich zu dem für die Hydrolyse des Enolethers IV benötigten Wasser noch Wasserstoff in einem Molverhältnis, bezogen auf das dem Reaktor zugeführte Addukt II, von im allgemeinen 1:1 bis 100:1, vorzugsweise von 1:1 bis 10:1 und besonders bevorzugt von 1:1 bis 2:1 zugemischt. Diese Zumischung kann bei diskontinuierlicher Betriebsweise des Verfahrens durch Einpressen der erforderlichen Wasserstoffmenge in den Reaktor oder durch Dispergieren des Wasserstoffs im Reaktionsmedium, beispielsweise mittels Blasensäulen oder mittels mit Strahldüsen zur Dispergierung des Wasserstoffs ausgestatteten Schlaufenreaktoren erfolgen. Die Zumischung des Wasserstoffs kann bei der Beschickung des Reaktors mit den übrigen Reaktanten, also dem Addukt II, dem Wasser und dem Homogenkatalysator, erfolgen, der Wasserstoff kann aber auch vorteilhaft nachträglich, nach dem Beginn der Umsetzung, in die Reaktionsapparatur eingespeist werden. Welche dieser Verfahrensweisen letztlich gewählt wird, hängt vom verwendeten Katalysator und den jeweils angewandten Druck- und Temperaturbedingungen sowie von der Konstruktion des Reaktors ab. Zweckmäßigerweise wird die optimale Verfahrensweise in einem Vorversuch ermittelt. In analoger Weise kann bei der kontinuierlichen Ausgestaltung des Verfahrens, z.B. in einem Rohrreaktor, einem Blasensäulenreaktor oder einer Füllkörperkolonne, der Wasserstoff gemeinsam mit den übrigen Reaktanten in den Reaktor eingebracht oder aber erst nach einer gewissen Verweilzeit der Reaktanten im Reaktor, über einen separaten Einlaß in diesen eingeleitet werden.

Ist das gewünschte Endprodukt eine Mischung aus n-Butanol und n-Butyraldehyd, so kann das Mengenverhältnis dieser Produkte in der Produktmischung beispielsweise über den Zusatz des Wasserstoffs und/oder die angewandte Reaktionstemperatur eingestellt werden. Werden unterstöchiometrische Mengen an Wasserstoff eingesetzt, wird selbstverständlich nur ein Teil des Ausgangsmaterials zu n-Butanol hydriert und durch Anwendung einer niedrigeren Reaktionstemperatur kann die Geschwindigkeit der Hydrierreaktion so weit verlangsamt werden, daß nur ein Teil des Ausgangsmaterials zu n-Butanol hydriert wird.

Nach beendeter Umsetzung wird das Reaktionsprodukt im allgemeinen destillativ aufgearbeitet, wobei der verwendete Homogenkatalysator aus dem Sumpf der Destillation zurückgewonnen und gewünschtenfalls erneut verwendet werden kann, beispielsweise durch Rückführung der Katalysatorlösung in die Verfahrensstufe der Isomerisierung des Addukts II zum Enolether IV und dessen Hydrolyse und Hydrierung. Ist die Rückführung des Katalysators im erfindungsgemäßen Verfahren erwünscht, kann dem Reaktionsgemisch zweckmäßigerweise noch ein Lösungsmittel, vorzugsweise ein Lösungsmittel, das bei einer höheren Temperatur als die Reaktionsprodukte n-Butanol und n-Butyraldehyd siedet, zugefügt werden. Ist der verwendete Homogenkatalysator unter den Bedingungen der Destillation chemisch und thermisch stabil, kann vom Zusatz eines hochsiedenden Lösungsmittels abgesehen und der Homogenkatalysator in fester Form wieder in die Umsetzung zurückgeführt werden. Bei der destillativen Aufarbeitung wird weiterhin das Reaktionsprodukt n-Butyraldehyd und/oder n-Butanol von dem in der vorausgegangenen Verfahrensstufe aus dem Enolether IV durch Hydrolyse oder Hydrierung freigesetzten Alkohol ROH I abgetrennt, der wiederum in die erste Verfahrensstufe des erfindungsgemäßen Verfahrens, die Addition des Alkohols ROH I an 1,3-Butadien, zurückgeführt wird. Als wertvolle Nebenprodukte des erfindungsgemäßen Verfahrens können bei der destillativen Aufarbeitung des Reaktionsproduktes, die als Folge der teilweisen Dimerisierung und Trimerisierung des Butadiens entstandenen Octanole oder Dodecanole bzw. die diesen Alkoholen entsprechenden Aldehyde, gewonnen werden.

In einer besonders bevorzugten Ausgestaltung des erfindungsgemäßen Verfahrens wird die Isomerisierung des Addukts II zum Enolether IV und dessen Hydrolyse oder Hydrierung zu n-Butyraldehyd und/oder n-Butanol in einer einzigen Verfahrensstufe unter Verwendung eines heterogenen Katalysators ausgeführt, wobei das Verfahren wahlweise in flüssiger Phase oder in der Gasphase durchgeführt werden kann.

Es wurde überraschenderweise gefunden, daß als Katalysatoren sowohl für die Isomerisierung des Addukts II zum Enolether IV als auch als auch für die Hydrolyse des Enolethers IV zu n-Butyraldehyd oder für die kombinierte Hydrolyse/Hydrierung des Enolethers IV zu n-Butanol an sich gängige heterogene Hydrierkatalysatoren, die im Reaktionsmedium im wesentlichen unlöslich sind, verwendet werden können. Von diesen Hydrierkatalysatoren sind solche bevorzugt, die ein oder mehrere Elemente der Gruppe Ib, VIb, VIIb und VIIIb, gegebenenfalls in Kombination mit einem oder mehreren Elementen der Gruppe Vb, des Periodensystems der Elemente, insbesondere Kupfer, Chrom, Molybdän, Wolfram, Rhenium, Ruthenium, Kobalt, Nickel, Rhodium, Iridium, Palladium und/oder Platin, gegebenenfalls in Kombination mit Eisen, enthalten.

Besonders aktive Hydrierkatalysatoren wie Nickel oder die Platinmetalle können vorteilhaft mit Hauptgruppenelementen, die als Katalysatorgift wirken, dotiert und auf diese Weise partiell vergiftet werden. Durch diese Maßnahme kann eine höhere Selektivität bei der kombinierten Hydrolyse/Hydrierung des Enolethers IV zu n-Butanol erzielt werden. Geeignete Hauptgruppenelemente sind z.B. die Chalcogene, wie Schwefel, Selen und Tellur, sowie die Elemente Phosphor, Arsen, Antimon, Wismut, Zinn, Blei und Thallium.

Im erfindungsgemäßen Verfahren können als Heterogenkatalysatoren z.B. sogenannte Fällungskatalysatoren verwendet werden. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder -Carbonat-Lösungen, als z.B. schwerlösliche Hydroxyde, Oxidhydrate, basische Salze oder Carbonate ausfällt, die erhaltenen Niederschläge anschließend trocknet und diese dann durch Calcinierung bei im allgemeinen 300 bis 700°C, insbesondere 400 bis 600°C in die betreffenden Oxide, Mischoxide und/oder gemischtvalentigen Oxide umwandelt, welche z.B. durch eine Behandlung mit Reduktionsmitteln, wie Wasserstoff oder Wasserstoff enthaltenden Gasen, bei in der Regel 50 bis 700°C, insbesondere bei 100 bis 400°C, zu den betreffenden Metallen und/oder zu oxidischen Verbindungen niedriger Oxidationsstufe reduziert und in die eigentliche, katalytisch aktive Form überführt werden. Dabei wird in der Regel solange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden.

Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Außer den obengenannten Fällungskatalysatoren, welche außer den katalytisch aktiven Komponenten noch zusätzlich ein Trägermaterial enthalten, eignen sich für das erfindungsgemäße Verfahren im allgemeinen solche Trägerkatalysatoren, bei denen die katalytisch wirkenden Komponenten z.B. durch Imprägnierung auf ein Trägermaterial aufgebracht worden sind.

Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diese Trägermaterialien z.B. durch Tränkung mit Lösungen oder Suspensionen der Salze oder Oxide betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Hilfe von Wasserstoff, Wasserstoff enthaltenden Gasen oder Hydrazin, aufgebracht werden. Eine andere Möglichkeit zur Aufbringung der katalytisch aktiven Metalle auf diese Träger besteht darin, die Träger mit Lösungen thermisch leicht zersetzbarer Salze, z.B. mit Nitraten oder mit thermisch leicht zersetzbaren Komplexverbindungen, z.B. Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600°C zu erhitzen. Diese thermische Zersetzung wird vorzugsweise unter einer Schutzgasatmosphäre vorgenommen. Geeignete Schutzgase sind z.B. Stickstoff, Kohlendioxid, Wasserstoff oder die Edelgase. Weiterhin können die katalytisch aktiven Metalle auf dem Katalysatorträger durch Aufdampfung oder durch Flammspritzen abgeschieden werden.

Der Gehalt dieser Trägerkatalysatoren an den katalytisch aktiven Metallen ist prinzipiell für das Gelingen des erfindungsgemäßen Verfahrens nicht kritisch. Es versteht sich für den Fachmann von selbst, daß höhere Gehalte dieser Trägerkatalysatoren an katalytisch aktiven Metallen zu hoheren Raum-Zeit-Umsätzen führen als niedrigere Gehalte. Im allgemeinen werden aber Trägerkatalysatoren verwendet, deren Gehalt an katalytisch aktiven Metallen 0,1 bis 80 Gew.-%, vorzugsweise 0,5 bis 30 Gew.-%, bezogen auf den gesamten Katalysator beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben aber auch unter- oder überschritten werden, ohne daß sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach den Verfahren von DE-A 25 19 817, EP-A 147 219 und EP-A 285 420 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränkung auf einem Träger abgeschiedenen Salze oder Komplexe der zuvor genannten Metalle, erzeugt werden.

Die Aktivierung der Fällungskatalysatoren als auch der Trägerkatalysatoren kann auch in situ in der Reaktionsmischung durch den dort anwesenden Wasserstoff erfolgen, bevorzugt werden diese Katalysatoren jedoch vor ihrer Verwendung im erfindungsgemäßen Verfahren aktiviert.

Als Trägermaterialien können im allgemeinen die Oxide des Aluminiums und Titans, Zirkoniumdioxid, Siliziumdioxid, Kieselgur, Kieselgel, Tonerden, z.B. Montmorillonite, Silikate, wie Magnesium- oder Aluminiumsilikate, Zeolithe, wie ZSM-5 oder ZSM-10-Zeolithe sowie Aktivkohle verwendet werden. Bevorzugte Trägermaterialien sind Aluminiumoxide, Titandioxide, Zirkoniumdioxid und Aktivkohle. Selbstverständlich können auch Mischungen verschiedener Trägermaterialien als Träger für im erfindungsgemäßen Verfahren anwendbare Katalysatoren dienen.

Als für die Durchführung der Teilreaktionen c) und d) in einer einzigen Verfahrensstufe einsetzbare Heterogenkatalysatoren seien die folgenden Katalysatoren beispielhaft genannt: Platindioxid, Palladium auf Aluminiumoxid, Palladium auf Siliziumdioxid, Palladium auf Bariumsulfat, Rhodium auf Aktivkohle, Rhodium auf Aluminiumoxid, Ruthenium auf Siliziumdioxid oder Aktivkohle, Nickel auf Siliziumdioxid, Kobalt auf Siliziumdioxid, Kobalt auf Aluminiumoxid, Carbonyleisenpulver, Rheniumschwarz, Raney-Rhenium, Rhenium auf Aktivkohle, Rhenium-Palladium auf Aktivkohle, Rhenium-Platin auf Aktivkohle, Kupfer auf Siliziumdioxid, Kupfer auf Aluminiumoxid, Kupfer auf Aktivkohle, Kupfer auf Kieselgur, Kupfer auf Kieselgel, Kupfer auf Titandioxid, Kupfer auf Zirkoniumdioxid, Kupfer auf Magnesiumsilikat, Kupfer auf Aluminiumsilikat, Kupfer auf Montmorillonit, Kupfer auf Zeolith, Raney-Kupfer, Platinoxid-Rhodiumoxid-Mischungen, Platin-Palladium auf Aktivkohle, Kupferchromit, Bariumchromit, Nickel-Chromoxid auf Aluminiumoxid, Dirheniumheptoxid (Re₂O₇), Kobaltsulfid, Nickelsulfid, Molybdän(VI)sulfid, Kupfer-Molybdän(VI)oxid-Siliziumdioxid-Aluminiumoxid-Katalysatoren, mit Selen oder Blei partiell vergiftete Palladium auf Aktivkohle-Katalysatoren sowie die Katalysatoren gemäß DE-A 39 32 332, US-A 3 449 445, EP-A 44 444, EP-A 147 219, DE-A 39 04 083, DE-A 23 21 101, EP-A 415 202, DE-A 23 66 264 und EP-A 100 406.

Vorteilhaft können im erfindungsgemäßen Verfahren auch Hydrierkatalysatoren verwendet werden, die Brönsted- und/oder Lewisacide-Zentren enthalten.

Als Brönsted- oder Lewis-saure Zentren können z.B. die katalytisch aktiven Metalle selber wirken, wenn diese bei der Aktivierung des Katalysators mit Wasserstoff oder Wasserstoffenthaltenden Gasen nicht vollständig zu den betreffenden Metallen reduziert werden. Dies gilt z.B. für die rhenium- und chromithaltigen Katalysatoren, wie Rheniumträgerkatalysatoren und Kupferchromit. In den Rheniumträgerkatalysatoren liegt das Rhenium als Mischung von Rheniummetall mit Rheniumverbindungen in höheren Oxidationsstufen vor, wobei letztere Wirkungen wie Lewis- oder Brönsted-Säuren entfalten können. Weiterhin können solche Lewis- oder Brönsted-aciden Zentren über das verwendete Trägermaterial in den Katalysator eingebracht werden. Als Lewis- oder Brönsted-acide Zentren enthaltende Trägermaterialien seien z.B. die Aluminiumoxide, Titandioxide, Zirkoniumdioxid, Siliziumdioxid, die Silikate, Tonerden, Zeolithe und Aktivkohle genannt.

Besonders bevorzugt werden deshalb im erfindungsgemäßen Verfahren als Hydrierkatalysatoren Trägerkatalysatoren verwendet, die Elemente der I., VI., VII. und/oder VIII. Nebengruppe des Periodensystems der Elemente, insbesondere der Elemente der I., VII. und VIII. Nebengruppe des Periodensystems der Elemente auf einem Brönsted- oder Lewis-acide wirkenden Trägermaterial abgeschieden enthalten. Besonders vorteilhafte Katalysatoren sind z.B. Rhenium auf Aktivkohle, Rhenium auf Zirkoniumdioxid, Rhenium auf Titandioxid, Rhenium auf Siliziumdioxid, Kupfer auf Aktivkohle, Kupfer auf Siliziumdioxid, Kupfer auf Kieselgur, Kupfer auf Kieselgel, Kupfer auf Titandioxid, Kupfer auf Zirkoniumdioxid, Kupfer auf Magnesiumsilikat, Kupfer auf Aluminiumsilikat, Kupfer auf Bleicherde, Kupfer auf Zeolith, Ruthenium auf Aktivkohle, Ruthenium auf Aluminiumoxid, Ruthenium auf Siliziumdioxid, Ruthenium auf Magnesiumoxid, Ruthenium auf Zirkoniumdioxid, Ruthenium auf Titandioxid und mit Selen oder Blei partiell vergiftete Palladium auf Aktivkohle-Katalysatoren.

Hydrierkatalysatoren, welche selbst keine derartigen Brönsted- oder Lewis-aciden Zentren haben, können Lewis- oder Brönstedacide-Komponenten, wie Zeolithe, Aluminium- oder Siliziumoxide, Phosphorsäure oder Schwefelsäure, zugesetzt werden. Sie werden im allgemeinen in Mengen von 0,01 bis 5 Gew.-%, vorzugsweise von 0,05 bis 0,5 Gew.-% und besonders bevorzugt von 0,1 bis 0,4 Gew.-%, bezogen auf das Gewicht des eingesetzten Katalysators, zugesetzt.

Weiterhin sind zur Isomerisierung des Addukts II zum Enolether IV und dessen Hydrolyse oder Hydrierung zu n-Butyraldehyd und/oder n-Butanol in einer einzigen Verfahrensstufe Heterogenkatalysatoren geeignet, welche die zur homogenen Katalyse dieser Verfahrensstufe verwendbaren Komplexverbindungen von Übergangsmetallelementen aus der Gruppe Ib, VIb, VIIb und VIIIb des Periodensystems der Elemente in heterogenisierter Form enthalten, beispielsweise solche, in denen das betreffende Übergangsmetallelement an eine polymere Matrix fixiert ist.

Solche polymeren Matrizes können Harze, wie Styrol-Divinylbenzol-Harze oder Phenol-Formaldehyd-Harze sein, an die die betreffenden zur Komplexierung des Übergangsmetallelements dienenden Liganden vorzugsweise kovalent gebunden sind, welche wiederum mit den betreffenden Übergangsmetallen Komplexe bilden und diese derart quasi immobilisieren. Derartige heterogenisierte, polymer gebundene Übergangsmetallkomplex-Katalysatoren mit 2,2'-Bipyridin- oder 1,10-Phenanthrolin-Liganden oder heterogenisierte Phosphin- oder Phosphitkomplexe der katalytisch aktiven Übergangsmetallelemente können z.B. nach den bei der Erläuterung von Teilreaktion a) für die Herstellung dieser Katalysatoren genannten Literaturverfahren hergestellt werden. Organotrioxorhenium(VII)-Katalysatoren können z.B. nach dem Verfahren von DE-A 39 02 357 an stickstoffhaltige Polymere, wie Polyvinylpyrrolidon, Poly(2-vinylpyridin), (2-Vinylpyridin-)Styrol-Copolymere, Polyacrylsäureamide, Polyimide, Polyamide und Polyurethane durch koordinative Bindung fixiert und auf diese Weise heterogenisiert, als Heterogenkatalysator erfindungsgemäß verwendet werden.

Mit den genannten Heterogenkatalysatoren kann die Isomerisierung des Addukts II zum Enolether IV und dessen Hydrolyse oder Hydrierung zu n-Butyraldehyd und/oder n-Butanol in einer einzigen Verfahrensstufe sowohl kontinuierlich als auch diskontinuierlich durchgeführt werden.

Wird diese Umsetzung in flüssiger Phase durchgeführt, so kann der Heterogenkatalysator sowohl im flüssigen Reaktionsmedium suspendiert oder vorzugsweise in einem Festbett oder mehreren Festbetten angeordnet, eingesetzt werden. Das Verfahren kann bei Einsatz eines im flüssigen Reaktionsmedium suspendierten Heterogenkatalysators z.B. in Rührkesseln oder Schlaufenreaktoren durchgeführt werden. Bei Anwendung eines in einem Festbett angeordneten Heterogenkatalysators wird das Reaktionsgut im allgemeinen in der Sumpf- oder Rieselfahrweise über das Katalysatorfestbett geleitet.

Sowohl die Hydrierung des Enolethers IV als auch dessen Hydrolyse oder Hydrierung können in adiabatisch oder isotherm betriebenen Reaktoren vorgenommen werden. Im allgemeinen wird hierbei der Katalysator mit der flüssigen Reaktionsmischung mit einer Raumgeschwindigkeit von 0,01 bis 10, vorzugsweise von 0,05 bis 6 und besonders bevorzugt von 0,08 bis 3 kg Ether/1 Katalysator·h belastet. Bei Anwendung der Heterogenkatalysatoren kann die Umsetzung in An- oder Abwesenheit eines Lösungsmittels erfolgen. Als Lösungsmittel können die gleichen Lösungsmittel Anwendung finden, welche auch bei der Durchführung des Verfahrens unter homogener Katalyse verwendet werden können.

Wie bei der Durchführung der Teilreaktionen c) und d) des erfindungsgemäßen Verfahrens unter homogener Katalyse bereits beschrieben wurde, kann das zur Herstellung der Endprodukte n-Butyraldehyd und/oder n-Butanol benötigte Wasser entweder gemeinsam mit dem Addukt II dem Reaktor zugeführt und/oder über separate Zuleitungen, in einen oder in mehrere Teilströme aufgeteilt, an verschiedenen Stellen in die Katalysatorschüttung eingespeist werden. Gleiches gilt für die Zufuhr von Wasser und Wasserstoff bei der Herstellung des Endprodukts n-Butanol.

Das zur Herstellung von n-Butyraldehyd benötigte Wasser wird bei Durchführung des Verfahrens unter heterogener Katalyse bezüglich des dem Reaktor zugeführten Addukts II in einem Molverhältnis von im allgemeinen 1 bis 100, vorzugsweise von 1 bis 70 und besonders bevorzugt von 1 bis 30 zugesetzt. Die kombinierte Isomerisierung des Addukts II zum Enolether IV und dessen Hydrolyse zu n-Butyraldehyd in einer einzigen Verfahrensstufe am Heterogenkatalysator in flüssiger Phase wird im allgemeinen bei einer Temperatur von 20 bis 400°C, vorzugsweise von 30 bis 350°C und besonders bevorzugt von 80 bis 300°C und bei einem Druck von im allgemeinen 1 bis 300 bar, vorzugsweise von 2 bis 150 bar, insbesondere von 5 bis 100 bar durchgeführt.

Der zur Herstellung von n-Butanol zusätzlich zum Wasser benötigte Wasserstoff wird bei der Durchführung des Verfahrens unter heterogener Katalyse bezüglich des dem Reaktor zugeführten Addukts II in einem Molverhältnis von im allgemeinen 1 bis 100, vorzugsweise von 1,5 bis 80, insbesondere von 2 bis 40 zugesetzt. Die kombinierte Isomerisierung des Addukts II zum Enolether IV und dessen Hydrolyse/Hydrierung zu n-Butanol in einer einzigen Verfahrensstufe am Heterogenkatalysator in flüssiger Phase wird im allgemeinen bei einer Temperatur von 20 bis 400°C, vorzugsweise von 30 bis 350°C und besonders bevorzugt von 80 bis 300°C und bei einem Druck von im allgemeinen 1 bis 300 bar, vorzugsweise von 5 bis 250 bar, insbesondere von 20 bis 200 bar ausgeführt. Es versteht sich von selbst, daß die zur Herstellung von n-Butanol aus dem Addukt II benötigte Wassermenge gleich der zur Herstellung von n-Butyraldehyd aus dem Addukt II benötigten Wassermenge ist.

Ist das gewünschte Endprodukt eine Mischung aus n-Butyraldehyd und n-Butanol, werden im allgemeinen Wasser und Wasserstoff bezüglich des in den Reaktor eingebrachten Addukts II in analoger Weise wie zuvor beschrieben, in einem Mengenverhältnis zugemischt, das die Gewinnung beider Endprodukte im gewünschten Produktverhältnis ermöglicht. Darüber hinaus läßt sich das Produktverhältnis dieser beiden Endprodukte im Reaktoraustrag auch über die Verwendung bestimmter Heterogenkatalysatoren steuern, beispielsweise indem man Heterogenkatalysatoren einsetzt, die über eine hohe Hydrolyseaktivität und eine verglichen damit, relativ geringe Hydrieraktivität verfügen. Zu diesem Zweck können z.B. bezüglich ihrer Hydriereigenschaften inaktivierte oder partiell vergiftete Katalysatoren, z.B. mit Selen oder Blei partiell vergiftete Palladium auf Aktivkohle-Katalysatoren vorteilhaft angewandt werden.

Der flüssige Reaktionsaustrag aus dieser Verfahrensstufe wird im allgemeinen destillativ, in analoger Weise, wie es für die Durchführung dieser Verfahrensstufe mit Homogenkatalysatoren bereits beschrieben wurde, aufgearbeitet. Naturgemäß entfällt bei der Anwendung von Heterogenkatalysatoren die Rückführung des Katalysators, wie sie unter Umständen bei Anwendung von Homogenkatalysatoren zweckmäßig und vorteilhaft ist. Die Rückführung des in dieser Verfahrensstufe wieder freigesetzten Alkohols ROH I in die Verfahrensstufe der Addition des Alkohols ROH I an 1,3-Butadien kann vorteilhaft auf analoge Weise, wie für die Umsetzung dieser Verfahrensstufe mit Homogenkatalysatoren bereits beschrieben, geschehen.

Wie bereits erwähnt, kann die Isomerisierung des Addukts II zum Enolether IV und dessen Hydrolyse oder Hydrierung zu n-Butyraldehyd und/oder n-Butanol in einer einzigen Verfahrensstufe vorteilhaft in der Gasphase durchgeführt werden. Hierzu können an sich übliche Reaktoren für Gasphasenreaktionen verwendet werden, beispielsweise solche, in denen der Katalysator in einem Festbett oder in einer Wirbelschicht angeordnet ist. Die Reaktoren können adiabatisch oder isotherm betrieben werden. Bei Anwendung einer Festbettanordnung des Katalysators kann der Katalysator in einem oder vorteilhaft zwecks Verbesserung der Abführung der Reaktionswärme in mehreren, beispielsweise 2 bis 10, vorzugsweise 2 bis 5 Festbetten, angeordnet sein. Bei Verwendung mehrerer Katalysatorfestbette oder bei adiabatischer Betriebsweise des Reaktors kann es vorteilhaft sein, durch eine Zwischenbettkühlung des Reaktionsgases und/oder durch Eindüsen zusätzlicher Mengen an kühlen Reaktanten wie Wasserstoff, Wasser, Addukt II oder Enolether IV zwischen den einzelnen Festbetten, die Temperatur des Reaktionsgases nach Verlassen des vorhergehenden Festbetts und vor Eintritt in das nachfolgende Festbett abzusenken, um die Selektivität der Umsetzung zu erhöhen. Zweckmäßigerweise wird bei Anwendung mehrerer Festbette in den einzelnen Festbetten vor dem letzten Festbett die Umsetzung nur bis zu einem Teilumsatz, beispielsweise bis zu einem Umsatz von 50 bis 98 %, durchgeführt. Die Reaktionsgase können gewünschtenfalls mit einem unter den Reaktionsbedingungen inerten Gas, wie Stickstoff, gesättigten Kohlenwasserstoffen oder Argon, verdünnt werden.

Das zur Herstellung des Endprodukts n-Butyraldehyd benötigte Wasser wird bei der Durchführung des Verfahrens in der Gasphase bezüglich des dem Reaktor zugeführten Addukts II in einem Molverhältnis von im allgemeinen 1 bis 100, vorzugsweise von 1 bis 70 und besonders bevorzugt von 1 bis 30 zugesetzt. Das Wasser kann dem Reaktor gemeinsam mit dem Addukt II und/oder, wie oben dargelegt, in mehrere Teilströme aufgeteilt an unterschiedlichen Stellen des Reaktors zugeführt werden. Im allgemeinen wird der Katalysator mit dem Reaktionsgas, enthaltend im wesentlichen das Addukt II, Wasser und gewünschtenfalls ein Inertgas, mit einer Raumgeschwindigkeit von 0,01 bis 10, vorzugsweise von 0,05 bis 5 und besonders bevorzugt von 0,07 bis 3 kg Reaktionsgas/l Katalysator·h belastet. Die Umsetzung, umfassend die Isomerisierung des Addukts II zum Enolether IV und dessen Hydrolyse, wird im allgemeinen bei einer Temperatur von 70 bis 400°C, vorzugsweise von 90 bis 350°C und besonders bevorzugt von 110 bis 330°C und bei einem Druck von im allgemeinen 0,5 bis 100 bar, vorzugsweise von 0,8 bis 20 bar und besonders bevorzugt 1 bis 10 bar ausgeführt.

Der zur Herstellung des Endprodukts n-Butanol zusätzlich zum Wasser benötigte Wasserstoff wird bei der Durchführung des Verfahrens in der Gasphase bezüglich des dem Reaktor zugeführten Addukts II in einem Molverhältnis von im allgemeinen 1 bis 200, vorzugsweise von 1,5 bis 80 und besonders bevorzugt von 2 bis 40 zugesetzt. Wasserstoff kann dem Reaktor gemeinsam mit dem Addukt II und/oder, wie oben dargelegt, in mehrere Teilströme aufgeteilt, an verschiedenen Stellen des Reaktors zugeführt werden. Im allgemeinen wird der Katalysator mit dem Reaktionsgas, enthaltend im wesentlichen das Addukt II, Wasser, Wasserstoff und gewünschtenfalls ein Inertgas, mit einer Raumgeschwindigkeit von 0,01 bis 10, vorzugsweise von 0,05 bis 5, insbesondere von 0,07 bis 3 kg Reaktionsgas/1 Katalysator·h belastet. Die Umsetzung, umfassend die Isomerisierung des Addukts II zum Enolether IV und dessen kombinierte Hydrolyse/Hydrierung, wird im allgemeinen bei Temperaturen von 20 bis 400°C, vorzugsweise von 100 bis 350°C und besonders bevorzugt von 150 bis 300°C und bei einem Druck von im allgemeinen 0,5 bis 100 bar, vorzugsweise von 0,9 bis 50 bar, insbesondere von 1 bis 30 bar vorgenommen.

In analoger Weise, wie für die Isomerisierung des Addukts II zum Enolether IV und dessen Hydrolyse oder Hydrierung zu n-Butyraldehyd und/oder n-Butanol in flüssiger Phase mit Heterogenkatalysatoren beschrieben, kann durch Zufuhr eines Gemisches, das bestimmte Mengen an Wasser und Wasserstoff enthält, als auch durch die Wahl des verwendeten Katalysators die Umsetzung in der Gasphase in der Weise gesteuert werden, daß der Reaktionsaustrag aus dieser Verfahrensstufe n-Butyraldehyd und n-Butanol in dem gewünschten Produktverhältnis enthält.

Zur Aufarbeitung des gasförmigen Reaktionsaustrages kann dieser vorteilhaft, gegebenenfalls nach Entspannung auf Atmosphärendruck, direkt in eine Destillationsapparatur eingeleitet und dort destillativ in seine Bestandteile aufgetrennt werden.

Als Katalysatoren für die Isomerisierung des Addukts II zum Enolether IV und dessen Hydrolyse oder Hydrierung zu n-Butyraldehyd und/oder n-Butanol in der Gasphase in einer einzigen Verfahrensstufe können grundsätzlich die gleichen Heterogenkatalysatoren eingesetzt werden, wie sie auch bei der gleichen Umsetzung in flüssiger Phase Anwendung finden. Vorzugsweise werden im Gasphasenverfahren rein anorganische, mineralische Katalysatoren verwendet. Bevorzugte Katalysatoren sind beispielsweise Trägerkatalysatoren, die Elemente der I., VI., VII und/oder VIII. Nebengruppe, gegebenenfalls in Kombination mit einem oder mehreren Elementen der V. Nebengruppe, des Periodensystems der Elemente, insbesondere der Elemente der I., VII. und VIII. Nebengruppe des Periodensystems der Elemente auf einem Brönsted- oder Lewis-acide wirkenden Trägermaterial abgeschieden enthalten. Besonders vorteilhafte Katalysatoren sind z.B. Rhenium auf Aktivkohle, Rhenium auf Zirkoniumdioxid, Rhenium auf Titandioxid, Rhenium auf Siliziumdioxid, Kupfer auf Aktivkohle, Kupfer auf Siliziumdioxid, Kupfer auf Kieselgur, Kupfer auf Kieselgel, Kupfer auf Titandioxid, Kupfer auf Zirkoniumdioxid, Kupfer auf Magnesiumsilikat, Kupfer auf Aluminiumsilikat, Kupfer auf Bleicherde, Kupfer auf Zeolith, Ruthenium auf Aktivkohle, Ruthenium auf Siliziumdioxid, Ruthenium auf Aluminiumoxid Ruthenium auf Zirkoniumdioxid, Ruthenium auf Magnesiumoxid und Ruthenium auf Titandioxid und mit Selen oder Blei partiell vergiftete Palladium auf Aktivkohle-Katalysatoren.

Die Isomerisierung des Addukts II zum Enolether IV und dessen Hydrolyse oder Hydrierung zu n-Butyraldehyd und/oder n-Butanol in einer einzigen Verfahrensstufe unter Verwendung von Heterogenkatalysatoren kann sowohl bei Anwendung des Flüssigphasenverfahrens als auch bei Anwendung des Gasphasenverfahrens dahingehend vorteilhaft weiter ausgestaltet werden, daß man bei Verwendung eines einzigen Festbetts zur Durchführung dieser Reaktionen eine kombinierte Katalysatorschüttung, bestehend aus mindestens 2 Schichten unterschiedlicher Heterogenkatalysatoren, einsetzt, die sich in Aktivität und gegebenenfalls Selektivität für die beiden Teilreaktionen c) und d) unterscheiden, so daß z.B. in der ersten, zum Einlaß des Reaktionsgutes in den Reaktor hin gelegenen Schicht zunächst das Addukt II mit hoher Aktivität und Selektivität zum Enolether IV isomerisiert wird, welcher dann beim Passieren der nächsten, zum Ausgang des Reaktors hin gelegenen Schicht oder hin gelegenen Schichten aus Katalysatoren mit geringerer Isomerisierungs-, dafür höherer Hydrolyse- und/oder Hydrieraktivität mit hoher Aktivität und Selektivität zu n-Butyraldehyd und/oder n-Butanol umgewandelt wird.

Durch die Aneinanderreihung mehrerer Schichten unterschiedlich aktiver und/oder selektiver Katalysatoren kann die Wärmeentwicklung bei der Hydrolyse bzw. der kombinierten Hydrolyse/Hydrierung des Enolethers IV gezielt beeinflußt und dadurch die Gesamtselektivität der Umsetzung erhöht werden. Dieser Effekt kann weiter verstärkt werden, indem z.B. die Reaktanten Wasser und/oder Wasserstoff nicht gemeinsam mit dem Addukt II in den Reaktor eingeleitet, sondern getrennt vom Addukt II in die Zone der Katalysatorschüttung eingespeist werden, in der die Hydrolyse bzw. der kombinierten Hydrolyse/Hydrierung stattfindet. Das Wasser und der Wasserstoff können gemeinsam in die betreffenden Zonen der Katalysatorschüttung eingespeist werden oder aber auch einzeln in verschiedene Zonen der Katalysatorschüttung geleitet werden. Statt in einer kombinierten Schüttung können die verschiedenen, bei dieser Verfahrensausgestaltung für die einzelnen Teilreaktionen verwendeten Katalysatoren auch einzeln, auf mehrere Festbette verteilt, angeordnet sein.

Obgleich die Durchführung der Teilreaktionen c) und d) des erfindungsgemäßen Verfahrens in einer einzigen Verfahrensstufe, z.B. nach den zuvor beschriebenen Verfahrensweisen eine bevorzugte Ausführungsform des erfindungsgemäßen Verfahrens darstellt, kann es sich unter Umständen als vorteilhaft erweisen, die einzelnen Teilreaktionen, nämlich die Isomerisierung des Addukts II zum Enolether IV, die Hydrolyse des Enolethers IV zu n-Butyraldehyd oder die Hydrierung des Butyraldehyds zu n-Butanol in mehreren Verfahrensstufen durchzuführen. Beispielsweise ist es möglich, jede einzelne dieser Reaktionen in einer Verfahrensstufe vorzunehmen, indem man zunächst in einer Verfahrensstufe das Addukt II zum Enolether IV isomerisiert, anschließend den Enolether IV zu n-Butyraldehyd hydrolysiert und dann den n-Butyraldehyd zu n-Butanol hydriert. Ebenso kann die Isomerisierung des Addukts II zum Enolether IV für sich in einer Verfahrensstufe durchgeführt werden und der Enolether IV anschließend zu n-Butyraldehyd hydrolysiert oder in einer Hydrolyse-/Hydrierumsetzung zu n-Butanol oder einer Mischung aus n-Butanol und n-Butyraldehyd verarbeitet werden. Eine weitere Variante des erfindungsgemäßen Verfahrens besteht darin, die Isomerisierung des Addukts II zum Enolether IV und dessen Hydrolyse zu n-Butyraldehyd in einer einzigen Verfahrensstufe auszuführen und anschließend den hierbei erhaltenen n-Butyraldehyd in einer weiteren Verfahrensstufe zu n-Butanol zu hydrieren.

Bei der Aufteilung der Teilreaktionen c) und d) auf mehrere Verfahrensstufen können in den einzelnen Verfahrensstufen verschiedenerlei Betriebsweisen angewandt werden. Beispielsweise kann die Isomerisierung des Addukt II zum Enolether IV wahlweise unter homogener Katalyse oder an Heterogenkatalysatoren vorgenommen werden, desgleichen kann die Hydrolyse bzw. die kombinierte Hydrolyse/Hydrierung des Enolethers IV zu n-Butyraldehyd und/oder n-Butanol wahlweise in flüssiger Phase unter Verwendung von Homogenkatalysatoren oder Heterogenkatalysatoren oder in der Gasphase ausgeführt werden.

Bei der Aufteilung der einzelnen Teilreaktionen c) und d) auf mehrere Verfahrensstufen ist es auch möglich, anstelle der zuvor beschriebenen Katalysatoren, welche sowohl die Isomerisierung des Addukts II zum Enolether IV als auch dessen Hydrolyse und Hydrierung katalysieren, in den einzelnen Verfahrensstufen Katalysatoren zu verwenden, welche nur die jeweilige Teilreaktion katalysieren können. So kann der Enolether IV beispielsweise mittels Brönsted-Säure-Katalysatoren, wie Mineralsäuren, z.B. Halogenwasserstoffsäuren, Schwefelsäure, verdünnter Salpetersäure, Phosphorsäure oder heterogenen Brönsted-Säuren, wie Ionenaustauschern, Zeolithen, Bleicherden oder sauren Phosphaten, beispielsweise Aluminiumphosphate, zu n-Butyraldehyd hydrolysiert werden oder es kann der in einer einzigen Verfahrensstufe durch Isomerisierung des Addukts II zum Enolether IV und dessen Hydrolyse erhaltene n-Butyraldehyd mit Katalysatoren, die nur Hydrieraktivität, jedoch keine Hydrolyseaktivität, haben, zu n-Butanol umgesetzt werden. Desgleichen kann in diesem Falle für die Durchführung der Teilreaktion c) ein Isomerisierungskatalysator verwendet werden, der weder Hydrolyse- noch Hydrieraktivität besitzt.

Der bei der Hydrolyse oder kombinierten Hydrolyse/Hydrierung aus dem Enolether IV freigesetzte Alkohol ROH I wird vorzugsweise wieder in die Umsetzung gemäß Teilreaktion a) zurückgeführt. Aufgrund der Möglichkeit, die Teilreaktionen der Isomerisierung des Addukts II zum Enolether IV und dessen Hydrolyse bzw. dessen kombinierte Hydrolyse/Hydrierung in mehrere Verfahrensschritte aufzuteilen, erhält man eine höhere Flexibilität beim Bau einer Anlage zur Durchführung des erfindungsgemäßen Verfahrens, wodurch erhebliche Einsparungen möglich sind.

Das erfindungsgemäße Verfahren wird anhand des Fließbilds gemäß Fig., das schematisch eine vorteilhafte Ausgestaltung des erfindungsgemäßen Verfahrens wiedergibt, in der sowohl die Addition des Alkohols ROH an 1,3-Butadien als auch die Isomerisierung des Addukts II zum Enolether IV und dessen Hydrolyse oder kombinierte Hydrolyse/Hydrierung zu n-Butyraldehyd und/oder n-Butanol in einer einzigen Verfahrensstufe in flüssiger Phase ausgeführt werden, näher erläutert. Da dieses Verfahrensfließbild lediglich die Führung der Edukt-, Zwischenprodukt- und Produktströme im erfindungsgemäßen Verfahren erläutern soll, wurden der Übersichtlichkeit halber, selbstverständliche Details der Anlage, wie Pumpen, Wärmetauscher, Ventile oder Relais, nicht in das Verfahrensfließbild eingezeichnet.

Über die Zuleitung 1 wird dem Reaktor 2 ein Gemisch aus 1,3-Butadien und Alkohol ROH I, vorzugsweise n-Butanol, zugeführt. Die Zuleitung 1 wird über die Zuleitungen 3 bzw. 4 mit 1,3-Butadien bzw. dem Alkohol ROH I beschickt. Im Reaktor 2 wird der Alkohol ROH I katalytisch, vorzugsweise mittels einer Brönsted-Säure, insbesondere mittels eines sauren Kationenaustauschers an 1,3-Butadien addiert, wobei sich im allgemeinen ein Gemisch der Addukte II und III bildet. Der Reaktionsaustrag aus dem Reaktor 2, der im wesentlichen aus den Addukten II und III, höhersiedenden Butadien-Derivaten sowie nicht umgesetztem 1,3-Butadien und Alkohol ROH I besteht, wird über Leitung 5 dem Gas-Flüssigkeit-Abscheider 6 zugeführt, in dem gasförmiges 1,3-Butadien von den flüssigen Bestandteilen des Reaktionsaustrages von Reaktor 2 abgetrennt und entweder über die Leitungen 7, 8 und 1 wieder in den Reaktor 2 zurückgeführt oder über die Leitungen 7 und 9 der Fackel zur Verbrennung zugeführt wird. Das im Abscheider 9 abgeschiedene Flüssigkeitsgemisch wird über Leitung 10 in die Destillationskolonne 11 geleitet, in der das leichter flüchtige Addukt III vom schwerer flüchtigen Addukt II sowie von noch gegebenenfalls vorhandenem Alkohol ROH I und höhersiedenden Butadien-Derivaten destillativ abgetrennt wird. Das Addukt III, nicht umgesetzter Alkohol ROH I sowie gegebenenfalls noch vorhandenes, nicht umgesetztes 1,3-Butadien werden anschließend über die Leitungen 12 und 1 in den Reaktor 2 zurückgeleitet, wo das Addukt III in Gegenwart von frisch zugeführtem 1,3-Butadien und Alkohol ROH I zum Addukt II isomerisiert wird. Die mit dem Reaktoraustrag aus Reaktor 2 der Kolonne 11 zugeführten Leichtsieder, z.B. Vinylcyclohexen, werden, gewünschtenfalls zusammen mit dem in Kolonne 11 abgetrennten Rest-Butadien, über Auslaß 42 der Fackel zugeführt. Anstelle einer einzigen Destillationskolonne 11 können auch mehrere, hintereinandergeschaltete Destillationskolonnen zur destillativen Auftrennung des flüssigen Reaktionsaustrags von Reaktor 2 eingesetzt werden. Bei Verwendung mehrerer Destillationskolonnen anstelle einer einzigen Destillationskolonne 11 können höhersiedende, im Austrag von Reaktor 2 enthaltene Reaktionsprodukte, beispielsweise Dibutylether und gewünschtenfalls die Alkoxyoctadiene oder Alkoxydodecatriene, vom Addukt II abgetrennt und aus dem Verfahren ausgeschleust werden.

Der vom leichter flüchtigen Addukt III und von leichtsiedenden und gegebenenfalls von höhersiedenden Nebenprodukten befreite flüssige Austrag von Kolonne 11 wird über Leitung 13 dem Reaktor 14 zugeführt, in dem das Addukt II in einer einzigen Verfahrensstufe in Gegenwart eines homogenen oder heterogenen Übergangsmetallkatalysators zum Enolether IV isomerisiert und dieser zu n-Butyraldehyd hydrolysiert bzw. in einer kombinierten Hydrolyse/Hydrierung in n-Butanol und gewünschtenfalls n-Butyraldehyd umgewandelt wird. Über Leitung 15 wird dem Reaktor 14 der zu dieser Umsetzung benötigte Wasserstoff und über Leitung 16 das erforderliche Wasser zugeführt. Alternativ zur Einleitung über die Zuläufe 15 und 16 können das Wasser oder der Wasserstoff über die Zuläufe 17 und 18 in den Reaktor eingespeist werden. Soll in der Anlage allein n-Butyraldehyd erzeugt werden, können die Wasserstoffzuleitungen 15 oder 18 geschlossen bleiben oder über diese Leitungen nur soviel Wasserstoff in den Reaktor eingeschleust werden, wie zur Verbesserung der Standzeit des Katalysators erforderlich ist. Gewünschtenfalls kann zu diesem Zweck gemeinsam mit dem Wasserstoff auch noch Kohlenmonoxid in den Reaktor eingeleitet werden.

Der flüssige Reaktionsaustrag aus Reaktor 14, der im wesentlichen n-Butyraldehyd und/oder n-Butanol, höhersiedende Butadien-Derivate, beispielsweise Octanole oder Dodecanole, und gegebenenfalls überschüssiges Wasser sowie, falls im Reaktor 14 ein Homogenkatalysator verwendet wurde, Katalysatorlösung enthält, wird über Leitung 19 der Destillationskolonne 20 zugeführt. Nicht umgesetzter Wasserstoff wird zum größten Teil aus dem Reaktor 14 über Leitung 21 abgezogen und entweder über die Leitungen 15 oder 18 wieder in die Umsetzung zurückgeführt oder abgefackelt. Gewünschtenfalls kann der Wasserstoff auch über einen zwischen dem Reaktor 14 und der Destillationskolonne 20 angebrachten Gas-Flüssigkeit-Abscheider abgetrennt und wie vorher dargestellt, weiter behandelt werden.

In der Destillationskolonne 20 wird der Reaktionsaustrag aus Reaktor 14 destillativ in seine Bestandteile aufgetrennt. Der leichterflüchtige n-Butyraldehyd wird über Kopf über Leitung 22 gegebenenfalls zusammen mit leichtsiedenden Nebenprodukten abgezogen und zwecks weiterer Reinigung einer zusätzlichen Destillationsstufe zugeführt, die in Fig. nicht eingezeichnet ist. Neu gebildetes n-Butanol wird über Leitung 23 aus der Kolonne abgeführt und über Leitung 24 der weiteren Verwendung zugeführt. Höhersiedende Produkte, beispielsweise Dibutylether, Octanole und Dodecanole werden über mehrere Auslässe im unteren Teil der Kolonne 20 entnommen, für die in Fig. stellvertretend der Auslaß 26 eingezeichnet ist. Wurde im Reaktor 14 ein Homogenkatalysator verwendet, so wird die Katalysatorlösung aus dem Sumpf der Kolonne 20 über Leitung 27 entnommen und gegebenenfalls nach Ausschleusung eines Teilstroms an verbrauchtem Katalysator über Leitung 28 und Ergänzung mit frischer Katalysatorlösung über Leitung 29, wieder in den Reaktor 14 zurückgeführt.

Gewünschtenfalls kann die Umsetzung im Reaktor 14 so gesteuert werden, daß dort nur n-Butanol, jedoch kein n-Butyraldehyd erzeugt wird. n-Butyraldehyd kann in einer weiteren Ausgestaltung des erfindungsgemäßen Verfahrens im parallel zum Reaktor 14 geschalteten Reaktor 30, der über Leitung 31 mit einem Teilstrom des Austrags von Kolonne 11 versorgt wird, produziert werden. Ähnlich wie im Reaktor 14 wird im Reaktor 30 das Addukt II in einer einzigen Verfahrensstufe zum Enolether IV isomerisiert und dieser zu n-Butyraldehyd hydrolysiert, jedoch nicht zu n-Butanol hydriert. Das zur Hydrolyse benötigte Wasser wird in den Reaktor 30, je nach Art und Anordnung des Katalysators in Reaktor 30, über die Zuleitungen 32 und/oder 33 eingespeist. Der flüssige Austrag von Reaktor 30 gelangt über Leitung 34 in die Destillationskolonne 35, aus der n-Butyraldehyd über Leitung 36 abgezogen wird. Das bei der Hydrolyse aus dem Enolether IV freigesetzte n-Butanol bzw. der anstelle von n-Butanol in Reaktor 2 eingesetzte Alkohol ROH I wird über Leitung 37 der Kolonne entnommen und über die Leitungen 25 und 1 wieder in den Reaktor 2 zurückgeführt, wo es erneut mit frischem 1,3-Butadien zu den Addukten II und III umgesetzt wird. Höhersiedende Produkte, beispielsweise dimere und trimere Butadien-Derivate, werden über mehrere Auslässe, für die stellvertretend der Auslaß 38 eingezeichnet ist, im unteren Teil der Kolonne 35 entnommen. Wurde in Reaktor 30 ein Homogenkatalysator eingesetzt, so wird die Katalysatorlösung aus dem Sumpf der Kolonne 35 über Leitung 39, gegebenenfalls nach Ausschleusung eines Teilstroms an verbrauchtem Katalysator über Leitung 40 und Ergänzung mit frischer Katalysatorlösung über Leitung 41, wieder in den Reaktor 30 zurückgefahren.

Bei Verwendung von n-Butanol als Alkohol ROH I, gemäß bevorzugter Ausführungsform des erfindungsgemäßen Verfahrens, wird das in Kolonne 20 isolierte und über Leitung 23 abgeführte n-Butanol, das aus dem aus 1,3-Butadien neu gebildeten und dem ursprünglich über Leitung 4 zugesetzten und in Reaktor 14 wieder freigesetzten n-Butanol besteht, in zwei Teilströme aufgeteilt, wobei die neugebildete Menge an n-Butanol über Leitung 24 der weiteren Verwendung zugeführt und die ursprünglich als Alkohol ROH I eingesetzte Menge an n-Butanol über die Leitungen 25 und 1 wieder in den Reaktor 2 zurückgeleitet wird. Bei Verwendung eines anderen Alkohols ROH I als n-Butanol wird dieser der Kolonne 20 entsprechend seinem Siedepunkt einem separaten Auslaß 43 entnommen und über die Leitungen 25 und 1 wieder in den Reaktor 2 zurückgeführt.

Das aus Kolonne 20 über Leitung 23 abgezogene n-Butanol bzw. der gegebenenfalls über Auslaß 43 abgeführte, von n-Butanol verschiedene Alkohol ROH I werden erforderlichenfalls vor ihrer Weiterverwendung bzw. ihrer Rückführung in den Reaktor 2 einer weiteren, in Fig. nicht eingezeichneten, destillativen Reinigung unterzogen, um gegebenenfalls darin enthaltene Verunreinigungen, wie Dibutylether, und Restmengen von Wasser aus der Umsetzung in Reaktor 14 zu entfernen. Gleiches gilt für die weitere destillative Reinigung der über Auslaß 26 abgezogenen, höhersiedenden Produkte. Die destillative Reinigung des über Leitung 43 abgezogenen Alkohols ROH I bzw. des über Leitung 23 abgezogenen n-Butanols kann unter Umständen erforderlich sein, um ein Aufpegeln von Verunreinigungen und Wasser im Prozeßkreislauf zu vermeiden. Die destillative Reinigung der Austräge aus Kolonne 20 kann nach herkömmlichen Destillationsverfahren erfolgen und ist nicht Gegenstand der vorliegenden Erfindung. Für die über die Leitungen 36, 37 und 38 aus Kolonne 35 abgezogenen Produkte gilt das obengesagte entsprechend. Es wird in diesem Zusammenhang noch einmal darauf hingewiesen, daß die Auslässe aus den Kolonnen 11, 20 und 35 in Fig. rein schematisch eingezeichnet sind. Die Zusammensetzung der in diesen Kolonnen zu destillierenden Produkte variiert in Abhängigkeit von der in den Reaktoren 2, 14 und 30 angewandten Verfahrensweise und es ist eine Routineaufgabe des Fachmanns in Anbetracht der jeweils vorliegenden Produktzusammensetzung die für die Auftrennung der Produkte erforderliche Destillationskolonne entsprechend zu dimensionieren.

### Beispiele

### Beispiel 1 (Teilreaktion a))

Ein 0,3 l Rührautoklav wurde mit 67,0 g (0,90 mol) n-Butanol und mit 15,0 g Lewatit® SPC 118 in der H⁺-Form, der vorher mit Wasser und n-Butanol gewaschen worden war, befüllt. Anschließend wurden 47,9 g (0,88 mol) 1,3-Butadien in den Reaktor gepreßt. Nach einer Reaktionszeit von 10 h bei einer Temperatur von 90°C und einem Druck von 9 bar wurde bei einem Umsatz von 46 % eine Selektivität für 3-Butoxy-but-1-en von 48,4 % und eine Selektivität für 1-Butoxy-but-1-en von 41,1 % festgestellt.

### Beispiel 2

Ein 0,3 l Rührautoklav wurde mit 67,0 g (0,90 mol) n-Butanol sowie mit 11,5 g Lewatit® SPC 118 in der H⁺-Form, der vorher mit Wasser und n-Butanol gewaschen worden war, und mit 3,5 g eines mit Kupfer(II)chlorid dotierten Lewatit® SPC 118-Ionenaustauscher befüllt. Anschließend wurden 47,0 g (0,88 mol) 1,3-Butadien in den Autoklaven gepreßt. Nach 10 h Reaktion bei 90°C und unter Eigendruck wurde bei einem Umsatz von 69,1 % eine Selektivität von 46,8 % für 3-Butoxy-but-1-en und eine Selektivität von 44,3 % für 1-Butoxy-but-2-en erhalten.

### Beispiel 3

Ein beheizbarer 1,4 1-Rohrreaktor wurde mit 1 kg eines mit Wasser und n-Butanol gewaschenen, gelförmigen Ionenaustauschers der Marke Amberlite® IR 120 in der H⁺-Form beschickt. 1,3-Butadien und n-Butanol wurden in flüssiger Phase bei einem Druck von 20 bar vor dem Reaktor gemischt und anschließend kontinuierlich über das Ionenaustauscherbett geleitet. Der Einfluß der Reaktionsparameter Temperatur, Flußrate und des 1,3-Butadien/n-Butanol-Molverhältnisses wurde in einem weiten Bereich untersucht. Die unter den verschiedenen Versuchsbedingungen erhaltenen Ergebnisse sind in Tabelle 1 aufgeführt. Die Analyse der Produktzusammensetzung wurde mittels kalibrierter Gaschromatographie durchgeführt.

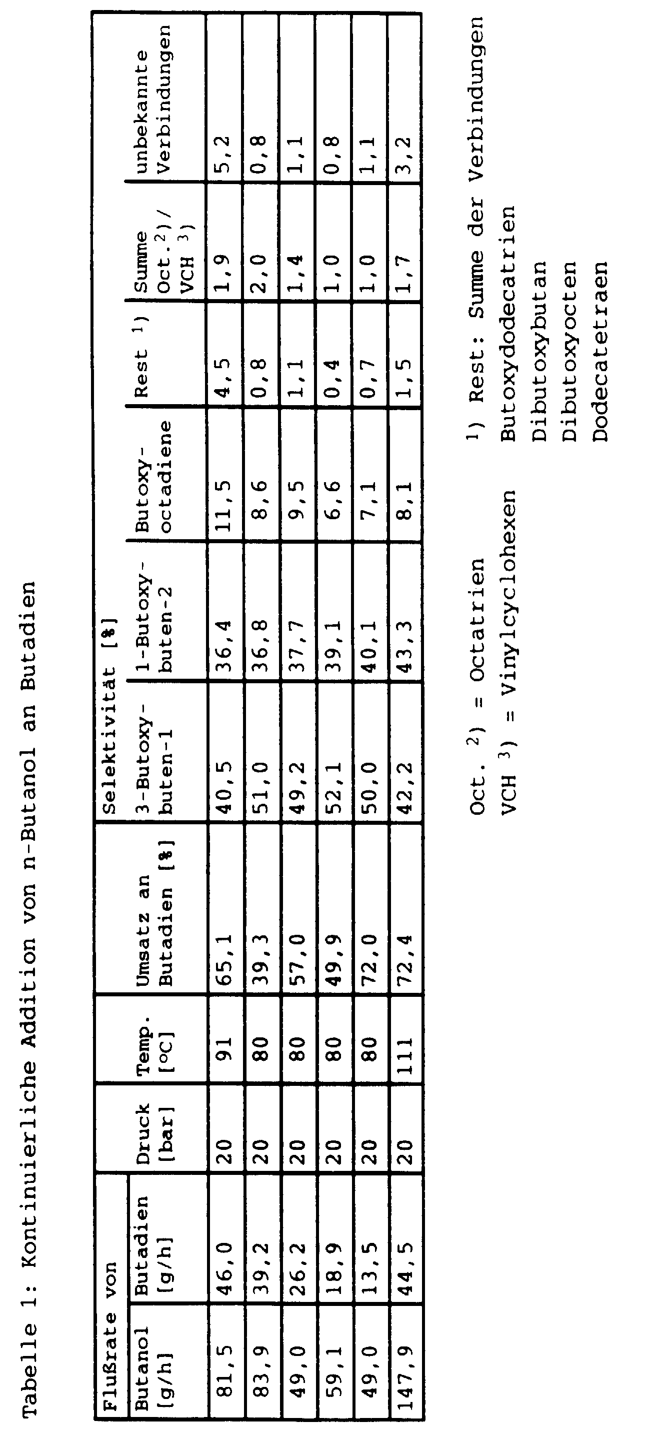

### Beispiel 4

Es wurden in einem 0,3 l Rührautoklaven Reihenversuche zur Addition von n-Butanol an 1,3-Butadien unter den in den Tabellen 2, 3 und 4 angegebenen Reaktionsbedingungen durchgeführt und dabei die in diesen Tabellen angegebenen Ergebnisse erzielt. Tabelle 2 betrifft die Verwendung verschiedener, saurer undotierter Ionenaustauscher als Katalysatoren, Tabelle 3 gibt die Ergebnisse von Versuchen wieder, bei denen unterschiedliche Mengen an undotiertem im Gemisch mit Kupfer(II)chlorid dotiertem Lewatit® SPC 118-Ionenaustauscher als Katalysatoren verwendet wurden und in Tabelle 4 sind die Ergebnisse aufgelistet, die mit Mischungen von mit verschiedenen Kupfer(II)salzen dotierten Ionenaustauschern mit den jeweils undotierten Ionenaustauschern als Katalysatoren erzielt wurden.

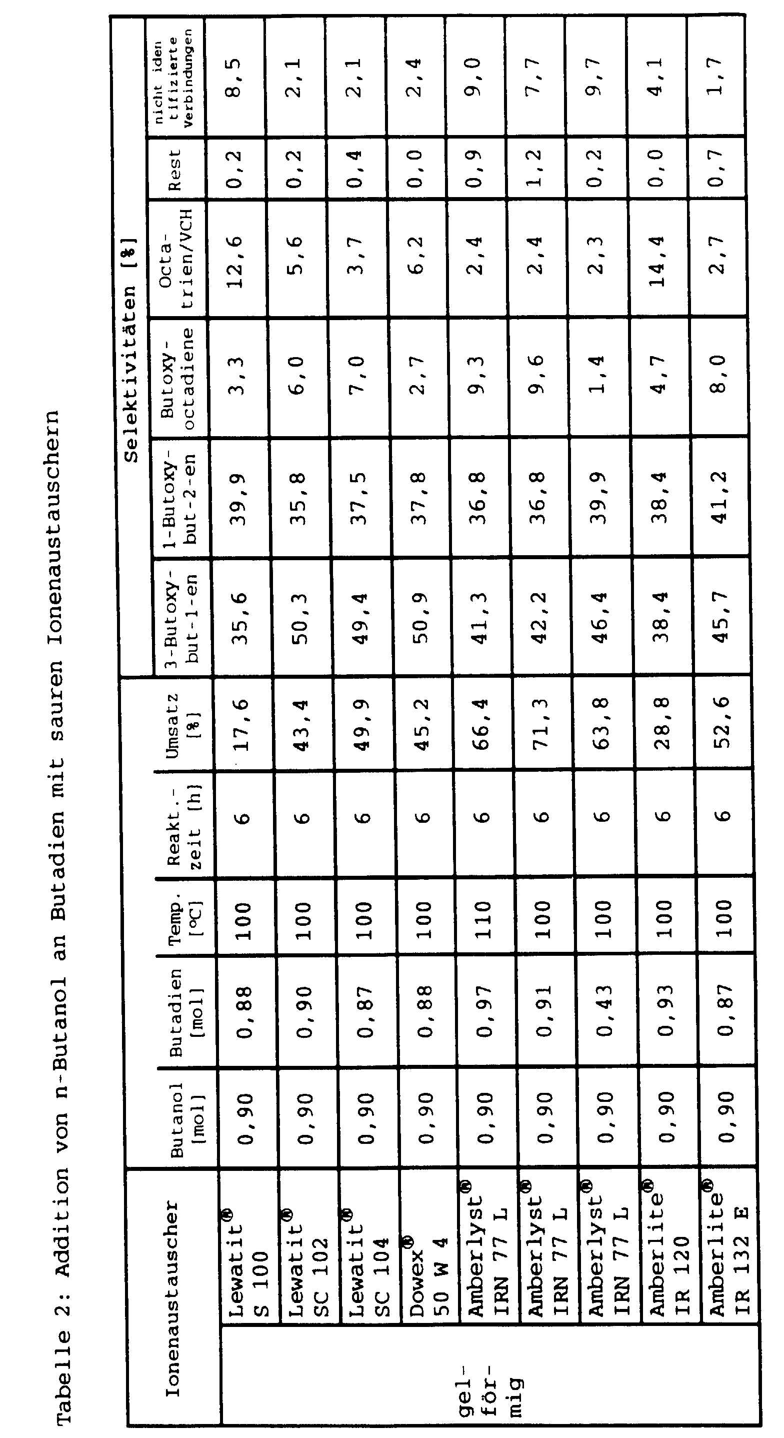

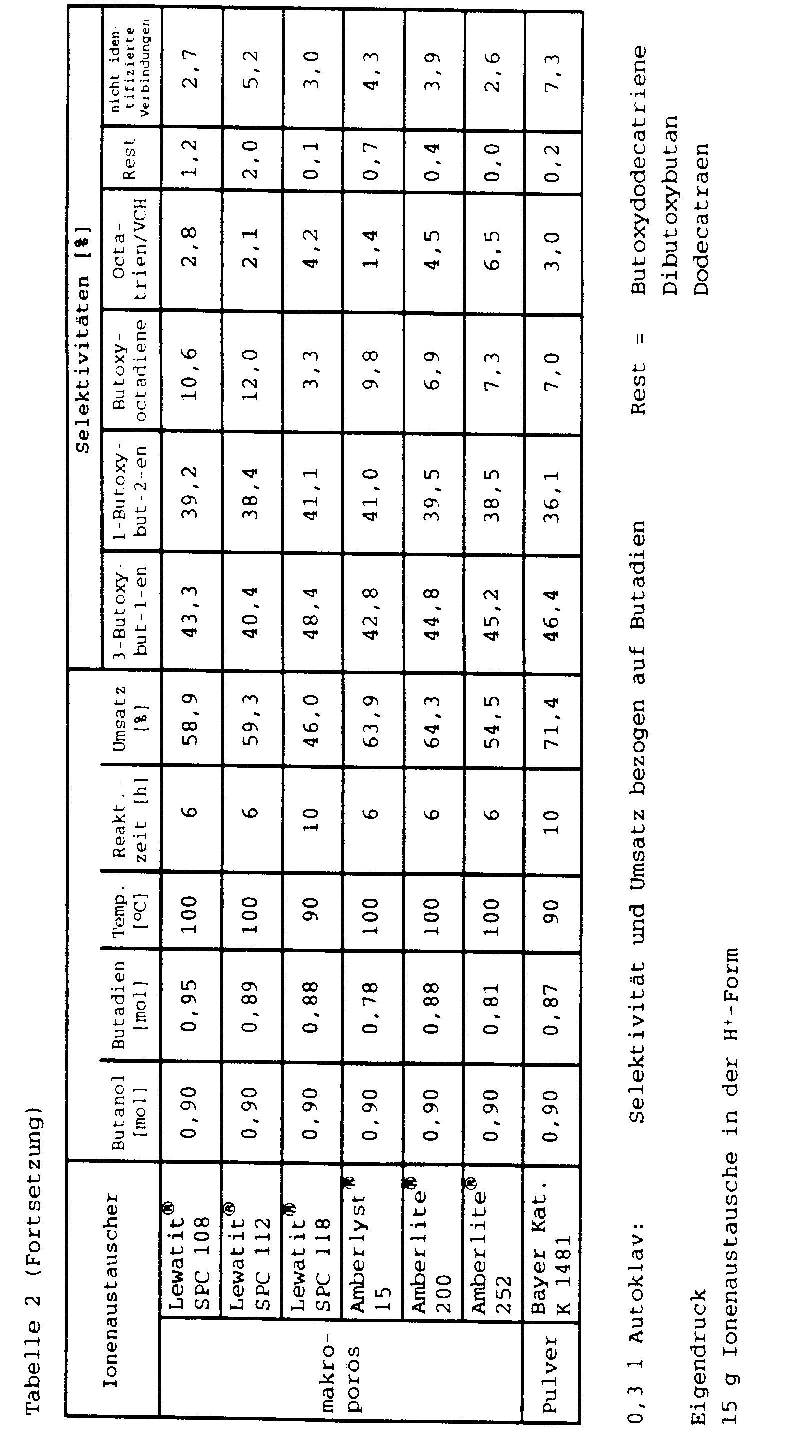

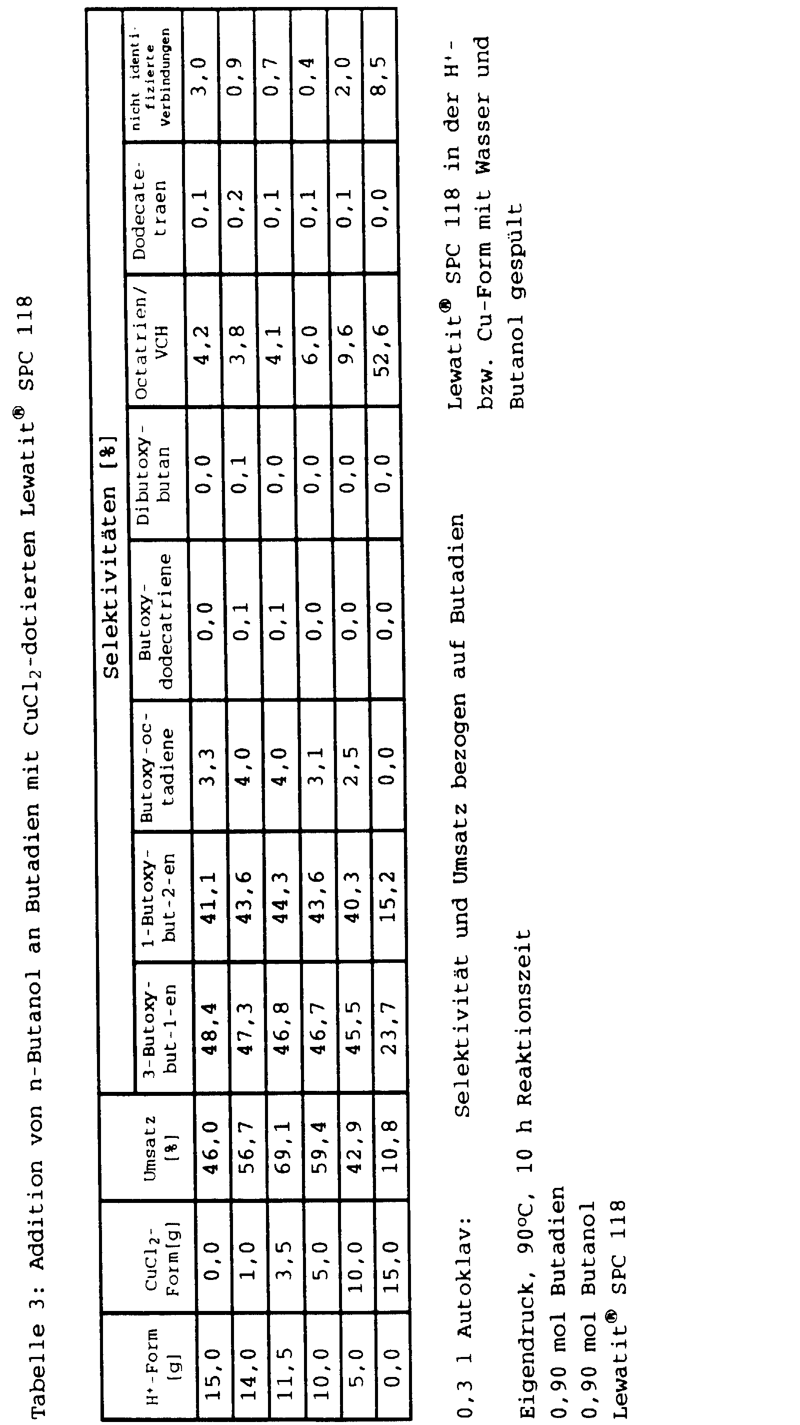

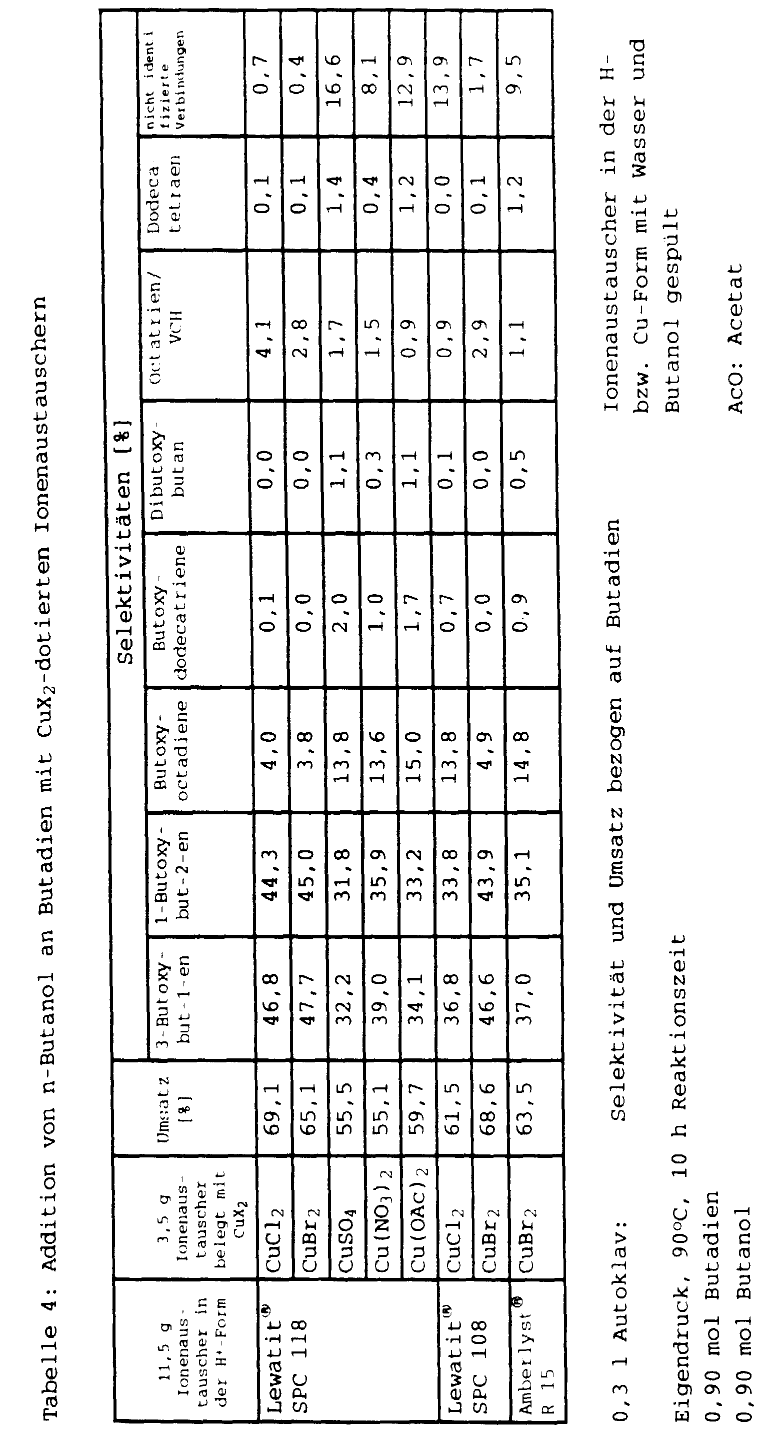

### Beispiel 5 (Isomerisierung von Addukt III zu Addukt II)

Ein Rührautoklav wurde mit 6,0 g n-Butanol, 2,0 g 3-Butoxybut-1-en und 1,2 g getrocknetem Lewatit® SPC 118 Ionenaustauscher in der H⁺-Form befüllt. Die Reaktionsmischung wurde auf 105°C erhitzt und nach 2 und 6 h Reaktionszeit eine Probe entnommen und das Verhältnis 3-Butoxybut-1-en/1-Butoxybut-2-en gaschromatographisch bestimmt. Die Änderung dieses Verhältnisses mit der Reaktionszeit ist in Tabelle 5 dargestellt.

**Tabelle 5**

| | Molverhältnis von | |
|---|---|---|
| Reaktionszeit [h] | 3-Butoxy-buten-1 | 1-Butoxy-buten-2 |
| 0 | 100 | 0 |
| 2 | 70 | 30 |
| 6 | 61 | 39 |

### Beispiel 6

In der in Beispiel 3 beschriebenen Apparatur wurden 1,3-Butadien, n-Butanol und ein Gemisch aus Butoxybutenen, wie es bei der Addition von n-Butanol an 1,3-Butadien entstanden war und von dem vorher der größte Teil des 1-Butoxy-2-ens destillativ abgetrennt worden war, in flüssiger Phase vor dem Reaktor vermischt und anschließend kontinuierlich bei einem Druck von 20 bar und bei verschiedenen Temperaturen über das Ionenaustauscherbett geleitet. Die Ergebnisse dieser Versuche sind in Tabelle 6 aufgelistet. Alle Analysen wurden mittels kalibrierter Gaschromatographie erstellt.

### Beispiel 7 (Vergleich zu Beispiel 6)

Beispiel 7 wurde wie Beispiel 6 durchgeführt, mit dem Unterschied, daß nur 1,3-Butadien und n-Butanol, jedoch keine Butoxybutene dem Reaktor zugeführt wurden. Die Ergebnisse sind in Tabelle 6 aufgelistet.

Aus dem Vergleich der Ergebnisse der Beispiele 6 und 7 in Tabelle 6 ergibt sich, daß durch die Rückführung des bei der Addition von n-Butanol an 1,3-Butadien gebildeten, unerwünschten 3-Butoxy-but-1-ens in die Additionsreaktion die Neubildung dieses Nebenproduktes unterdrückt wird.

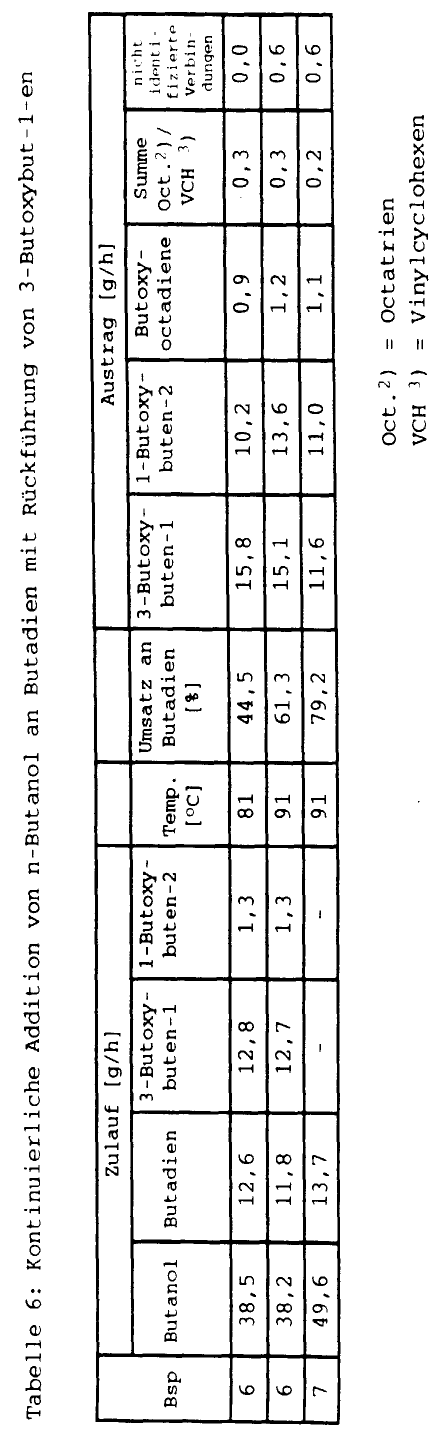

### Beispiel 8 Addition von n-Butanol an 1,3-Butadien unter Zeolith-Katalyse

a) Herstellung der H⁺-Form der Zeolithe
   Ein handelsüblicher Y-Zeolith in der Na-Form (Modul: 5) wurde, wie folgt, in die H⁺-Form überführt:
   100 g des Zeolithen wurden bei 80°C zwecks Ionenaustausch mit Ammoniumsulfatlösung behandelt, anschließend mit Wasser gewaschen, bei 110°C getrocknet und bei 500°C fünf Stunden calciniert. Diese Behandlung wurde noch einmal wiederholt. Der erhaltene Y-Zeolith (H⁺-Form) enthielt noch 0,02 Gew.-% Natrium und sein Röntgendiffraktogramm entsprach dem typischen Röntgenbeugungsbild eines Y-Zeolithen in der H⁺-Form (FAU-Struktur).
   Auf die gleiche Weise wurde ein Na-β-Zeolith behandelt, der gemäß Beispiel 1 von US-A 4 891 458 hergestellt worden war.
b) Ein 0,3 Rührautoklav wurde mit 67,0 g (0,90 mol) n-Butanol und mit 5 g des gemäß Beispiel 8a) hergestellten Y-Zeolithen in der H⁺-Form befüllt. Anschließend wurden 49,7 g (0,92 mol) 1,3-Butadien aufgepreßt. Nach einer Reaktionszeit von 6 Stunden bei 130°C und einem Druck von 9 bar wurde bei einem 1,3-Butadien-Umsatz von 35,9 % eine Selektivität für die Bildung von 3-Butoxybut-1-en von 32,4 % und für die Bildung von 1-Butoxybut-2-en von 20,3 % gemessen.
c) In analoger Weise wie in Beispiel 8b) wurden 0,90 mol n-Butanol und 0,88 mol 1,3-Butadien in Gegenwart von 5 g des gemäß Beispiel 8a) hergestellten β-Zeolithen in der H⁺-Form umgesetzt. Bei einem Umsatz von 40,0 % wurde 3-Butoxybut-1-en mit einer Selektivität von 42,5 % und 1-Butoxybut-2-en mit einer Selektivität von 16,5 % gebildet.

### Beispiel 9 Addition n-Butanol an 1,3-Butadien in Gegenwart eines Übergangsmetallelement-Homogenkatalysators

Ein 0,3 l Rührautoklav wurde mit 74,0 g (1,0 mol) n-Butanol, 0,205 g (0,66 mmol) Palladiumacetonylacetonat und 2,02 g (7,3 mmol) 1-(Di-isopropyl-phosphino)-3-(di-tert.butyl-phosphino)propan unter einer Stickstoffatmosphäre befüllt. Anschließend wurden 34,7 g (0,64 mol) 1,3-Butadien aufgepreßt. Nach einer Reaktionszeit von 20 Stunden bei 80°C und einem Druck von 9 bar wurde die Reaktion abgebrochen und die Reaktionsmischung gaschromatographisch analysiert.

| | |
|---|---|
| Ergebnis Butadien-Umsatz | 88 % |
| Selektivität der Bildung von 3-Butoxybut-1-en | 64,5 % |
| Selektivität der Bildung von 1-Butoxybut-2-en | 34,3 % |

### Beispiel 10 (Isomerisierung des Addukts II zum Enolether IV)

Unter einer Argonatmosphäre wurden 40 g (312 mmol) 1-Butoxybut-2-en in einer Schmelze von 100 g (382 mmol) Triphenylphosphin (PPh₃) und 5 g (5,4 mmol) HRh(PPh₃)₃CO 200 min bei 120°C unter Rühren umgesetzt. Anschließend wurden die Produkte in Toluol aufgenommen und gaschromatographisch analysiert. Der Umsatz an 1-Butoxybut-2-en betrug 48,5 %, die Selektivität für die Bildung von 1-Butoxybut-1-en 95,5 % und die Selektivität für die Bildung von 1-Butoxybut-3-en 4,5 %.

### Beispiel 11

Beispiel 10 wurde wiederholt, wobei anstelle von Argon eine äquimolare CO/H₂-Mischung verwendet wurde. Nach 240 min Reaktionszeit wurden die Produkte in Toluol aufgenommen und gaschromatographisch analysiert. Der Umsatz an 1-Butoxy-1-en betrug 50,3 %, die Selektivität für die Bildung von 1-Butoxybut-1-en 99,5 % und die Selektivität für die Bildung von 1-Butoxybut-3-en 0,5 %.

### Beispiel 12 (Isomerisierung des Addukts II zum Enolether IV und Hydrolyse des Enolethers IV zu n-Butyraldehyd in einer einzigen Stufe mit einem Homogenkatalysator)

Eine Mischung aus 2,0 g 1-Butoxybut-2-en, 3,0 g Wasser und 0,20 g des Katalysators HRuCl(CO)(PPh₃)₃ wurden in einem Rührautoklaven bei 160°C unter Eigendruck 7 h lang umgesetzt. Bei einem Umsatz von 69,3 % betrug die Selektivität für die Bildung von n-Butyraldehyd 85,6 %. 1-Butanol wurde aus 1-Butoxy-2-en mit einer Selektivität von 96,1 % freigesetzt.

### Beispiel 13

Beispiel 12 wurde wiederholt, jedoch wurden 2,0 g des Lösungsmittels Diethylenglykoldimethylether zugesetzt. Bei einem Umsatz von 78,3 % wurde n-Butyraldehyd mit einer Selektivität von 86,5 % gebildet.

### Beispiel 14 (Isomerisierung des Addukts II zum Enolether IV und Hydrolyse/Hydrierung des Enolethers IV zu n-Butanol in einer einzigen Stufe mit einem Homogenkatalysator)

In eine Mischung aus 3,0 g Wasser, 0,022 g des Katalysators HRuCl(CO)(PPh₃)₃, 0,028 g Triphenylphosphin und 2,02 g 1-Butoxybut-2-en wurde bei 120°C und bei einem Druck von 12 bar unter Rühren solange Wasserstoff eingeleitet, bis keine weitere Wasserstoffaufnahme mehr beobachtet werden konnte. Nach 3 h Reaktionszeit wurde das Reaktionsgemisch mittels kalibrierter Gaschromatographie analysiert. Bei einem Umsatz von 99 % wurde n-Butanol mit einer Selektivität von 97,7 % gebildet.

### Beispiel 15 (Isomerisierung des Addukts II zum Enolether IV und Hydrierung des Enolethers zu n-Butanol in einer einzigen Stufe mit einem Heterogenkatalysator in der Gasphase)

In einen Reaktor wurden 200 ml eines Kupfer auf Aktivkohle-Katalysators, der einen Kupfergehalt, berechnet als CuO, von 10 Gew.-% hatte, eingefüllt und der Katalysator innerhalb einer Stunde im Wasserstoffstrom bei Atmosphärendruck bei einer Temperatur von anfangs 150°C und einer Endtemperatur von 260°C aktiviert.

Danach wurde der Reaktor auf 210°C abgekühlt und über einen auf 200°C erhitzten Vorheizer 32,0 g/h Wasser und 9,4 g/h 1-Butoxy-2-en bei Atmosphärendruck durch den Reaktor geleitet. Gleichzeitig wurde dem Reaktor ein Wasserstoffstrom von 15 l/h zugeführt. Nach Abkühlung wurde der zweiphasige, flüssige Reaktoraustrag mittels kalibrierter Gaschromatographie analysiert. Bei einem Umsatz von 94 % wurde n-Butanol mit einer Selektivität von 95,8 % gebildet. Die Selektivität für n-Butyraldehyd betrug 4,2 %.

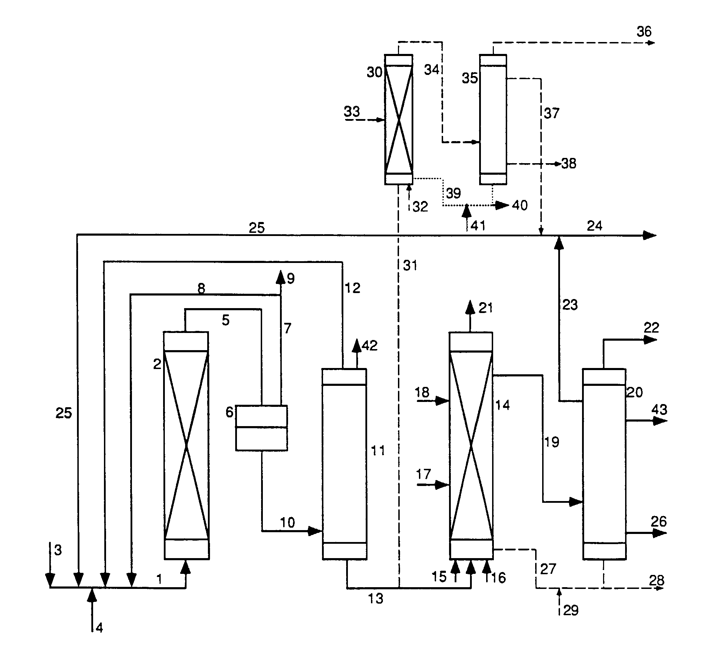

## Patentansprüche

1. Verfahren zur Herstellung n-Butyraldehyd und/oder n-Butanol, dadurch gekennzeichnet, daß man
a) 1,3-Butadien oder 1,3-Butadien-haltige Kohlenwasserstoffströme mit einem Alkohol der Formel I
ROH I,
in der der Rest R eine unsubstituierte oder mit 1 bis 2 C₁- bis C₁₀-Alkoxy- oder Hydroxygruppen substituierte C₂- bis C₂₀-Alkyl- oder Alkenyl-, eine C₆- bis C₁₀-Aryl- oder eine C₇- bis C₁₁-Aralkylgruppe oder die Methylgruppe ist, bei erhöhter Temperatur und bei erhöhtem Druck in Gegenwart einer Brönsted-Säure oder in Gegenwart eines Komplexes eines Elementes aus der Gruppe Ib, VIIb oder VIIIb des Periodensystems der Elemente mit Phosphor- oder Stickstoff-haltigen Liganden zu einem Gemisch der Addukte der Formeln II 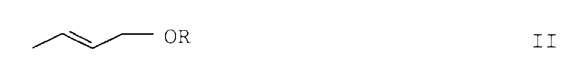 und III 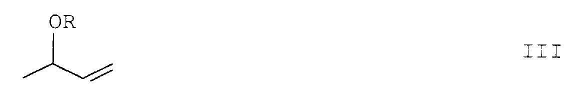 umsetzt,
b) das Addukt III zum Addukt II isomerisiert,
c) das Addukt II in Gegenwart eines homogenen oder heterogenen Übergangsmetallelement-Katalysators in flüssiger Phase oder in Gegenwart eines heterogenen, Übergangsmetallelement-haltigen Katalysators in der Gasphase zum Enolether der Formel IV 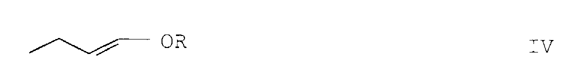 isomerisiert, und
d) aus diesem Enolether IV durch dessen Umsetzung mit Wasserstoff und Wasser oder Wasser in Gegenwart eines homogenen oder heterogenen Übergangsmetallelement-Katalysators in flüssiger Phase oder in Gegenwart eines heterogenen Übergangsmetallelement-haltigen Katalysators in der Gasphase n-Butyraldehyd und/oder n-Butanol erzeugt und den Alkohol ROH I wieder freisetzt, und
den freigesetzten Alkohol ROH I wieder in die Umsetzung gemäß Teilreaktion a) zurückführt.

2. Verfahren nach Anspruch 1, dadurch gekennzeichnet, daß man die Umsetzung von 1,3-Butadien mit einem Alkohol ROH I in Gegenwart eines sauren Ionenaustauschers vornimmt.

3. Verfahren nach Anspruch 1, dadurch gekennzeichnet, daß man die Umsetzung von 1,3-Butadien mit einem Alkohol ROH I in Gegenwart eines sauren Ionenaustauschers durchführt, der zusätzlich mit mindestens einer Lewis-Säure dotiert ist.

4. Verfahren nach Anspruch 1, dadurch gekennzeichnet, daß man die Umsetzung von 1,3-Butadien mit einem Alkohol ROH I in Gegenwart eines Zeolithen in der H⁺-Form, einer Bleicherde oder eines sauren Aluminiumphosphats vornimmt.

5. Verfahren nach Anspruch 1, dadurch gekennzeichnet, daß man die Umsetzung von 1,3-Butadien mit einem Alkohol ROH I in Gegenwart eines Katalysators aus einem Alkyl-, Aryl- oder Arylalkyl-Phosphin-Komplex eines Übergangsmetalls aus der Gruppe Ib, VIIb oder VIIIb des Periodensystems der Elemente durchführt.

6. Verfahren nach Anspruch 1, dadurch gekennzeichnet, daß man die Umsetzung von 1,3-Butadien mit einem Alkohol ROH I in Gegenwart eines Katalysators aus einem Alkyl-, Aryl- oder Arylalkyl-Phosphin-Komplex des Rhodiums, Rutheniums, Nickels, Palladiums, Iridiums oder Platins durchführt.

7. Verfahren nach Anspruch 1, dadurch gekennzeichnet, daß man die Isomerisierung des Addukts III zum Addukt II in Gegenwart eines Katalysators durchführt, wie er zur Katalyse der Addition des Alkohols ROH I an 1,3-Butadien gemäß Teilreaktion a) verwendet wird, durchführt.

8. Verfahren nach Anspruch 1, dadurch gekennzeichnet, daß man das Addukt III vom Addukt II abtrennt und das Addukt III anschließend in die Teilreaktion a) zurückführt und dort zum Addukt II isomerisiert.

9. Verfahren nach Anspruch 1, dadurch gekennzeichnet, daß man die Teilreaktionen c) - Isomerisierung des Addukts II zum Enolether IV - und d) - Hydrolyse oder kombinierte Hydrolyse/ Hydrierung des Enolethers IV zu n-Butyraldehyd und/oder n-Butanol - in einer einzigen Verfahrensstufe durchführt.

10. Verfahren nach Anspruch 1, dadurch gekennzeichnet, daß man die Teilreaktionen c) und d) in einer einzigen Verfahrensstufe in der flüssigen Phase in An- oder Abwesenheit eines zugesetzten Lösungsmittels vornimmt.

11. Verfahren nach Anspruch 1, dadurch gekennzeichnet, daß man die Teilreaktionen c) und d) in einer einzigen Verfahrensstufe in der flüssigen Phase in Gegenwart eines homogen im Reaktionsmedium löslichen Katalysators und in An- oder Abwesenheit eines zugesetzten Lösungsmittels vornimmt.

12. Verfahren nach Anspruch 1, dadurch gekennzeichnet, daß man die Teilreaktionen c) und d) in einer einzigen Verfahrensstufe in der flüssigen Phase in Gegenwart eines zugesetzten Lösungsmittels, das ein Alkohol ist, vornimmt.

13. Verfahren nach Anspruch 1, dadurch gekennzeichnet, daß man die Teilreaktionen c) und d) in einer einzigen Verfahrensstufe in der flüssigen Phase in Gegenwart eines zugesetzten Lösungsmittels, das n-Butanol ist, vornimmt.

14. Verfahren nach Anspruch 1, dadurch gekennzeichnet, daß man die Teilreaktionen c) und d) in einer einzigen Verfahrensstufe in der flüssigen Phase in An- oder Abwesenheit eines zugesetzten Lösungsmittels und in Gegenwart eines homogen im Reaktionsmedium löslichen Katalysators vornimmt, der ein ein- oder mehrzähniger Phosphin- oder Phosphit-Komplex eines Elementes aus den Gruppen Ib, VIb, VIIb und/oder VIIIb des Periodensystems der Elemente ist.

15. Verfahren nach Anspruch 1, dadurch gekennzeichnet, daß man die Teilreaktionen c) und d) in einer einzigen Verfahrensstufe in flüssiger Phase in An- oder Abwesenheit eines zugesetzten Lösungsmittels und in Gegenwart eines homogen im Reaktionsmedium löslichen Katalysators aus eine Phosphin- oder Phosphit-Komplex eines Elementes der Gruppe Ib, VIb, VIIb und/oder VIIIb des Periodensystems der Elemente und in Gegenwart von Wasserstoff und Wasser und überschüssigem Phosphin- oder Phosphit-Liganden vornimmt und dabei n-Butanol erzeugt.

16. Verfahren nach Anspruch 1, dadurch gekennzeichnet, daß man die Teilreaktionen c) und d) in einer einzigen Verfahrensstufe in flüssiger Phase in An- oder Abwesenheit eines zugesetzten Lösungsmittels und in Gegenwart eines homogen im Reaktionsmedium löslichen Katalysators, der ein Komplex eines stickstoffhaltigen Chelat-Liganden mit einem Element aus der Gruppe Ib, VIb, VIIb oder VIIIb des Periodensystems der Elemente ist, vornimmt.

17. Verfahren nach Anspruch 1, dadurch gekennzeichnet, daß man die Teilreaktionen c) und d) in einer einzigen Verfahrensstufe in flüssiger Phase in An- oder Abwesenheit eines zugesetzten Lösungsmittels und in Gegenwart eines homogen im Reaktionsmedium löslichen Katalysators, der ein Chelatkomplex eines Bipyridin- oder Phenanthrolin-Liganden mit einem Element aus der Gruppe Ib, VIb, VIIb und/oder VIIIb des Periodensystems der Elemente ist, vornimmt.

18. Verfahren nach Anspruch 1, dadurch gekennzeichnet, daß man die Teilreaktionen c) und d) in einer einzigen Verfahrensstufe in flüssiger Phase in An- oder Abwesenheit eines zugesetzten Lösungsmittels und in Gegenwart eines homogen im Reaktionsmedium löslichen Katalysators, der ein Salz eines Elementes aus der Gruppe Ib, VIb, VIIb und/oder VIIIb des Periodensystems der Elemente ist, durchführt.

19. Verfahren nach Anspruch 1, dadurch gekennzeichnet, daß man die Teilreaktionen c) und d) in einer einzigen Verfahrensstufe in flüssiger Phase in An- oder Abwesenheit eines zugesetzten Lösungsmittels und in Gegenwart eines homogen im Reaktionsmedium löslichen Katalysators, der ein Aquo-, Ammin-, Halogeno-, Cyano-, Carbonyl-, Amino- und/oder Acetylacetonato-Komplex eines Elementes aus der Gruppe Ib, VIb, VIIb oder VIIIb des Periodensystems der Elemente ist, vornimmt.

20. Verfahren nach Anspruch 1, dadurch gekennzeichnet, daß man die Teilreaktionen c) und a) in einer einzigen Verfahrensstufe in flüssiger Phase in Gegenwart eines homogen im Reaktionsmedium löslichen Katalysators, der ein Salz oder einen Aquo-, Ammin-, Halogeno-, Cyano-, Amino- und/oder Acetylacetonato-Komplex eines Elementes aus der Gruppe Ib, VIb, VIIb und/oder VIIIb des Periodensystems der Elemente ist, in Gegenwart von Wasser und in An- oder Abwesenheit eines zugesetzten Lösungsmittels vornimmt und dabei n-Butyraldehyd erzeugt.

21. Verfahren nach Anspruch 1, dadurch gekennzeichnet, daß man die Teilreaktionen c) und d) in einer einzigen Verfahrensstufe in flüssiger Phase in Gegenwart einer Organotrioxorhenium-Verbindung der Formel V 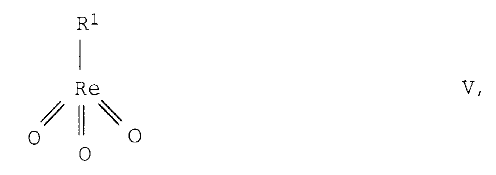 in der R¹ eine C₁- bis C₂₀-Alkylgruppe, eine unsubstituierte oder eine mit 1 bis 5 C₁- bis C₄-Alkylgruppen substituierte Cyclopentadienylgruppe, eine C₆- bis C₁₀-Aryl- oder eine C₇- bis C₁₁-Aralkylgruppe ist und in Gegenwart von Wasser und in An- oder Abwesenheit eines zugesetzten Lösungsmittels vornimmt und dabei n-Butyraldehyd erzeugt.

22. Verfahren nach Anspruch 1, dadurch gekennzeichnet, daß man die Teilreaktionen c) und d) in einer einzigen Verfahrensstufe in flüssiger Phase in Gegenwart eines homogen im Reaktionsmedium löslichen Katalysators, der ein Alkalimetall-, Erdalkalimetall- oder Oniumsalz einer Oxosäure des Vanadiums oder Rheniums ist oder in Gegenwart von Dirheniumheptoxid und in Gegenwart von Wasser und in An- oder Abwesenheit eines zugesetzten Lösungsmittels vornimmt und dabei n-Butyraldehyd erzeugt.

23. Verfahren nach Anspruch 1, dadurch gekennzeichnet, daß man die Teilreaktionen c) und d) in einer einzigen Verfahrensstufe in flüssiger Phase in An- oder Abwesenheit eines zugesetzten Lösungsmittels in Gegenwart eines homogen im Reakionsmedium löslichen Katalysators und in Gegenwart von Wasserstoff und Wasser oder Wasser vornimmt und die nach Abtrennung der Produkte n-Butyraldehyd und/oder n-Butanol erhaltene Katalysatorlösung zur Durchführung der Teilreaktionen c) und d) wiederverwendet.

24. Verfahren nach Anspruch 1, dadurch gekennzeichnet, daß man die Teilreaktionen c) und d) in flüssiger Phase in An- oder Abwesenheit eines zugesetzten Lösungsmittels in einer einzigen Verfahrensstufe in Gegenwart mindestens eines heterogenen, im Reaktionsmedium im wesentlichen unlöslichen Katalysators vornimmt.

25. Verfahren nach Anspruch 1, dadurch gekennzeichnet, daß man die Teilreaktionen c) und d) in flüssiger Phase in An- oder Abwesenheit eines zugesetzten Lösungsmittels in einer einzigen Verfahrensstufe in Gegenwart mindestens einen heterogenen Katalysators, der ein oder mehrere Elemente aus den Gruppen Ib, VIb, VIIb und/oder VIIIb des Periodensystems der Elemente in An- oder Abwesenheit eines oder mehrerer Elemente aus der Gruppe Vb enthält, durchführt.

26. Verfahren nach Anspruch 1, dadurch gekennzeichnet, daß man die Teilreaktionen c) und d) in flüssiger Phase in An- oder Abwesenheit eines zugesetzten Lösungsmittels in einer einzigen Verfahrensstufe in Gegenwart mindestens einen heterogenen Katalysators, der ein oder mehrere Elemente aus den Gruppen Ib, VIb, VIIb und/oder VIIIb des Periodensystems der Elemente in An- oder Abwesenheit eines oder mehrerer Elemente aus der Gruppe Vb und zusätzlich ein Trägermaterial enthält, durchführt.

27. Verfahren nach Anspruch 1, dadurch gekennzeichnet, daß man die Teilreaktionen c) und d) in flüssiger Phase in An- oder Abwesenheit eines zugesetzten Lösungsmittels in einer einzigen Verfahrensstufe in Gegenwart mindestens einen heterogenen Katalysators, der ein oder mehrere Elemente aus den Gruppen Ib, VIb, VIIb und/oder VIIIb des Periodensystems der Elemente in An- oder Abwesenheit eines oder mehrerer Elemente aus der Gruppe Vb und zusätzlich Aluminiumoxid, Titandioxid, Siliziumdioxid, Zirkoniumdioxid, ein Silikat, eine Tonerde, einen Zeolithen und/oder Aktivkohle als Trägermaterial enthält, durchführt.

28. Verfahren nach Anspruch 1, dadurch gekennzeichnet, daß man die Teilreaktionen c) und d) in flüssiger Phase in An- oder Abwesenheit eines zusätzlichen Lösungsmittels in einer einzigen Verfahrensstufe in Gegenwart mindestens eines heterogenen Katalysators, der Kupfer enthält, durchführt.

29. Verfahren nach Anspruch 1, dadurch gekennzeichnet, daß man die Teilreaktionen c) und d) in flüssiger Phase in An- oder Abwesenheit eines zugesetzten Lösungsmittels in einer einzigen Verfahrensstufe in Gegenwart mindestens einen heterogenen Katalysators vornimmt, der in einem Festbett angeordnet ist.

30. Verfahren nach Anspruch 1, dadurch gekennzeichnet, daß man die Teilreaktionen c) und d) in flüssiger Phase in An- oder Abwesenheit eines zugesetzten Lösungsmittels in einer einzigen Verfahrensstufe in Gegenwart mindestens einen heterogenen Katalysators vornimmt, der in 2 bis 5 Festbetten angeordnet ist.

31. Verfahren nach Anspruch 1, dadurch gekennzeichnet, daß man die Teilreaktionen c) und d) in der Gasphase in einer einzigen Verfahrensstufe in Gegenwart mindestens einen heterogenen Katalysators durchführt.

32. Verfahren nach Anspruch 1, dadurch gekennzeichnet, daß man die Teilreaktionen c) und d) in der Gasphase in einer einzigen Verfahrensstufe in Gegenwart mindestens einen heterogenen Katalysators, der ein oder mehrere Elemente aus den Gruppen Ib, VIb, VIIb und/oder VIIIb des Periodensystems der Elemente in An- oder Abwesenheit eines oder mehrerer Elemente aus der Gruppe Vb enthält, durchführt.

33. Verfahren nach Anspruch 1, dadurch gekennzeichnet, daß man die Teilreaktionen c) und d) in der Gasphase in einer einzigen Verfahrensstufe in Gegenwart mindestens einen heterogenen Katalysators, der ein oder mehrere Elemente aus den Gruppen Ib, VIb, VIIb und/oder VIIIb des Periodensystems der Elemente in An- oder Abwesenheit eines oder mehrerer Elemente aus der Gruppe Vb und zusätzlich ein Trägermaterial enthält, durchführt.

34. Verfahren nach Anspruch 1, dadurch gekennzeichnet, daß man die Teilreaktionen c) und d) in der Gasphase in einer einzigen Verfahrensstufe in Gegenwart mindestens einen heterogenen Katalysators, der ein oder mehrere Elemente aus den Gruppen Ib, VIb, VIIb und/oder VIIIb des Periodensystems der Elemente in An- oder Abwesenheit eines oder mehrerer Elemente aus der Gruppe Vb und zusätzlich Aluminiumoxid, Titandioxid, Zirkoniumdioxid, Siliziumdioxid, ein Silikat, eine Tonerde, einen Zeolithen und/oder Aktivkohle als Trägermaterial enthält, durchführt.

35. Verfahren nach Anspruch 1, dadurch gekennzeichnet, daß man die Teilreaktionen c) und d) in der Gasphase in einer einzigen Verfahrensstufe in Gegenwart mindestens einen heterogenen Katalysators, der ein Element aus den Gruppen Ib, VIb, VIIb und/oder VIIIb des Periodensystems der Elemente in An- oder Abwesenheit eines oder mehrerer Elemente aus der Gruppe Vb enthält und in mindestens einem Festbett oder in einer Wirbelschicht angeordnet ist, durchführt.

36. Verfahren nach Anspruch 1, dadurch gekennzeichnet, daß man die Teilreaktionen c) und d) in der Gasphase in einer einzigen Verfahrensstufe in Gegenwart von mindestens zwei Katalysatoren durchführt, die ein oder mehrere Elemente aus den Gruppen Ib, VIb, VIIb und/oder VIIIb des Periodensystems der Elemente in An- oder Abwesenheit eines oder mehrerer Elemente aus der Gruppe Vb enthalten und in einer kombinierten Schüttung in mindestens einem Festbett angeordnet sind.

37. Verfahren nach Anspruch 1, dadurch gekennzeichnet, daß man die Teilreaktionen c) und d) in der Gasphase in einer einzigen Verfahrensstufe in Gegenwart eines heterogenen Katalysators oder mehrerer heterogener Katalysatoren durchführt, die ein oder mehrere Elemente aus den Gruppen Ib, VIb, VIIb und/oder VIIIb des Periodensystems der Elemente in An- oder Abwesenheit eines oder mehrerer Elemente aus der Gruppe Vb enthalten und die in mindestens 2 Festbetten angeordnet sind.

38. Verfahren nach Anspruch 1, dadurch gekennzeichnet, daß man die Teilreaktionen c) und d) in flüssiger Phase und/oder in der Gasphase in aufeinanderfolgenden Verfahrensstufen ausführt.

39. Verfahren nach Anspruch 1, dadurch gekennzeichnet, daß man die Teilreaktionen c) und d) in der flüssigen Phase in aufeinanderfolgenden Verfahrensstufen unter Verwendung homogener und/oder heterogener Katalysatoren in den einzelnen Verfahrensstufen durchführt.

40. Verfahren nach Anspruch 1, dadurch gekennzeichnet, daß man die Teilreaktionen c) und d) in aufeinanderfolgenden Verfahrensstufen in flüssiger Phase und in der Gasphase unter Verwendung homogener und/oder heterogener Katalysatoren durchführt.

41. Verfahren nach Anspruch 1, dadurch gekennzeichnet, daß man als Alkohol ROH I n-Butanol verwendet.

## Claims

1. A process for the preparation of n-butyraldehyde and/or n-butanol, wherein
a) 1,3-butadiene or a 1,3-butadiene-containing hydrocarbon stream is reacted with an alcohol of the formula I
ROH I
where R is a C₂- to C₂₀-alkyl or alkenyl group which is unsubstituted or monosubstituted or disubstituted by C₁- to C₁₀-alkoxy or by hydroxyl, or is C₆- to C₁₀-aryl, C₇- to C₁₁-aralkyl or methyl, at elevated temperatures and at superatmospheric pressure in the presence of a Brönsted acid or in the presence of a complex of an element of group Ib, VIIb or VIIIb of the Periodic Table of Elements having phosphorus- or nitrogen-containing ligands to give a mixture of the adducts of the formulae II 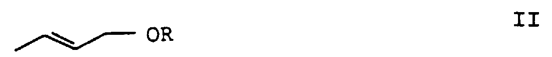 and III 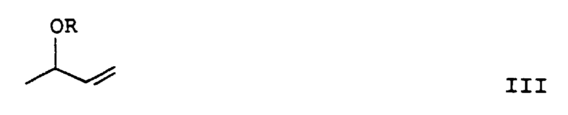
b) the adduct III is isomerized to the adduct II,
c) the adduct II is isomerized in the presence of a homogeneous or heterogeneous transition metal catalyst in the liquid phase or in the presence of a heterogeneous transition metal-containing catalyst in the gas phase to give an enol ether of the formula IV 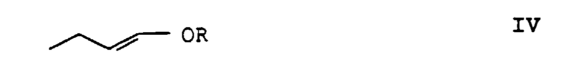 and
d) n-butyraldehyde and/or n-butanol are produced and the alcohol ROH I is liberated from this enol ether IV by reacting it with hydrogen and water or water in the presence of a homogeneous or heterogeneous transition metal catalyst in the liquid phase or in the presence of a heterogeneous transition metal-containing catalyst in the gas phase, and
the liberated alcohol ROH I is recycled to the reaction according to reaction step a).

2. A process as claimed in claim 1, wherein the reaction of 1,3-butadiene with an alcohol ROH I is carried out in the presence of an acidic ion exchanger.

3. A process as claimed in claim 1, wherein the reaction of 1,3-butadiene with an alcohol ROH I is carried out in the presence of an acidic ion exchanger which is additionally doped with at least one Lewis acid.

4. A process as claimed in claim 1, wherein the reaction of 1,3-butadiene with an alcohol ROH I is carried out in the presence of a zeolite in H⁺ form, of a bleaching earth or of an acidic aluminum phosphate.

5. A process as claimed in claim 1, wherein the reaction of 1,3-butadiene with an alcohol ROH I is carried out in the presence of a catalyst comprising an alkyl-, aryl- or arylalkylphosphine complex of a transition metal of group Ib, VIIb or VIIIb of the Periodic Table of Elements.

6. A process as claimed in claim 1, wherein the reaction of 1,3-butadiene with an alcohol ROH I is carried out in the presence of a catalyst comprising an alkyl-, aryl- or arylalkylphosphine complex of rhodium, of ruthenium, of nickel, of palladium, of iridium or of platinum.

7. A process as claimed in claim 1, wherein the isomerization of the adduct III to the adduct II is carried out in the presence of a catalyst as used for the catalysis of the addition reaction of an alcohol ROH I with 1,3-butadiene according to reaction step a).

8. A process as claimed in claim 1, wherein the adduct III is separated from the adduct II and the adduct III is then recycled to reaction step a) and is isomerized there to give the adduct II.

9. A process as claimed in claim 1, wherein reaction steps c), isomerization of the adduct II to the enol ether IV, and d), hydrolysis or combined hydrolysis/ hydrogenation of enol ether IV to n-butyraldehyde and/or n-butanol, are carried out in a single process stage.

10. A process as claimed in claim 1, wherein reaction steps c) and d) are carried out in a single process stage in the liquid phase in the presence of an added solvent or in the absence of a solvent.

11. A process as claimed in claim 1, wherein reaction steps c) and d) are carried out in a single process stage in the liquid phase in the presence of a catalyst which dissolves homogeneously in the reaction medium and in the presence of an added solvent or in the absence of a solvent.

12. A process as claimed in claim 1, wherein reaction steps c) and d) are carried out in a single process stage in the liquid phase in the presence of an added solvent which is an alcohol.

13. A process as claimed in claim 1, wherein reaction steps c) and d) are carried out in a single process stage in the liquid phase in the presence of an added solvent which is n-butanol.

14. A process as claimed in claim 1, wherein reaction steps c) and d) are carried out in a single process stage in the liquid phase in the presence of an added solvent or in the absence of a solvent and in the presence of a catalyst which dissolves homogeneously in the reaction medium and is a monodentate or polydentate phosphine or phosphite complex of an element of groups Ib, VIb, VIIb and/or VIIIb of the Periodic Table of Elements.

15. A process as claimed in claim 1, wherein the reaction steps c) and d) are carried out in a single process stage in liquid phase in the presence of an added solvent or in the absence of a solvent and in the presence of a catalyst which dissolves homogeneously in the reaction medium and comprises a phosphine or phosphite complex of an element of group Ib, VIb, VIIb and/or VIIIb of the Periodic Table of Elements and in the presence of hydrogen and water and excess phosphine or phosphite ligands, and n-butanol is produced.

16. A process as claimed in claim 1, wherein the reaction steps c) and d) are carried out in a single process stage in the liquid phase in the presence of an added solvent or in the absence of a solvent and in the presence of a catalyst which dissolves homogeneously in the reaction medium and is a complex of a nitrogen-containing chelate ligand with an element of group Ib, VIb, VIIb or VIIIb of the Periodic Table of Elements.

17. A process as claimed in claim 1, wherein the reaction steps c) and d) are carried out in a single process stage in the liquid phase in the presence of an added solvent or in the absence of a solvent and in the presence of a catalyst which dissolves homogeneously in the reaction medium and is a chelate complex of a by- pyridine or phenanthroline ligand with an element of group Ib, VIb, VIIb or VIIIb of the Periodic Table of Elements.

18. A process as claimed in claim 1, wherein reaction steps c) and d) are carried out in a single process stage in the liquid phase in the presence of an added solvent or in the absence of a solvent and in the presence of a catalyst which dissolves homogeneously in the reaction medium and is a salt of an element of group Ib, VIb, VIIb and/or VIIIb of the Periodic Table of Elements.

19. A process as claimed in claim 1, wherein reaction steps c) and d) are carried out in a single process stage in the liquid phase in the presence of an added solvent or in the absence of a solvent and in the presence of a catalyst which dissolves homogeneously in the reaction medium and is an aquo, ammine, halo, cyano, carbonyl, amino and/or acetylacetonato complex of an element of group Ib, VIb, VIIb or VIIIb of the Periodic Table of Elements.

20. A process as claimed in claim 1, wherein reaction steps c) and d) are carried out in a single process stage in the liquid phase in the presence of a catalyst which dissolves homogeneously in the reaction medium and is a salt or an aquo, ammine, halo, cyano, amino and/or acetylacetonato complex of an element of group Ib, VIb, VIIb and/or VIIIb of the Periodic Table of Elements, in the presence of water and in the presence of an added solvent or in the absence of a solvent, and n-butyraldehyde is produced.

21. A process as claimed in claim 1, wherein reaction steps c) and d) are carried out in a single process stage in the liquid phase in the presence of an organotrioxorhenium compound of the formula V 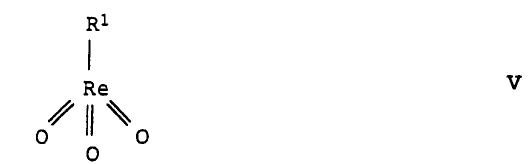 where R¹ is C₁- to C₂₀-alkyl, cyclopentadienyl which is unsubstituted or monosubstituted to pentasubstituted by C₁- to C₄-alkyl, or is C₆- to C₁₀-aryl or C₇- to C₁₁-aralkyl, and in the presence of water and in the presence of an added solvent or in the absence of a solvent, and n-butyraldehyde is produced.

22. A process as claimed in claim 1, wherein reaction steps c) and d) are carried out in a single process stage in the liquid phase in the presence of a catalyst which dissolves homogeneously in the reaction medium and is an alkali metal, alkaline earth metal or onium salt of an oxo acid of vanadium or of rhenium or in the presence of dirhenium heptoxide and in the presence of water and in the presence of an added solvent or in the absence of a solvent, and n-butyraldehyde is produced.

23. A process as claimed in claim 1, wherein reaction steps c) and d) are carried out in a single process stage in the liquid phase in the presence of an added solvent or in the absence of a solvent, in the presence of a catalyst which dissolves homogeneously in the reaction medium and in the presence of hydrogen and water or water, and the catalyst solution obtained after the products n-butyraldehyde and n-butanol have been separated off is used again for carrying out reaction steps c) and d).

24. A process as claimed in claim 1, wherein reaction steps c) and d) are carried out in the liquid phase in the presence of an added solvent or in the absence of a solvent in a single process stage in the presence of at least one heterogeneous catalyst which is essentially insoluble in the reaction medium.

25. A process as claimed in claim 1, wherein reaction steps c) and d) are carried out in the liquid phase in the presence of an added solvent or in the absence of a solvent in a single process stage in the presence of at least one heterogeneous catalyst which contains one or more elements of groups Ib, VIb, VIIb and/or VIIIb of the Periodic Table of Elements in the presence or absence of one or more elements of group Vb.

26. A process as claimed in claim 1, wherein reaction steps c) and d) are carried out in the liquid phase in the presence of an added solvent or in the absence of a solvent in a single process stage in the presence of at least one heterogeneous catalyst which contains one or more elements of groups Ib, VIb, VIIb and/or VIIIb of the Periodic Table of Elements in the presence or absence of one or more elements of group Vb and additionally contains a carrier.

27. A process as claimed in claim 1, wherein reaction steps c) and d) are carried out in the liquid phase in the presence of an added solvent or in the absence of a solvent in a single process stage in the presence of at least one heterogeneous catalyst which contains one or more elements of group Ib, VIb, VIIb and/or VIIIb of the Periodic Table of Elements in the presence or absence of one or more elements of group Vb and additionally contains aluminum oxide, titanium dioxide, silica, zirconium dioxide, a silicate, an alumina, a zeolite and/or active carbon as the carrier.

28. A process as claimed in claim 1, wherein reaction steps c) and d) are carried out in the liquid phase in the presence of an additional solvent or in the absence of a solvent in a single process stage in the presence of at least one heterogeneous catalyst which contains copper.

29. A process as claimed in claim 1, wherein reaction steps c) and d) are carried out in the liquid phase in the presence of an added solvent or in the absence of a solvent in a single process stage in the presence of at least one heterogeneous catalyst which is arranged in a fixed bed.

30. A process as claimed in claim 1, wherein reaction steps c) and d) are carried out in the liquid phase in the presence of an added solvent or in the absence of a solvent in a single process stage in the presence of at least one heterogeneous catalyst which is arranged in from 2 to 5 fixed beds.

31. A process as claimed in claim 1, wherein reaction steps c) and d) are carried out in the gas phase in a single process stage in the presence of at least one heterogeneous catalyst.

32. A process as claimed in claim 1, wherein reaction steps c) and d) are carried out in the gas phase in a single process stage in the presence of at least one heterogeneous catalyst which contains one or more elements of groups Ib, VIb, VIIb and/or VIIIb of the Periodic Table of Elements in the presence or absence of one or more elements of group Vb.

33. A process as claimed in claim 1, wherein reaction steps c) and d) are carried out in the gas phase in a single process stage in the presence of at least one heterogeneous catalyst which contains one or more elements of groups Ib, VIb, VIIb and/or VIIIb of the Periodic Table of Elements in the presence or absence of elements of group Vb, and additionally contains a carrier.

34. A process as claimed in claim 1, wherein reaction steps c) and d) are carried out in the gas phase in a single process stage in the presence of at least one heterogeneous catalyst which contains one or more elements of groups Ib, VIb, VIIb and/or VIIIb of the Periodic Table of Elements in the presence of one or more elements of group Vb and additionally contains aluminum oxide, titanium dioxide, zirconium dioxide, silica, a silicate, an alumina and/or active carbon as the carrier.

35. A process as claimed in claim 1, wherein reaction steps c) and d) are carried out in the gas phase in a single process stage in the presence of at least one heterogeneous catalyst which contains an element of groups Ib, VIb, VIIb and/or VIIIb of the Periodic Table of Elements in the presence or absence of one or more elements of group Vb and is arranged in at least one fixed bed or in a fluidized bed.

36. A process as claimed in claim 1, wherein reaction steps c) and d) are carried out in the gas phase in a single process stage in the presence of at least two catalysts which contain one or more elements of groups Ib, VIb, VIIb and/or VIIIb of the Periodic Table of Elements in the presence or absence of one or more elements of group Vb and are arranged in a combined bed in at least one fixed bed.

37. A process as claimed in claim 1, wherein reaction steps c) and d) are carried out in the gas phase in a single process stage in the presence of a heterogeneous catalyst or of a plurality of heterogeneous catalysts which contains or contain one more elements of groups Ib, VIb, VIIb and/or VIIIb of the Periodic Table of Elements in the presence or absence of one or more elements of group Vb and is or are arranged in at least 2 fixed beds.

38. A process as claimed in claim 1, wherein reaction steps c) and d) are carried out in the liquid phase and/or in the gas phase in successive process stages.

39. A process as claimed in claim 1, wherein reaction steps c) and d) are carried out in the liquid phase in successive process stages using homogeneous and/or heterogeneous catalysts in the individual process stages.

40. A process as claimed in claim 1, wherein reaction steps c) and d) are carried out in successive process stages in the liquid phase and in the gas phase using homogeneous and/or heterogeneous catalysts.

41. A process as claimed in claim 1, wherein the alcohol ROH I used is n-butanol.

## Revendications

1. Procédé de préparation du n-butyraldéhyde et/ou du n-butanol, caractérisé en ce que l'on fait réagir
a) le 1,3-butadiène ou des courants d'hydrocarbures contenant du 1,3-butadiène, avec un alcool de la formule I
ROH I,
dans laquelle le symbole R représente un radical aralkyle en C₇ à C₁₁, aryle en C₆ à C₁₀, alcényle ou alkyle en C₂ à C₂₀, substitués par un ou deux radicaux hydroxyle ou alcoxy en C₁ à C₁₀, ou le radical méthyle, à température élevée et sous pression élevée, en présence d'un acide de Brönsted, ou en présence d'un complexe d'un élément du groupe Ib, Vllb ou Vlllb du système périodique des éléments avec des ligands contenant du phosphore ou de l'azote, de manière à obtenir un mélange des adduits des formules Il 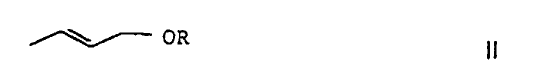 et III 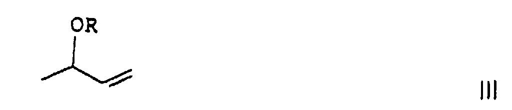
b) on isomérise l'adduit III en adduit Il,
c) on isomérise l'adduit II, en présence d'un catalyseur à base d'un élément du type métal de transition, homogène ou hétérogène, en phase liquide ou en présence d'un catalyseur contenant un élément du type métal de transition, hétérogène, en phase gazeuse, de manière à obtenir un éther énolique de la formule IV 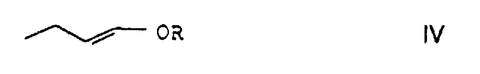 et
d) on obtient le n-butyraldéhyde et/ou le n-butanol à partir de cet éther énolique IV par la réaction de ce demier avec de l'hydrogène et de l'eau, ou de l'eau, en présence d'un catalyseur à base d'un élément du type métal de transition, homogène ou hétérogène, en phase gazeuse, ou en présence d'un catalyseur contenant un élément du type métal de transition hétérogène, en phase gazeuse et on relibère l'alcool ROH I et
on ramène l'alcool ROH I libéré dans la réaction suivant la rection partielle a).

2. Procédé suivant la revendication 1, caractérisé en ce que l'on entreprend la réaction du 1,3-butadiène avec un alcool ROHI en présence d'un échangeur d'ions acide.

3. Procédé suivant la revendication 1, caractérisé en ce que l'on entreprend la réaction du 1,3-butadiène avec un alcool ROH I, en présence d'un échangeur d'ions acide, qui est complémentairement dopé par au moins un acide de Lewis.

4. Procédé suivant la revendication 1, caractérisé en ce que l'on entreprend la réaction du 1,3-butadiène avec un alcool ROH I, en présence d'une zéolite sous la forme H⁺, d'une terre de blanchiment, ou d'un phosphate d'aluminium acide.

5. Procédé suivant la revendication 1, caractérisé en ce que l'on entreprend la réaction du 1 ,3-butadiène avec un alcool ROH I en présence d'un catalyseur formé d'un complexe d'alkyl, d'aryl- ou d'arylalkyl-phosphine d'un métal de transition appartenant au groupe Ib, Vllb ou Vlllb du système périodique des éléments.

6. Procédé suivant la revendication 1, caractérisé en ce que l'on entreprend la réaction du 1,3-butadiène avec un alcool ROHI en présence d'un catalyseur constitué d'un complexe d'alkyl-, d'aryl- ou d'aryl-alkyl-phosphine du rhodium, du ruthénium, du nickel, du palladium, de l'iridium ou du platine.

7. Procédé suivant la revendication 1, caractérisé en ce que l'on entreprend l'isomérisation de l'adduit III en adduit Il en présence d'un catalyseur, tel qu'on l'utilise pour la catalyse de l'addition de l'alcool ROH I sur le 1,3-butadiène selon la réaction partielle a).

8. Procédé suivant la revendication 1, caractérisé en ce que l'on sépare l'adduit III de l'adduit Il et on ramène ensuite l'adduit III à la réaction partielle a) et on l'y isomérise en adduit Il.

9. Procédé suivant la revendication 1, caractérisé en ce que l'on entreprend les réactions partielles c) - isomérisation de l'adduit Il en éther énolique IV - et d) - hydrolyse ou hydrolyse combinée/hydrogénation de l'éther énolique IV en n-butyraldéhyde et/ou n-butanol- au cours d'une seule et unique étape opératoire.

10. Procédé suivant la revendication 1, caractérisé en ce que l'on entreprend les réactions partielles c) et d) en une seule et unique étape opératoire, en phase liquide, en présence ou en l'absence d'un solvant ajouté.

11. Procédé suivant la revendication 1, caractérisé en ce que l'on entreprend les réactions partielles c) et d) en une seule et unique étape opératoire, en phase liquide, en présence d'un catalyseur soluble, de manière homogène dans le milieu réactionnel et en présence ou en l'absence d'un solvant ajouté.

12. Procédé suivant la revendication 1, caractérisé en ce que l'on entreprend les réactions partielles c) et d) en une seule et unique étape opératoire, en phase liquide, en présence d'un solvant ajouté, qui est un alcool.

13. Procédé suivant la revendication 1, caractérisé en ce que l'on entreprend les réactions partielles c) et d) en une seule et unique étape opératoire, en phase liquide, en présence d'un solvant ajouté qui est le n-butanol.

14. Procédé suivant la revendication 1, caractérisé en ce que l'on entreprend les réactions partielles c) et d) en une seule et unique étape opératoire. en phase liquide, en présence ou en l'absence d'un solvant ajouté et en présence d'un catalyseur soluble de manière homogène dans le milieu réactionnel, qui est un complexe de phosphine ou de phosphite monodentate ou polydentate d'un élément appartenant aux groupes Ib, Vlb, Vllb et/ou Vlllb du tableau périodique des éléments.

15. Procédé suivant la revendication 1, caractérisé en ce que l'on entreprend les réactions partielles c) et d) en une seule et unique étape opératoire, en phase liquide, en présence ou en l'absence d'un solvant ajouté et en présence d'un catalyseur soluble, de manière homogène dans le milieu réactionnel, constitué d'un complexe de phosphine ou de phosphite d'un élément appartenant aux groupes Ib, Vlb, Vllb et/ou Vlllb du système périodique des éléments et en présence d'hydrogène et d'eau et d'un ligand à phosphine ou à phosphite excédentaire et on obtient ainsi le n-butanol.

16. Procédé suivant la revendication 1, caractérisé en ce que l'on entreprend les réactions partielles c) et d) en une seule et unique étape opératoire, en phase liquide, en présence ou en l'absence d'un solvant ajouté et en présence d'un catalyseur soluble de manière homogène dans le milieu réactionnel qui est un complexe d'un ligand du type chélate contenant de l'azote avec un élément appartenant aux groupes Ib, Vlb, Vllb ou VIIIb du système périodique des éléments.

17. Procédé suivant la revendication 1, caractérisé en ce que l'on entreprend les réactions partielles c) et d) en une seule et unique étape opératoire, en phase liquide, en présence ou en l'absence d'un solvant ajouté et en présence d'un catalyseur soluble, de manière homogène dans le milieu réactionnel, qui est un complexe du type chélate d'un ligand du type bipyridine ou phénanthroline, avec un élément appartenant aux groupes Ib, Vlb, Vllb et/ou Vlllb du système périodique des éléments.

18. Procédé suivant la revendication 1, caractérisé en ce que l'on entreprend les réactions partielles c) et d) en une seule et unique étape opératoire, en phase liquide, en présence ou en l'absence d'un solvant ajouté et en présence d'un catalyseur soluble, de manière homogène dans le milieu réactionnel, qui est un sel d'un élément appartenant aux groupes Ib, Vlb, Vllb et/ou Vlllb du système périodique des éléments.

19. Procédé suivant la revendication 1, caractérisé en ce que l'on entreprend les réactions partielles c) et d) en une seule et unique étape opératoire, en phase liquide, en présence d'un catalyseur soluble, qui est un cyano-, carbonylo-, amino- et/ou acétylacétonato-complexe d'un élément appartenant aux groupes Ib, Vlb, Vllb ou Vlllb du système périodique des éléments.

20. Procédé suivant la revendication 1, caractérisé en ce que l'on entreprend les réactions partielles c) et d) en une seule et unique étape opératoire, en phase liquide, en présence d'un catalyseur soluble de manière homogène dans le milieu réactionnel, qui est un sel ou un aquo-, ammino-, halogéno-, cyano-, amino- et/ou acétylacétonato-complexe d'un élément appartenant aux groupes Ib, Vlb, Vllb et/ou Vlllb du système périodique des éléments, en présence d'eau et en présence ou en l'absence d'un solvant ajouté et on obtient ainsi le n-butyraldéhyde.

21. Procédé suivant la revendication 1, caractérisé en ce que l'on entreprend les réactions partielles c) et d) en une seule et unique étape opératoire, en phase liquide, en présence d'un composé d'organotrioxorhénium de la formule V 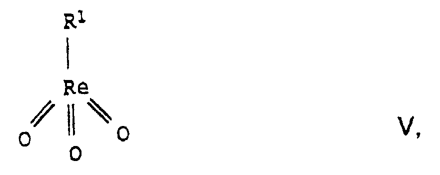 dans laquelle R¹ représente un radical alkyle en C₁ à C₂₀, un radical cyclopentadiényle non substitué ou substitué par de 1 à 5 radicaux alkyle en C₁ à C₄, un radical aryle en C₆ à C₁₀ ou un radical aralkyle en C₇ à C₁₁ et en présence d'eau et en présence ou en l'absence d'un solvant ajouté et on obtient ainsi le n-butyraldéhyde.

22. Procédé suivant la revendication 1, caractérisé en ce que l'on entreprend les réactions partielles c) et d) en une seule et unique étape opératoire, en phase liquide, en présence d'un catalyseur soluble de manière homogène dans le milieu réactionnel, qui est un sel de métal alcalin, de métal alcalino-terreux, ou d'onium, d'un oxoacide du vanadium ou du rhénium, ou en présence d'heptoxyde de dirhénium et en présence d'eau et en présence ou l'absence d'un solvant ajouté et on obtient ainsi le n-butyraldéhyde.

23. Procédé suivant la revendication 1, caractérisé en ce que l'on entreprend les réactions partielles c) et d) en une seule et unique étape opératoire, en phase liquide, en présence ou en l'absence d'un solvant ajouté, en présence d'un catalyseur soluble de manière homogène dans le milieu réactionnel et en présence d'hydrogène et d'eau, ou d'eau et on réutilise la solution de catalyseur obtenue après la séparation des produits que sont le n-butyraldéhyde et/ou le n-butanol, pour la réalisation des réactions partielles c) et d).

24. Procédé suivant la revendication 1, caractérisé en ce que l'on entreprend les réactions partielles c) et d) en phase liquide, en présence ou en l'absence d'un solvant ajouté en une seule et unique étape opératoire, en présence d'au moins un catalyseur hétérogène, sensiblement insoluble dans le milieu réactionnel.

25. Procédé suivant la revendication 1, caractérisé en ce que l'on entreprend les réactions partielles c) et d) en phase liquide, en présence ou en l'absence d'un solvant ajouté en une seule et unique étape opératoire, en présence d'au moins un catalyseur hétérogène, qui contient un ou plusieurs éléments appartenant aux groupes Ib, Vlb, Vllb et/ou VIIIb du système périodique des éléments, en présence ou en l'absence d'un ou plusieurs éléments appartenant au groupe Vb.

26. Procédé suivant la revendication 1, caractérisé en ce que l'on entreprend les réactions partielles c) et d) en phase liquide, en présence ou en l'absence d'un solvant ajouté en une seule et unique étape opératoire, en présence d'au moins un catalyseur hétérogène, qui contient un ou plusieurs éléments appartenant aux groupes Ib, Vlb, Vllb et/ou Vllb, du système périodique des éléments, en présence ou en l'absence d'un ou plusieurs éléments appartenant au groupe Vb et, complémentairement, une matière de support.

27. Procédé suivant la revendication 1, caractérisé en ce que l'on entreprend les réactions partielles c) et d) en phase liquide, en présence ou en l'absence d'un solvant ajouté en une seule et unique étape opératoire, en présence d'au moins un catalyseur hétérogène, qui contient un ou plusieurs éléments appartenant aux groupes Ib, Vlb, Vllb et/ou Vllb, du système périodique des éléments, en présence ou en l'absence d'un ou plusieurs éléments appartenant au groupe Vb et, complémentairement, de l'oxyde d'aluminium, du dioxyde de titane, du dioxyde de silicium, du dioxyde de zirconium, un silicate, une alumine, une zéolite et/ou du charbon activé, à titre de matière de support.

28. Procédé suivant la revendication 1, caractérisé en ce que l'on entreprend les réactions partielles c) et d) en phase liquide, en présence ou en l'absence d'un solvant complémentaire, en une seule et unique étape opératoire, en présence d'au moins un catalyseur hétérogène qui contient du cuivre.

29. Procédé suivant la revendication 1, caractérisé en ce que l'on entreprend les réactions partielles c) et d) en phase liquide, en présence ou en l'absence d'un solvant ajouté, en une seule et unique étape opératoire, en présence d'au moins un catalyseur hétérogène qui est agencé en lit fixe.

30. Procédé suivant la revendication 1, caractérisé en ce que l'on entreprend les réactions partielles c) et d) en phase liquide, en présence ou en l'absence d'un solvant ajouté, en une seule et unique étape opératoire, en présence d'au moins un catalyseur hétérogène, en 2 à 5 lits fixes.

31. Procédé suivant la revendication 1, caractérisé en ce que l'on entreprend les réactions partielles c) et d) en phase gazeuse, en une seule et unique étape opératoire, en présence d'au moins un catalyseur hétérogène.

32. Procédé suivant la revendication 1, caractérisé en ce que l'on entreprend les réactions partielles c) et d) en phase gazeuse, en une seule et unique étape opératoire, en présence d'au moins un catalyseur hétérogène qui contient un ou plusieurs éléments appartenant aux groupes Ib, Vlb, Vllb et/ou Vlllb du système périodique des éléments, en présence ou en l'absence d'un ou plusieurs éléments appartenant au groupe Vb.

33. Procédé suivant la revendication 1, caractérisé en ce que l'on entreprend les réactions partielles c) et d) en phase gazeuse, en une seule et unique étape opératoire, en présence d'au moins un catalyseur hétérogène qui contient un ou plusieurs éléments appartenant aux groupes Ib, Vlb, Vllb et/ou Vlllb du système périodique des éléments, en présence ou en l'absence d'un ou plusieurs éléments appartenant au groupe Vb et complémentairement une matière de support.

34. Procédé suivant la revendication 1, caractérisé en ce que l'on entreprend les réactions partielles c) et d) en phase gazeuse, en une seule et unique étape opératoire, en présence d'au moins un catalyseur hétérogène qui contient un ou plusieurs éléments appartenant aux groupes Ib, Vlb, Vllb et/ou Vlllb du système périodique des éléments, en présence ou en l'absence d'un ou plusieurs éléments appartenant au groupe Vb et, complémentairement, de l'oxyde d'aluminium, du dioxyde de titane, du dioxyde de zirconium, du dioxyde de silicium, un silicate, une alumine, une zéolite et/ou du charbon activé à titre de matière de support.

35. Procédé suivant la revendication 1, caractérisé en ce que l'on entreprend les réactions partielles c) et d) en phase gazeuse, en une seule et unique étape opératoire, en présence d'au moins un catalyseur hétérogène qui contient un ou plusieurs éléments appartenant aux groupes Ib, Vlb, Vllb et/ou Vlllb du système périodique des éléments, en présence ou en l'absence d'un ou plusieurs éléments appartenant au groupe Vb et qui est agencé en un lit fixe ou en une couche fluidisée.

36. Procédé suivant la revendication 1, caractérisé en ce que l'on entreprend les réactions partielles c) et d) en phase gazeuse, en une seule et unique étape opératoire, en présence d'au moins deux catalyseurs qui contiennent un ou plusieurs éléments appartenant aux groupes Ib, Vlb, Vllb et/ou Vlllb du système périodique des éléments, en présence ou en l'absence d'un ou plusieurs éléments appartenant au groupe Vb et qui sont agencés en une garniture combinée dans au moins un lit fixe.

37. Procédé suivant la revendication 1, caractérisé en ce que l'on entreprend les réactions partielles c) et d) en phase gazeuse, en une seule et unique étape opératoire, en présence d'un catalyseur hétérogène ou de plusieurs catalyseurs hétérogènes, qui contiennent un ou plusieurs éléments appartenant aux groupes Ib, Vlb, Vllb et/ou Vlllb du système périodique des éléments, en présence ou en l'absence d'un ou plusieurs éléments appartenant au groupe Vlb et qui sont agencés en au moins deux lits fixes.

38. Procédé suivant la revendication 1, caractérisé en ce que l'on entreprend les réactions partielles c) et d) en phase liquide et/ou en phase gazeuse dans des étapes opératoires successives.

39. Procédé suivant la revendication 1, caractérisé en ce que l'on entreprend les réactions partielles c) et d) en phase liquide, dans des étapes opératoires successives et en recourant à l'utilisation de catalyseurs homogènes et/ou hétérogènes dans les étapes opératoires individuelles.

40. Procédé suivant la revendication 1, caractérisé en ce que l'on entreprend les réactions partielles c) et d), au cours d'étapes opératoires successives, en phase liquide et en phase gazeuse, en recourant à l'emploi de catalyseurs homogènes et/ou hétérogènes.

41. Procédé suivant la revendication 1, caractérisé en ce que l'on utilise le n-butanol, à titre d'alcool ROH I.
